# EUROPEAN PATENT SPECIFICATION

(11) **EP 3 519 049 B1**
(45) Date of publication and mention of the grant of the patent: **03.12.2025**
(21) Application number: 17857471.1
(22) Date of filing: 29.09.2017
(51) Int. Cl.: C07K 16/24, A61P 25/00, A61P 37/00

(54) **SAFE AND EFFECTIVE METHOD OF TREATING PSORIASIS WITH ANTI-IL23 SPECIFIC ANTIBODY**
SICHERES UND EFFIZIENTES VERFAHREN ZUR BEHANDLUNG VON PSORIASIS MIT ANTI-IL23-SPEZIFISCHEM ANTIKÖRPER
PROCÉDÉ SÛR ET EFFICACE DE TRAITEMENT DU PSORIASIS AVEC UN ANTICORPS SPÉCIFIQUE CONTRE L'IL-23

(30) Priority: 30.09.2016 US 201662402403 P; 13.07.2017 US 201762532068 P
(43) Date of publication of application: 07.08.2019
(73) Proprietor: Janssen Biotech, Inc., Horsham, PA 19044 (US)
(72) Inventor: RANDAZZO, Bruce, Spring House Pennsylvania 19477 (US); WASFI, Yasmine, Spring House Pennsylvania 19477 (US)
(74) Representative: Carpmaels & Ransford LLP
(86) International application number: PCT/US2017/054217
(87) International publication number: WO 2018/064436

(56) References cited:
- WO-A1-2016/031250
- WO-A1-2018/093841
- US-A1- 2009 181 027
- US-A1- 2011 287 028
- US-A1- 2015 147 337
- US-A1- 2016 222 102
- HOWARD SOFEN ET AL: "Guselkumab (an IL-23-specific mAb) demonstrates clinical and molecular response in patients with moderate-to-severe psoriasis", JOURNAL OF ALLERGY AND CLINICAL IMMUNOLOGY, vol. re133, no. 4, 1 April 2014 (2014-04-01), pages 1032 - 1040, XP055217623, ISSN: 0091-6749, DOI: 10.1016/j.jaci.2014.01.025
- KENNETH B. GORDON ET AL: "A Phase 2 Trial of Guselkumab versus Adalimumab for Plaque Psoriasis", THE NEW ENGLAND JOURNAL OF MEDICINE, - NEJM -, vol. 373, no. 2, 9 July 2015 (2015-07-09), US, pages 136 - 144, XP055268322, ISSN: 0028-4793, DOI: 10.1056/NEJMoa1501646
- REICH KRISTIAN ET AL: "Efficacy and safety of guselkumab, an anti-interleukin-23 monoclonal antibody, compared with adalimumab for the treatment of patients with moderate to severe psoriasis with randomized withdrawal and retreatment: Results from the phase III, double-blind, placebo- and active comparator-controlled VOYA", JOURNAL OF THE AMERICAN ACADEMY OF DERMATOLOGY, MOSBY, INC, US, vol. 76, no. 3, 2 January 2017 (2017-01-02), pages 418 - 431, XP029920483, ISSN: 0190-9622, DOI: 10.1016/J.JAAD.2016.11.042
- BLAUVELT ANDREW ET AL: "Efficacy and safety of guselkumab, an anti-interleukin-23 monoclonal antibody, compared with adalimumab for the continuous treatment of patients with moderate to severe psoriasis: Results from the phase III, double-blinded, placebo- and active comparator-controlled VOYAGE 1 trial", JOURNAL OF THE AMERICAN ACADEMY OF DERMATOLOGY, MOSBY, INC, US, vol. 76, no. 3, 2 January 2017 (2017-01-02), pages 405 - 417, XP029920482, ISSN: 0190-9622, DOI: 10.1016/J.JAAD.2016.11.041
- MIO NAKAMURA ET AL: "Guselkumab for the Treatment of Psoriasis: A Review of Phase III Trials", DERMATOLOGY AND THERAPY, vol. 7, no. 3, 21 June 2017 (2017-06-21), pages 281 - 292, XP055685360, ISSN: 2193-8210, DOI: 10.1007/s13555-017-0187-0
- NAOMI TAKAHASHI ET AL: "Efficacy comparison of ustekinumab between anti-tumor necrosis factor-[alpha] drug-na�ve and anti-tumor necrosis factor-[alpha] drug-resistant Japanese psoriasis cases", INTERNATIONAL JOURNAL OF DERMATOLOGY, WILEY-BLACKWELL PUBLISHING LTD, UK, vol. 54, no. 10, 27 May 2015 (2015-05-27), pages 1194 - 1198, XP071190858, ISSN: 0011-9059, DOI: 10.1111/IJD.12859
- FOULKES A C ET AL: "What's new in psoriasis? An analysis of guidelines and systematic reviews published in 2009-2010", CLINICAL AND EXPERIMENTAL DERMATOLOGY, BLACKWELL SCIENTIFIC PUBLICATIONS, GB, vol. 36, no. 6, 1 July 2011 (2011-07-01), pages 585 - 589, XP071607709, ISSN: 0307-6938, DOI: 10.1111/J.1365-2230.2011.04108.X

## Description

### FIELD OF THE INVENTION

The present invention concerns methods for treating psoriasis with an antibody that binds the human IL-23 protein. In particular, it relates to a method of administering the anti-IL-23 specific antibody guselkumab and specific pharmaceutical compositions of guselkumab, which is safe and effective for patients suffering from psoriasis.

### BACKGROUND OF THE INVENTION

Interleukin (IL)-12 is a secreted heterodimeric cytokine comprised of 2 disulfide-linked glycosylated protein subunits, designated p35 and p40 for their approximate molecular weights. IL-12 is produced primarily by antigen-presenting cells and drives cell-mediated immunity by binding to a two-chain receptor complex that is expressed on the surface of T cells or natural killer (NK) cells. The IL-12 receptor beta-1 (IL-12Rβ1) chain binds to the p40 subunit of IL-12, providing the primary interaction between IL-12 and its receptor. However, it is IL-12p35 ligation of the second receptor chain, IL-12Rβ2, that confers intracellular signaling (e.g. STAT4 phosphorylation) and activation of the receptor-bearing cell (Presky et al, 1996). IL-12 signaling concurrent with antigen presentation is thought to invoke T cell differentiation towards the T helper 1 (Th1) phenotype, characterized by interferon gamma (IFNy) production (Trinchieri, 2003). Th1 cells are believed to promote immunity to some intracellular pathogens, generate complement-fixing antibody isotypes, and contribute to tumor immunosurveillance. Thus, IL-12 is thought to be a significant component to host defense immune mechanisms.

It was discovered that the p40 protein subunit of IL-12 can also associate with a separate protein subunit, designated p19, to form a novel cytokine, IL-23 (Oppman et al, 2000). IL-23 also signals through a two-chain receptor complex. Since the p40 subunit is shared between IL-12 and IL-23, it follows that the IL-12Rβ1 chain is also shared between IL-12 and IL-23. However, it is the IL-23p19 ligation of the second component of the IL-23 receptor complex, IL-23R, that confers IL-23 specific intracellular signaling (e.g., STAT3 phosphorylation) and subsequent IL-17 production by T cells (Parham et al, 2002; Aggarwal et al. 2003). Recent studies have demonstrated that the biological functions of IL-23 are distinct from those of IL-12, despite the structural similarity between the two cytokines (Langrish et al, 2005).

Abnormal regulation of IL-12 and Th1 cell populations has been associated with many immune-mediated diseases since neutralization of IL-12 by antibodies is effective in treating animal models of psoriasis, multiple sclerosis (MS), rheumatoid arthritis, inflammatory bowel disease, insulin-dependent (type 1) diabetes mellitus, and uveitis (Leonard et al, 1995; Hong et al, 1999; Malfait et al, 1998; Davidson et al, 1998). However, since these studies targeted the shared p40 subunit, both IL-12 and IL-23 were neutralized *in vivo.* Therefore, it was unclear whether IL-12 or IL-23 was mediating disease, or if both cytokines needed to be inhibited to achieve disease suppression. Recent studies have confirmed through IL-23p19 deficient mice or specific antibody neutralization of IL-23 that IL-23 inhibition can provide equivalent benefit as anti-IL-12p40 strategies (Cua et al, 2003, Murphy et al, 2003, Benson et al 2004). Therefore, there is increasing evidence for the specific role of IL-23 in immune-mediated disease. Neutralization of IL-23 without inhibition of IL-12 pathways could then provide effective therapy of immune-mediated disease with limited impact on important host defense immune mechanism. This would represent a significant improvement over current therapeutic options.

Psoriasis is a common, chronic immune-mediated skin disorder with significant co-morbidities, such as psoriatic arthritis (PsA), depression, cardiovascular disease, hypertension, obesity, diabetes, metabolic syndrome, and Crohn's disease. Plaque psoriasis is the most common form of the disease and manifests in well demarcated erythematous lesions topped with white silver scales. Plaques are pruritic, painful, often disfiguring and disabling, and a significant proportion of psoriatic patients have plaques on hands/nails face, feet and genitalia. As such, psoriasis negatively impacts health-related quality of life (HRQoL) to a significant extent, including imposing physical and psychosocial burdens that extend beyond the physical dermatological symptoms and interfere with everyday activities. For example, psoriasis negatively impacts familial, spousal, social, and work relationships, and is associated with a higher incidence of depression and increased suicidal tendencies.

Histologic characterization of psoriasis lesions reveals a thickened epidermis resulting from aberrant keratinocyte proliferation and differentiation as well as dermal infiltration and co-localization of CD3+ T lymphocytes and dendritic cells. While the etiology of psoriasis is not well defined, gene and protein analysis have shown that IL-12, IL-23 and their downstream molecules are over-expressed in psoriatic lesions, and some may correlate with psoriasis disease severity. Some therapies used in the treatment of psoriasis modulate IL-12 and IL-23 levels, which is speculated to contribute to their efficacy. Th1 and Th17 cells can produce effector cytokines that induce the production of vasodilators, chemoattractants and expression of adhesion molecules on endothelial cells which in turn, promote monocyte and neutrophil recruitment, T cell infiltration, neovascularization and keratinocyte activation and hyperplasia. Activated keratinocytes can produce chemoattractant factors that promote neutrophil, monocyte, T cell, and dendritic cell trafficking, thus establishing a cycle of inflammation and keratinocyte hyperproliferation.

Elucidation of the pathogenesis of psoriasis has led to effective biologic treatments targeting tumor necrosis factor-alpha (TNF-α), both interleukin (IL)-12 and IL-23 and, most recently, IL-17 as well as IL-23 alone (including in Phase 1 and 2 clinical trials using guselkumab). Guselkumab (also known as CNTO 1959) is a fully human IgG1 lambda monoclonal antibody that binds to the p19 subunit of IL-23 and inhibits the intracellular and downstream signaling of IL-23, required for terminal differentiation of T helper (Th) 17 cells.

Sofen et al., J. Allergy Clin. Immunol 33:4 (2014) reports that guselkumab demonstrates clinical and molecular response in patients with moderate-to-sever psoriasis. Gordon et al., NEJM 373:2 (2015) describes a Phase 2 trial of guselkumab versus adalimumab for plaque psoriasis. Takahashi et al., Int. J. Dermatol., 54(10):1194-1198 (2015) compares the efficacy of ustekinumab between anti-TNFα drug naive and anti-TNFα drug resistant Japanese psoriasis cases. Foulkes et al., Clin. Exp. Dermatol. 36(6):585-589 (2011) presents an analysis of guidelines and systematic reviews on psoriasis published in 2009-2010.

### SUMMARY OF THE INVENTION

The invention provides an IL-23 antibody for use in a method of treating psoriasis in a patient that is a non responder to a TNF inhibitor, comprising administering the antibody to the patient in a safe and effective amount, wherein the antibody is guselkumab and the TNF inhibitor is adalimumab.

Any references in the description to methods of treatment refer to the compounds, pharmaceutical compositions and medicaments of the present invention for use in a method for treatment of the human (or animal) body by therapy (or for diagnosis).

In an embodiment, the anti-IL-23 specific antibody is administered at an initial dose, a dose 4 weeks thereafter, and at a dosing interval of once every 8 weeks thereafter, e,g., a dose at 0, 4, 8, 16, 24, 32, 40 and 48 weeks. In another aspect, the composition used in the method of the invention comprises a pharmaceutical composition comprising: an anti-IL-23 specific antibody in an amount from about 1.0 µg/ml to about 1000 mg/ml, specifically at 50 mg or 100 mg. In a preferred embodiment the anti-IL-23 specific antibody is guselkumab at 100 mg/mL; 7.9% (w/v) sucrose, 4.0mM Histidine, 6.9 mM L-Histidine monohydrochloride monohydrate; 0.053% (w/v) Polysorbate 80 of the pharmaceutical composition; wherein the diluent is water at standard state.

In an embodiment, the psoriasis patient achieved the endpoints of achieving an IGA score of cleared or minimal disease (IGA 0/1) and 90% improvement in PASI response (PASI 90) or 100% improvement in PASI response (PASI 100) at week 16.

### DETAILED DESCRIPTION OF THE PREFERRED EMBODIMENTS

As used herein the method of treatment of psoriasis comprises administering isolated, recombinant and/or synthetic anti-IL- 23 specific human antibodies and diagnostic and therapeutic compositions, methods and devices.

As used herein, an "anti-IL-23 specific antibody," "anti-IL-23 antibody," "antibody portion," or "antibody fragment" and/or "antibody variant" and the like include any protein or peptide containing molecule that comprises at least a portion of an immunoglobulin molecule, such as but not limited to, at least one complementarity determining region (CDR) of a heavy or light chain or a ligand binding portion thereof, a heavy chain or light chain variable region, a heavy chain or light chain constant region, a framework region, or any portion thereof, or at least one portion of an IL-23 receptor or binding protein, which can be incorporated into an antibody. Such antibody optionally further affects a specific ligand, such as but not limited to, where such antibody modulates, decreases, increases, antagonizes, agonizes, mitigates, alleviates, blocks, inhibits, abrogates and/or interferes with at least one IL-23 activity or binding, or with IL-23 receptor activity or binding, *in vitro, in* situ and/or in *vivo.* As a non-limiting example, a suitable anti-IL-23 antibody, specified portion or variant can bind at least one IL-23 molecule, or specified portions, variants or domains thereof. A suitable anti-IL-23 antibody, specified portion, or variant can also optionally affect at least one of IL-23 activity or function, such as but not limited to, RNA, DNA or protein synthesis, IL-23 release, IL-23 receptor signaling, membrane IL-23 cleavage, IL-23 activity, IL-23 production and/or synthesis.

The term "antibody" is further intended to encompass antibodies, digestion fragments, specified portions and variants thereof, including antibody mimetics or comprising portions of antibodies that mimic the structure and/or function of an antibody or specified fragment or portion thereof, including single chain antibodies and fragments thereof. Functional fragments include antigen-binding fragments that bind to a mammalian IL-23. For example, antibody fragments capable of binding to IL-23 or portions thereof, including, but not limited to, Fab (e.g., by papain digestion), Fab' (e.g., by pepsin digestion and partial reduction) and F(ab')₂ (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the term (see, e.g., Colligan, Immunology, supra).

Such fragments can be produced by enzymatic cleavage, synthetic or recombinant techniques, as known in the art and/or as described herein. Antibodies can also be produced in a variety of truncated forms using antibody genes in which one or more stop codons have been introduced upstream of the natural stop site. For example, a combination gene encoding a F(ab')₂ heavy chain portion can be designed to include DNA sequences encoding the C_{H}1 domain and/or hinge region of the heavy chain. The various portions of antibodies can be joined together chemically by conventional techniques, or can be prepared as a contiguous protein using genetic engineering techniques.

As used herein, the term "human antibody" refers to an antibody in which substantially every part of the protein (e.g., CDR, framework, C_{L}, C_{H} domains (e.g., C_{H}1, C_{H}2, C_{H}3), hinge, (V_{L}, V_{H})) is substantially non-immunogenic in humans, with only minor sequence changes or variations. A "human antibody" may also be an antibody that is derived from or closely matches human germline immunoglobulin sequences. Human antibodies may include amino acid residues not encoded by germline immunoglobulin sequences (e.g., mutations introduced by random or site-specific mutagenesis in vitro or by somatic mutation in vivo). Often, this means that the human antibody is substantially non-immunogenic in humans. Human antibodies have been classified into groupings based on their amino acid sequence similarities. Accordingly, using a sequence similarity search, an antibody with a similar linear sequence can be chosen as a template to create a human antibody. Similarly, antibodies designated primate (monkey, baboon, chimpanzee, etc.), rodent (mouse, rat, rabbit, guinea pig, hamster, and the like) and other mammals designate such species, sub-genus, genus, sub-family, and family specific antibodies. Further, chimeric antibodies can include any combination of the above. Such changes or variations optionally and preferably retain or reduce the immunogenicity in humans or other species relative to non-modified antibodies. Thus, a human antibody is distinct from a chimeric or humanized antibody.

It is pointed out that a human antibody can be produced by a non-human animal or prokaryotic or eukaryotic cell that is capable of expressing functionally rearranged human immunoglobulin (e.g., heavy chain and/or light chain) genes. Further, when a human antibody is a single chain antibody, it can comprise a linker peptide that is not found in native human antibodies. For example, an Fv can comprise a linker peptide, such as two to about eight glycine or other amino acid residues, which connects the variable region of the heavy chain and the variable region of the light chain. Such linker peptides are considered to be of human origin.

Bispecific, heterospecific, heteroconjugate or similar antibodies can also be used that are monoclonal, preferably, human or humanized, antibodies that have binding specificities for at least two different antigens. In the present case, one of the binding specificities is for at least one IL-23 protein, the other one is for any other antigen. Methods for making bispecific antibodies are known in the art. Traditionally, the recombinant production of bispecific antibodies is based on the co-expression of two immunoglobulin heavy chain-light chain pairs, where the two heavy chains have different specificities (Milstein and Cuello, Nature 305:537 (1983)). Because of the random assortment of immunoglobulin heavy and light chains, these hybridomas (quadromas) produce a potential mixture of 10 different antibody molecules, of which only one has the correct bispecific structure. The purification of the correct molecule, which is usually done by affinity chromatography steps, is rather cumbersome, and the product yields are low. Similar procedures are disclosed, e.g., in WO 93/08829, US Patent Nos, 6210668, 6193967, 6132992, 6106833, 6060285, 6037453, 6010902, 5989530, 5959084, 5959083, 5932448, 5833985, 5821333, 5807706, 5643759, 5601819, 5582996, 5496549, 4676980, WO 91/00360, WO 92/00373, EP 03089, Traunecker et al., EMBO J. 10:3655 (1991), Suresh et al., Methods in Enzymology 121:210 (1986).

Anti-IL-23 specific (also termed IL-23 specific antibodies) (or antibodies to IL-23) useful in the methods and compositions of the present invention can optionally be characterized by high affinity binding to IL-23 and, optionally and preferably, having low toxicity. In particular, an antibody of the invention, where the individual components, such as the variable region, constant region and framework, individually and/or collectively, optionally and preferably possess low immunogenicity, is useful in the present invention. The antibodies that can be used in the invention are optionally characterized by their ability to treat patients for extended periods with measurable alleviation of symptoms and low and/or acceptable toxicity. Low or acceptable immunogenicity and/or high affinity, as well as other suitable properties, can contribute to the therapeutic results achieved. "Low immunogenicity" is defined herein as raising significant HAHA, HACA or HAMA responses in less than about 75%, or preferably less than about 50% of the patients treated and/or raising low titres in the patient treated (less than about 300, preferably less than about 100 measured with a double antigen enzyme immunoassay) (Elliott et al., Lancet 344:1125-1127 (1994)). "Low immunogenicity" can also be defined as the incidence of titrable levels of antibodies to the anti-IL-23 antibody in patients treated with anti-IL-23 antibody as occurring in less than 25% of patients treated, preferably, in less than 10% of patients treated with the recommended dose for the recommended course of therapy during the treatment period.

### Utility

The isolated nucleic acids disclosed herein can be used for production of at least one anti-IL-23 antibody or specified variant thereof, which can be used to measure or effect in an cell, tissue, organ or animal (including mammals and humans), to diagnose, monitor, modulate, treat, alleviate, help prevent the incidence of, or reduce the symptoms of psoriasis.

Such a method can comprise administering an effective amount of a composition or a pharmaceutical composition comprising at least one anti-IL-23 antibody to a cell, tissue, organ, animal or patient in need of such modulation, treatment, alleviation, prevention, or reduction in symptoms, effects or mechanisms. The effective amount can comprise an amount of about 0.001 to 500 mg/kg per single (e.g., bolus), multiple or continuous administration, or to achieve a serum concentration of 0.01-5000 µg/ml serum concentration per single, multiple, or continuous administration, or any effective range or value therein, as done and determined using known methods, as described herein or known in the relevant arts.

### Citations

Ausubel, et al., ed., Current Protocols in Molecular Biology, John Wiley & Sons, Inc., NY, NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY, NY, (1997-2001).

### Antibodies of the Present Invention - Production and Generation

An anti-IL-23 antibody can be optionally produced by a cell line, a mixed cell line, an immortalized cell or clonal population of immortalized cells, as well known in the art. See, e.g., Ausubel, et al., ed., Current Protocols in Molecular Biology, John Wiley & Sons, Inc., NY, NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY, NY, (1997-2001).

A preferred anti-IL-23 antibody is guselkumab (also referred to as CNTO1959) having the heavy chain variable region amino acid sequence of SEQ ID NO: 106 and the light chain variable region amino acid sequence of SEQ ID NO: 116 and having the heavy chain CDR amino acid sequences of SEQ ID NO: 5, SEQ ID NO: 20, and SEQ ID NO: 44; and the light chain CDR amino acid sequences of SEQ ID NO: 50, SEQ ID NO: 56, and SEQ ID NO: 73. Other anti-IL-23 antibodies have sequences listed herein and are described in U.S. Patent No. 7,935,344).

Human antibodies that are specific for human IL-23 proteins or fragments thereof can be raised against an appropriate immunogenic antigen, such as an isolated IL-23 protein and/or a portion thereof (including synthetic molecules, such as synthetic peptides). Other specific or general mammalian antibodies can be similarly raised. Preparation of immunogenic antigens, and monoclonal antibody production can be performed using any suitable technique.

In one approach, a hybridoma is produced by fusing a suitable immortal cell line (e.g., a myeloma cell line, such as, but not limited to, Sp2/0, Sp2/0-AG14, NSO, NS1, NS2, AE-1, L.5, L243, P3X63Ag8.653, Sp2 SA3, Sp2 MAI, Sp2 SS1, Sp2 SA5, U937, MLA 144, ACT IV, MOLT4, DA-1, JURKAT, WEHI, K-562, COS, RAJI, NIH 3T3, HL-60, MLA 144, NAMALWA, NEURO 2A, or the like, or heteromylomas, fusion products thereof, or any cell or fusion cell derived therefrom, or any other suitable cell line as known in the art) (see, e.g., www.atcc.org, www.lifetech.com., and the like), with antibody producing cells, such as, but not limited to, isolated or cloned spleen, peripheral blood, lymph, tonsil, or other immune or B cell containing cells, or any other cells expressing heavy or light chain constant or variable or framework or CDR sequences, either as endogenous or heterologous nucleic acid, as recombinant or endogenous, viral, bacterial, algal, prokaryotic, amphibian, insect, reptilian, fish, mammalian, rodent, equine, ovine, goat, sheep, primate, eukaryotic, genomic DNA, cDNA, rDNA, mitochondrial DNA or RNA, chloroplast DNA or RNA, hnRNA, mRNA, tRNA, single, double or triple stranded, hybridized, and the like or any combination thereof. See, e.g., Ausubel, supra, and Colligan, Immunology, supra, chapter 2.

Antibody producing cells can also be obtained from the peripheral blood or, preferably, the spleen or lymph nodes, of humans or other suitable animals that have been immunized with the antigen of interest. Any other suitable host cell can also be used for expressing heterologous or endogenous nucleic acid encoding an antibody of the present invention. The fused cells (hybridomas) or recombinant cells can be isolated using selective culture conditions or other suitable known methods, and cloned by limiting dilution or cell sorting, or other known methods. Cells which produce antibodies with the desired specificity can be selected by a suitable assay (e.g., ELISA).

Other suitable methods of producing or isolating antibodies of the requisite specificity can be used, including, but not limited to, methods that select recombinant antibody from a peptide or protein library (e.g., but not limited to, a bacteriophage, ribosome, oligonucleotide, RNA, cDNA, or the like, display library; e.g., as available from Cambridge antibody Technologies, Cambridgeshire, UK; MorphoSys, Martinsreid/Planegg, DE; Biovation, Aberdeen, Scotland, UK; BioInvent, Lund, Sweden; Dyax Corp., Enzon, Affymax/Biosite; Xoma, Berkeley, CA; Ixsys. See, e.g., EP 368,684, WO92/001047; WO93/006213; WO93/011236; WO92/020791; WO93/019172; US 5,962,255; WO95/001438; WO95/015388; WO98/001757; (CAT/MRC); WO90/14443; WO90/14424; WO90/14430; WO94/018219; WO92/18619; WO96/07754; (Scripps); WO96/13583, WO97/08320 (MorphoSys); WO95/16027 (BioInvent); WO88/06630; WO90/3809 (Dyax); US 4,704,692 (Enzon); WO91/017271 (Affymax); WO89/06283; EP 371 998; EP 550 400; (Xoma); EP 229 046; WO92/006204 (Ixsys); or stochastically generated peptides or proteins - US 5723323, 5763192, 5814476, 5817483, 5824514, 5976862, WO 86/05803, EP 590 689 (Ixsys, predecessor of Applied Molecular Evolution (AME))) or that rely upon immunization of transgenic animals (e.g., SCID mice, Nguyen et al., Microbiol. Immunol. 41:901-907 (1997); Sandhu et al., Crit. Rev. Biotechnol. 16:95-118 (1996); Eren et al., Immunol. 93:154-161 (1998)) that are capable of producing a repertoire of human antibodies, as known in the art and/or as described herein. Such techniques, include, but are not limited to, ribosome display (Hanes et al., Proc. Natl. Acad. Sci. USA, 94:4937-4942 (May 1997); Hanes et al., Proc. Natl. Acad. Sci. USA, 95:14130-14135 (Nov. 1998)); single cell antibody producing technologies (e.g., selected lymphocyte antibody method ("SLAM") (US pat. No. 5,627,052, Wen et al., J. Immunol. 17:887-892 (1987); Babcook et al., Proc. Natl. Acad. Sci. USA 93:7843-7848 (1996)); gel microdroplet and flow cytometry (Powell et al., Biotechnol. 8:333-337 (1990); One Cell Systems, Cambridge, MA; Gray et al., J. Imm. Meth. 182:155-163 (1995); Kenny et al., Bio/Technol. 13:787-790 (1995)); B-cell selection (Steenbakkers et al., Molec. Biol. Reports 19:125-134 (1994); Jonak et al., Progress Biotech, Vol. 5, In Vitro Immunization in Hybridoma Technology, Borrebaeck, ed., Elsevier Science Publishers B.V., Amsterdam, Netherlands (1988)).

Methods for engineering or humanizing non-human or human antibodies can also be used and are well known in the art. Generally, a humanized or engineered antibody has one or more amino acid residues from a source that is non-human, e.g., but not limited to, mouse, rat, rabbit, non-human primate or other mammal. These non-human amino acid residues are replaced by residues often referred to as "import" residues, which are typically taken from an "import" variable, constant or other domain of a known human sequence.

Known human Ig sequences are disclosed, e.g., www.ncbi.nlm.nih.gov/entrez/query.fcgi; www.ncbi.nih.gov/igblast; www.atcc.org/phage/hdb.html; www.mrc-cpe.cam.ac.uk/ALIGNMENTS.php; www.kabatdatabase.com/top.html; ftp.ncbi.nih.gov/repository/kabat; www.sciquest.com; www.abcam.com; www.antibodyresource.com/onlinecomp.html; www.public.iastate.edu/~pedro/research_tools.html; www.whfreeman.com/immunology/CH05/kuby05.htm; www.hhmi.org/grants/lectures/1996/vlab; www.path.cam.ac.uk/~mrc7/mikeimages.html; mcb.harvard.edu/BioLinks/Immunology.html; www.immunologylink.com; pathbox.wustl.edu/-hcenter/index.html; www.appliedbiosystems.com; www.nal.usda.gov/awic/pubs/antibody; www.m.ehime-u.ac.jp/~yasuhito/Elisa.html; www.biodesign.com; www.cancerresearchuk.org; www.biotech.ufl.edu; www.isac-net.org; baserv.uci.kun.nl/~jraats/links1.html; www.recab.uni-hd.de/immuno.bme.nwu.edu; www.mrc-cpe.cam.ac.uk; www.ibt.unam.mx/vir/V_mice.html; http://www.bioinf.org.uk/abs; antibody.bath.ac.uk; www.unizh.ch; www.cryst.bbk.ac.uk/~ubcg07s; www.nimr.mrc.ac.uk/CC/ccaewg/ccaewg.html; www.path.cam.ac.uk/~mrc7/humanisation/TAHHP.html; www.ibt.unam.mx/vir/structure/stat_aim.html; www.biosci.missouri.edu/smithgp/index.html; www.jerini.de; Kabat et al., Sequences of Proteins of Immunological Interest, U.S. Dept. Health (1983).

Such imported sequences can be used to reduce immunogenicity or reduce, enhance or modify binding, affinity, on-rate, off-rate, avidity, specificity, half-life, or any other suitable characteristic, as known in the art. In general, the CDR residues are directly and most substantially involved in influencing antigen binding. Accordingly, part or all of the non-human or human CDR sequences are maintained while the non-human sequences of the variable and constant regions may be replaced with human or other amino acids.

Antibodies can also optionally be humanized or human antibodies engineered with retention of high affinity for the antigen and other favorable biological properties. To achieve this goal, humanized (or human) antibodies can be optionally prepared by a process of analysis of the parental sequences and various conceptual humanized products using three-dimensional models of the parental and humanized sequences. Three-dimensional immunoglobulin models are commonly available and are familiar to those skilled in the art. Computer programs are available which illustrate and display probable three-dimensional conformational structures of selected candidate immunoglobulin sequences. Inspection of these displays permits analysis of the likely role of the residues in the functioning of the candidate immunoglobulin sequence, i.e., the analysis of residues that influence the ability of the candidate immunoglobulin to bind its antigen. In this way, framework (FR) residues can be selected and combined from the consensus and import sequences so that the desired antibody characteristic, such as increased affinity for the target antigen(s), is achieved.

In addition, the human IL-23 specific antibody may comprise a human germline light chain framework. The light chain germline sequence may be selected from human VK sequences including, but not limited to, A1, A10, A11, A14, A17, A18, A19, A2, A20, A23, A26, A27, A3, A30, A5, A7, B2, B3, L1, L10, L11, L12, L14, L15, L16, L18, L19, L2, L20, L22, L23, L24, L25, L4/18a, L5, L6, L8, L9, O1, O11, O12, O14, O18, O2, O4, and O8. Sometimes, this light chain human germline framework is selected from V1-11, V1-13, V1-16, V1-17, V1-18, V1-19, V1-2, V1-20, V1-22, V1-3, V1-4, V1-5, V1-7, V1-9, V2-1, V2-11, V2-13, V2-14, V2-15, V2-17, V2-19, V2-6, V2-7, V2-8, V3-2, V3-3, V3-4, V4-1, V4-2, V4-3, V4-4, V4-6, V5-1, V5-2, V5-4, and V5-6.

In other instances, the human IL-23 specific antibody may comprise a human germline heavy chain framework. Sometimes, this heavy chain human germline framework is selected from VH1-18, VH1-2, VH1-24, VH1-3, VH1-45, VH1-46, VH1-58, VH1-69, VH1-8, VH2-26, VH2-5, VH2-70, VH3-11, VH3-13, VH3-15, VH3-16, VH3-20, VH3-21, VH3-23, VH3-30, VH3-33, VH3-35, VH3-38, VH3-43, VH3-48, VH3-49, VH3-53, VH3-64, VH3-66, VH3-7, VH3-72, VH3-73, VH3-74, VH3-9, VH4-28, VH4-31, VH4-34, VH4-39, VH4-4, VH4-59, VH4-61, VH5-51, VH6-1, and VH7-81.

In particular embodiments, the light chain variable region and/or heavy chain variable region comprises a framework region or at least a portion of a framework region (e.g., containing 2 or 3 subregions, such as FR2 and FR3). In certain embodiments, at least FRL1, FRL2, FRL3, or FRL4 is fully human. In other embodiments, at least FRH1, FRH2, FRH3, or FRH4 is fully human. In some embodiments, at least FRL1, FRL2, FRL3, or FRL4 is a germline sequence (e.g., human germline) or comprises human consensus sequences for the particular framework (readily available at the sources of known human Ig sequences described above). In other embodiments, at least FRH1, FRH2, FRH3, or FRH4 is a germline sequence (e.g., human germline) or comprises human consensus sequences for the particular framework. In preferred embodiments, the framework region is a fully human framework region.

Humanization or engineering of antibodies can be performed using any known method, such as but not limited to those described in, Winter (Jones et al., Nature 321:522 (1986); Riechmann et al., Nature 332:323 (1988); Verhoeyen et al., Science 239:1534 (1988)), Sims et al., J. Immunol. 151: 2296 (1993); Chothia and Lesk, J. Mol. Biol. 196:901 (1987), Carter et al., Proc. Natl. Acad. Sci. U.S.A. 89:4285 (1992); Presta et al., J. Immunol. 151:2623 (1993), US Patent Nos: 5723323, 5976862, 5824514, 5817483, 5814476, 5763192, 5723323, 5,766886, 5714352, 6204023, 6180370, 5693762, 5530101, 5585089, 5225539; 4816567, WO99/006834, WO97/020032, WO92/011272, WO92/003461, WO94/018219, WO90/005144, WO92/001047, WO93/006213, WO90/14443, WO90/14424, WO90/14430, EP 229246.

Sometimes, a disclosed antibody comprises an altered (e.g., mutated) Fc region. For example, sometimes the Fc region has been altered to reduce or enhance the effector functions of the antibody. Sometimes, the Fc region is an isotype selected from IgM, IgA, IgG, IgE, or other isotype. Alternatively or additionally, it may be useful to combine amino acid modifications with one or more further amino acid modifications that alter C1q binding and/or the complement dependent cytotoxicity function of the Fc region of an IL-23 binding molecule. The starting polypeptide of particular interest may be one that binds to C1q and displays complement dependent cytotoxicity (CDC). Polypeptides with pre-existing C1q binding activity, optionally further having the ability to mediate CDC may be modified such that one or both of these activities are enhanced. Amino acid modifications that alter C1q and/or modify its complement dependent cytotoxicity function are described, for example, in WO0042072.

As disclosed above, one can design an Fc region of a human IL-23 specific antibody with altered effector function, e.g., by modifying C1q binding and/or FcyR binding and thereby changing complement dependent cytotoxicity (CDC) activity and/or antibody-dependent cell-mediated cytotoxicity (ADCC) activity. "Effector functions" are responsible for activating or diminishing a biological activity (e.g., in a subject). Examples of effector functions include, but are not limited to: C1q binding; CDC; Fc receptor binding; ADCC; phagocytosis; down regulation of cell surface receptors (e.g., B cell receptor; BCR), etc. Such effector functions may require the Fc region to be combined with a binding domain (e.g., an antibody variable domain) and can be assessed using various assays (e.g., Fc binding assays, ADCC assays, CDC assays, etc.).

For example, one can generate a variant Fc region of the human IL-23 (or anti-IL-23) antibody with improved C1q binding and improved FcyRIIIbinding (e.g., having both improved ADCC activity and improved CDC activity). Alternatively, if it is desired that effector function be reduced or ablated, a variant Fc region can be engineered with reduced CDC activity and/or reduced ADCC activity. Sometimes, only one of these activities may be increased, and, optionally, also the other activity reduced (e.g., to generate an Fc region variant with improved ADCC activity, but reduced CDC activity and vice versa).

Fc mutations can also be introduced in engineer to alter their interaction with the neonatal Fc receptor (FcRn) and improve their pharmacokinetic properties. A collection of human Fc variants with improved binding to the FcRn have been described (Shields et al., (2001). High resolution mapping of the binding site on human IgG1 for FcyRI, FcyRII, FcγRIII, and FcRn and design of IgG1 variants with improved binding to the FcyR, J. Biol. Chem. 276:6591-6604).

Another type of amino acid substitution serves to alter the glycosylation pattern of the Fc region of the human IL-23 specific antibody. Glycosylation of an Fc region is typically either N-linked or O-linked. N-linked refers to the attachment of the carbohydrate moiety to the side chain of an asparagine residue. O-linked glycosylation refers to the attachment of one of the sugars N-aceylgalactosamine, galactose, or xylose to a hydroxyamino acid, most commonly serine or threonine, although 5-hydroxyproline or 5-hydroxylysine may also be used. The recognition sequences for enzymatic attachment of the carbohydrate moiety to the asparagine side chain peptide sequences are asparagine-X-serine and asparagine-X-threonine, where X is any amino acid except proline. Thus, the presence of either of these peptide sequences in a polypeptide creates a potential glycosylation site.

The glycosylation pattern may be altered, for example, by deleting one or more glycosylation site(s) found in the polypeptide, and/or adding one or more glycosylation sites that are not present in the polypeptide. Addition of glycosylation sites to the Fc region of a human IL-23 specific antibody is conveniently accomplished by altering the amino acid sequence such that it contains one or more of the above-described tripeptide sequences (for N-linked glycosylation sites). An exemplary glycosylation variant has an amino acid substitution of residue Asn 297 of the heavy chain. The alteration may also be made by the addition of, or substitution by, one or more serine or threonine residues to the sequence of the original polypeptide (for O-linked glycosylation sites). Additionally, a change of Asn 297 to Ala can remove one of the glycosylation sites.

In certain embodiments, the human IL-23 specific antibody of the present invention is expressed in cells that express beta (1,4)-N-acetylglucosaminyltransferase III (GnT III), such that GnT III adds GlcNAc to the human IL-23 antibody. Methods for producing antibodies in such a fashion are provided in WO/9954342, WO/03011878, patent publication 20030003097A1, and Umana et al., Nature Biotechnology, 17:176-180, Feb. 1999.

The anti-IL-23 antibody can also be optionally generated by immunization of a transgenic animal (e.g., mouse, rat, hamster, non-human primate, and the like) capable of producing a repertoire of human antibodies, as described herein and/or as known in the art. Cells that produce a human anti-IL-23 antibody can be isolated from such animals and immortalized using suitable methods, such as the methods described herein.

Transgenic mice that can produce a repertoire of human antibodies that bind to human antigens can be produced by known methods (e.g., but not limited to, U.S. Pat. Nos: 5,770,428, 5,569,825, 5,545,806, 5,625,126, 5,625,825, 5,633,425, 5,661,016 and 5,789,650 issued to Lonberg et al.; Jakobovits et al. WO 98/50433, Jakobovits et al. WO 98/24893, Lonberg et al. WO 98/24884, Lonberg et al. WO 97/13852, Lonberg et al. WO 94/25585, Kucherlapate et al. WO 96/34096, Kucherlapate et al. EP 0463 151 B1, Kucherlapate et al. EP 0710 719 A1, Surani et al. US. Pat. No. 5,545,807, Bruggemann et al. WO 90/04036, Bruggemann et al. EP 0438 474 B1, Lonberg et al. EP 0814 259 A2, Lonberg et al. GB 2 272 440 A, Lonberg et al. Nature 368:856-859 (1994), Taylor et al., Int. Immunol. 6(4)579-591 (1994), Green et al, Nature Genetics 7:13-21 (1994), Mendez et al., Nature Genetics 15:146-156 (1997), Taylor et al., Nucleic Acids Research 20(23):6287-6295 (1992), Tuaillon et al., Proc Natl Acad Sci USA 90(8)3720-3724 (1993), Lonberg et al., Int Rev Immunol 13(1):65-93 (1995) and Fishwald et al., Nat Biotechnol 14(7):845-851 (1996)). Generally, these mice comprise at least one transgene comprising DNA from at least one human immunoglobulin locus that is functionally rearranged, or which can undergo functional rearrangement. The endogenous immunoglobulin loci in such mice can be disrupted or deleted to eliminate the capacity of the animal to produce antibodies encoded by endogenous genes.

Screening antibodies for specific binding to similar proteins or fragments can be conveniently achieved using peptide display libraries. This method involves the screening of large collections of peptides for individual members having the desired function or structure. Antibody screening of peptide display libraries is well known in the art. The displayed peptide sequences can be from 3 to 5000 or more amino acids in length, frequently from 5-100 amino acids long, and often from about 8 to 25 amino acids long. In addition to direct chemical synthetic methods for generating peptide libraries, several recombinant DNA methods have been described. One type involves the display of a peptide sequence on the surface of a bacteriophage or cell. Each bacteriophage or cell contains the nucleotide sequence encoding the particular displayed peptide sequence. Such methods are described in PCT Patent Publication Nos. 91/17271, 91/18980, 91/19818, and 93/08278.

Other systems for generating libraries of peptides have aspects of both in vitro chemical synthesis and recombinant methods. See, PCT Patent Publication Nos. 92/05258, 92/14843, and 96/19256. See also, U.S. Patent Nos. 5,658,754; and 5,643,768. Peptide display libraries, vector, and screening kits are commercially available from such suppliers as Invitrogen (Carlsbad, CA), and Cambridge antibody Technologies (Cambridgeshire, UK). See, e.g., U.S. Pat. Nos. 4704692, 4939666, 4946778, 5260203, 5455030, 5518889, 5534621, 5656730, 5763733, 5767260, 5856456, assigned to Enzon; 5223409, 5403484, 5571698, 5837500, assigned to Dyax, 5427908, 5580717, assigned to Affymax; 5885793, assigned to Cambridge antibody Technologies; 5750373, assigned to Genentech, 5618920, 5595898, 5576195, 5698435, 5693493, 5698417, assigned to Xoma, Colligan, supra; Ausubel, *supra;* or Sambrook, *supra.*

Antibodies used in the method of the present invention can also be prepared using at least one anti-IL23 antibody encoding nucleic acid to provide transgenic animals or mammals, such as goats, cows, horses, sheep, rabbits, and the like, that produce such antibodies in their milk. Such animals can be provided using known methods. See, e.g., but not limited to, US Patent Nos. 5,827,690; 5,849,992; 4,873,316; 5,849,992; 5,994,616; 5,565,362; 5,304,489, and the like.

Antibodies used in the method of the present invention can additionally be prepared using at least one anti-IL23 antibody encoding nucleic acid to provide transgenic plants and cultured plant cells (e.g., but not limited to, tobacco and maize) that produce such antibodies, specified portions or variants in the plant parts or in cells cultured therefrom. As a non-limiting example, transgenic tobacco leaves expressing recombinant proteins have been successfully used to provide large amounts of recombinant proteins, e.g., using an inducible promoter. See, e.g., Cramer et al., Curr. Top. Microbol. Immunol. 240:95-118 (1999) and references cited therein. Also, transgenic maize have been used to express mammalian proteins at commercial production levels, with biological activities equivalent to those produced in other recombinant systems or purified from natural sources. See, e.g., Hood et al., Adv. Exp. Med. Biol. 464:127-147 (1999) and references cited therein. Antibodies have also been produced in large amounts from transgenic plant seeds including antibody fragments, such as single chain antibodies (scFv's), including tobacco seeds and potato tubers. See, e.g., Conrad et al., Plant Mol. Biol. 38:101-109 (1998) and references cited therein. Thus, antibodies of the present invention can also be produced using transgenic plants, according to known methods. See also, e.g., Fischer et al., Biotechnol. Appl. Biochem. 30:99-108 (Oct., 1999), Ma et al., Trends Biotechnol. 13:522-7 (1995); Ma et al., Plant Physiol. 109:341-6 (1995); Whitelam et al., Biochem. Soc. Trans. 22:940-944 (1994); and references cited therein.

Antibodies can bind human IL-23 with a wide range of affinities (K_{D}). In a preferred embodiment, a human mAb can optionally bind human IL-23 with high affinity. For example, a human mAb can bind human IL-23 with a K_{D} equal to or less than about 10⁻⁷ M, such as but not limited to, 0.1-9.9 (or any range or value therein) X 10⁻⁷, 10⁻⁸, 10⁻⁹, 10⁻¹⁰, 10⁻¹¹, 10⁻¹², 10⁻¹³ or any range or value therein.

The affinity or avidity of an antibody for an antigen can be determined experimentally using any suitable method. (See, for example, Berzofsky, et al., "Antibody-Antigen Interactions," In Fundamental Immunology, Paul, W. E., Ed., Raven Press: New York, NY (1984); Kuby, Janis Immunology, W. H. Freeman and Company: New York, NY (1992); and methods described herein). The measured affinity of a particular antibody-antigen interaction can vary if measured under different conditions (e.g., salt concentration, pH). Thus, measurements of affinity and other antigen-binding parameters (e.g., K_{D}, Kₐ, K_{d}) are preferably made with standardized solutions of antibody and antigen, and a standardized buffer, such as the buffer described herein.

### Nucleic Acid Molecules

Using the information provided herein, for example, the nucleotide sequences encoding at least 70-100% of the contiguous amino acids of at least one of the light or heavy chain variable or CDR regions described herein, among other sequences disclosed herein, specified fragments, variants or consensus sequences thereof, or a deposited vector comprising at least one of these sequences, a nucleic acid molecule of the present disclosure encoding at least one anti-IL-23 antibody can be obtained using methods described herein or as known in the art.

Nucleic acid molecules can be in the form of RNA, such as mRNA, hnRNA, tRNA or any other form, or in the form of DNA, including, but not limited to, cDNA and genomic DNA obtained by cloning or produced synthetically, or any combinations thereof. The DNA can be triple-stranded, double-stranded or single-stranded, or any combination thereof. Any portion of at least one strand of the DNA or RNA can be the coding strand, also known as the sense strand, or it can be the non-coding strand, also referred to as the anti-sense strand.

Isolated nucleic acid molecules used in the method of the present disclosure can include nucleic acid molecules comprising an open reading frame (ORF), optionally, with one or more introns, e.g., but not limited to, at least one specified portion of at least one CDR, such as CDR1, CDR2 and/or CDR3 of at least one heavy chain or light chain; nucleic acid molecules comprising the coding sequence for an anti-IL-23 antibody or variable region; and nucleic acid molecules which comprise a nucleotide sequence substantially different from those described above but which, due to the degeneracy of the genetic code, still encode at least one anti-IL-23 antibody as described herein and/or as known in the art. Of course, the genetic code is well known in the art. Thus, it would be routine for one skilled in the art to generate such degenerate nucleic acid variants that code for specific anti-IL-23 antibodies used in the method of the present invention. See, e.g., Ausubel, et al., *supra.* Non-limiting examples of isolated nucleic acid molecules include nucleic acids encoding HC CDR1, HC CDR2, HC CDR3, LC CDR1, LC CDR2, and LC CDR3, respectively.

As indicated herein, nucleic acid molecules which comprise a nucleic acid encoding an anti-IL-23 antibody can include, but are not limited to, those encoding the amino acid sequence of an antibody fragment, by itself; the coding sequence for the entire antibody or a portion thereof; the coding sequence for an antibody, fragment or portion, as well as additional sequences, such as the coding sequence of at least one signal leader or fusion peptide, with or without the aforementioned additional coding sequences, such as at least one intron, together with additional, non-coding sequences, including but not limited to, non-coding 5' and 3' sequences, such as the transcribed, non-translated sequences that play a role in transcription, mRNA processing, including splicing and polyadenylation signals (for example, ribosome binding and stability of mRNA); an additional coding sequence that codes for additional amino acids, such as those that provide additional functionalities. Thus, the sequence encoding an antibody can be fused to a marker sequence, such as a sequence encoding a peptide that facilitates purification of the fused antibody comprising an antibody fragment or portion.

### Polynucleotides Selectively Hybridizing to a Polynucleotide as Described Herein

The method of the present disclosure uses isolated nucleic acids that hybridize under selective hybridization conditions to a polynucleotide disclosed herein. Thus, these polynucleotides can be used for isolating, detecting, and/or quantifying nucleic acids comprising such polynucleotides. For example, polynucleotides can be used to identify, isolate, or amplify partial or full-length clones in a deposited library. Sometimes, the polynucleotides are genomic or cDNA sequences isolated, or otherwise complementary to, a cDNA from a human or mammalian nucleic acid library.

Preferably, the cDNA library comprises at least 80% full-length sequences, preferably, at least 85% or 90% full-length sequences, and, more preferably, at least 95% full-length sequences. The cDNA libraries can be normalized to increase the representation of rare sequences. Low or moderate stringency hybridization conditions are typically, but not exclusively, employed with sequences having a reduced sequence identity relative to complementary sequences. Moderate and high stringency conditions can optionally be employed for sequences of greater identity. Low stringency conditions allow selective hybridization of sequences having about 70% sequence identity and can be employed to identify orthologous or paralogous sequences.

Optionally, polynucleotides will encode at least a portion of an antibody. The polynucleotides embrace nucleic acid sequences that can be employed for selective hybridization to a polynucleotide encoding an antibody of the present invention. See, e.g., Ausubel, supra; Colligan, supra.

### Construction of Nucleic Acids

The isolated nucleic acids can be made using (a) recombinant methods, (b) synthetic techniques, (c) purification techniques, and/or (d) combinations thereof, as well-known in the art.

The nucleic acids can conveniently comprise sequences in addition to a polynucleotide of the present disclosure. For example, a multi-cloning site comprising one or more endonuclease restriction sites can be inserted into the nucleic acid to aid in isolation of the polynucleotide. Also, translatable sequences can be inserted to aid in the isolation of the translated polynucleotide of the present disclosure. For example, a hexa-histidine marker sequence provides a convenient means to purify the proteins of the present disclosure. The nucleic acid of the present disclosure, excluding the coding sequence, is optionally a vector, adapter, or linker for cloning and/or expression of a polynucleotide of the present disclosure.

Additional sequences can be added to such cloning and/or expression sequences to optimize their function in cloning and/or expression, to aid in isolation of the polynucleotide, or to improve the introduction of the polynucleotide into a cell. Use of cloning vectors, expression vectors, adapters, and linkers is well known in the art. (See, e.g., Ausubel, *supra;* or Sambrook, *supra*)

### Recombinant Methods for Constructing Nucleic Acids

The isolated nucleic acid compositions, such as RNA, cDNA, genomic DNA, or any combination thereof, can be obtained from biological sources using any number of cloning methodologies known to those of skill in the art. Sometimes, oligonucleotide probes that selectively hybridize, under stringent conditions, to the polynucleotides of the present disclosure are used to identify the desired sequence in a cDNA or genomic DNA library. The isolation of RNA, and construction of cDNA and genomic libraries, are well known to those of ordinary skill in the art. (See, e.g., Ausubel, *supra;* or Sambrook, *supra*)

### Nucleic Acid Screening and Isolation Methods

A cDNA or genomic library can be screened using a probe based upon the sequence of a polynucleotide used in the method of the present disclosure, such as those disclosed herein. Probes can be used to hybridize with genomic DNA or cDNA sequences to isolate homologous genes in the same or different organisms. Those of skill in the art will appreciate that various degrees of stringency of hybridization can be employed in the assay; and either the hybridization or the wash medium can be stringent. As the conditions for hybridization become more stringent, there must be a greater degree of complementarity between the probe and the target for duplex formation to occur. The degree of stringency can be controlled by one or more of temperature, ionic strength, pH and the presence of a partially denaturing solvent, such as formamide. For example, the stringency of hybridization is conveniently varied by changing the polarity of the reactant solution through, for example, manipulation of the concentration of formamide within the range of 0% to 50%. The degree of complementarity (sequence identity) required for detectable binding will vary in accordance with the stringency of the hybridization medium and/or wash medium. The degree of complementarity will optimally be 100%, or 70-100%, or any range or value therein. However, it should be understood that minor sequence variations in the probes and primers can be compensated for by reducing the stringency of the hybridization and/or wash medium.

Methods of amplification of RNA or DNA are well known in the art and can be used according to the present disclosure without undue experimentation, based on the teaching and guidance presented herein.

Known methods of DNA or RNA amplification include, but are not limited to, polymerase chain reaction (PCR) and related amplification processes (see, e.g., U.S. Patent Nos. 4,683,195, 4,683,202, 4,800,159, 4,965,188, to Mullis, et al.; 4,795,699 and 4,921,794 to Tabor, et al; 5,142,033 to Innis; 5,122,464 to Wilson, et al.; 5,091,310 to Innis; 5,066,584 to Gyllensten, et al; 4,889,818 to Gelfand, et al; 4,994,370 to Silver, et al; 4,766,067 to Biswas; 4,656,134 to Ringold) and RNA mediated amplification that uses anti-sense RNA to the target sequence as a template for double-stranded DNA synthesis (U.S. Patent No. 5,130,238 to Malek, et al, with the tradename NASBA). (See, e.g., Ausubel, *supra;* or Sambrook, *supra*.)

For instance, polymerase chain reaction (PCR) technology can be used to amplify the sequences of polynucleotides used in the method of the present disclosure and related genes directly from genomic DNA or cDNA libraries. PCR and other in vitro amplification methods can also be useful, for example, to clone nucleic acid sequences that code for proteins to be expressed, to make nucleic acids to use as probes for detecting the presence of the desired mRNA in samples, for nucleic acid sequencing, or for other purposes. Examples of techniques sufficient to direct persons of skill through in vitro amplification methods are found in Berger, supra, Sambrook, supra, and Ausubel, supra, as well as Mullis, et al., U.S. Patent No. 4,683,202 (1987); and Innis, et al., PCR Protocols A Guide to Methods and Applications, Eds., Academic Press Inc., San Diego, CA (1990). Commercially available kits for genomic PCR amplification are known in the art. See, e.g., Advantage-GC Genomic PCR Kit (Clontech). Additionally, e.g., the T4 gene 32 protein (Boehringer Mannheim) can be used to improve yield of long PCR products.

### Synthetic Methods for Constructing Nucleic Acids

The isolated nucleic acids used in the method of the present disclosure can also be prepared by direct chemical synthesis by known methods (see, e.g., Ausubel, et al., supra). Chemical synthesis generally produces a single-stranded oligonucleotide, which can be converted into double-stranded DNA by hybridization with a complementary sequence, or by polymerization with a DNA polymerase using the single strand as a template. One of skill in the art will recognize that while chemical synthesis of DNA can be limited to sequences of about 100 or more bases, longer sequences can be obtained by the ligation of shorter sequences.

### Recombinant Expression Cassettes

The present disclosure uses recombinant expression cassettes comprising a nucleic acid. A nucleic acid sequence, for example, a cDNA or a genomic sequence encoding an antibody used in the method of the present disclosure, can be used to construct a recombinant expression cassette that can be introduced into at least one desired host cell. A recombinant expression cassette will typically comprise a polynucleotide operably linked to transcriptional initiation regulatory sequences that will direct the transcription of the polynucleotide in the intended host cell. Both heterologous and non-heterologous (i.e., endogenous) promoters can be employed to direct expression of the nucleic acids.

Sometimes, isolated nucleic acids that serve as promoter, enhancer, or other elements can be introduced in the appropriate position (upstream, downstream or in the intron) of a non-heterologous form of a polynucleotide of the present disclosure so as to up or down regulate expression of a polynucleotide. For example, endogenous promoters can be altered *in vivo* or *in vitro* by mutation, deletion and/or substitution.

### Vectors and Host Cells

The present disclosure also relates to vectors that include isolated nucleic acid molecules, host cells that are genetically engineered with the recombinant vectors, and the production of at least one anti-IL-23 antibody by recombinant techniques, as is well known in the art. See, e.g., Sambrook, et al., supra; Ausubel, et al., supra.

The polynucleotides can optionally be joined to a vector containing a selectable marker for propagation in a host. Generally, a plasmid vector is introduced in a precipitate, such as a calcium phosphate precipitate, or in a complex with a charged lipid. If the vector is a virus, it can be packaged in vitro using an appropriate packaging cell line and then transduced into host cells.

The DNA insert should be operatively linked to an appropriate promoter. The expression constructs will further contain sites for transcription initiation, termination and, in the transcribed region, a ribosome binding site for translation. The coding portion of the mature transcripts expressed by the constructs will preferably include a translation initiating at the beginning and a termination codon (e.g., UAA, UGA or UAG) appropriately positioned at the end of the mRNA to be translated, with UAA and UAG preferred for mammalian or eukaryotic cell expression.

Expression vectors will preferably but optionally include at least one selectable marker. Such markers include, e.g., but are not limited to, methotrexate (MTX), dihydrofolate reductase (DHFR, US Pat.Nos. 4,399,216; 4,634,665; 4,656,134; 4,956,288; 5,149,636; 5,179,017, ampicillin, neomycin (G418), mycophenolic acid, or glutamine synthetase (GS, US Pat.Nos. 5,122,464; 5,770,359; 5,827,739) resistance for eukaryotic cell culture, and tetracycline or ampicillin resistance genes for culturing in *E. coli* and other bacteria or prokaryotics. Appropriate culture mediums and conditions for the above-described host cells are known in the art. Suitable vectors will be readily apparent to the skilled artisan. Introduction of a vector construct into a host cell can be effected by calcium phosphate transfection, DEAE-dextran mediated transfection, cationic lipid-mediated transfection, electroporation, transduction, infection or other known methods. Such methods are described in the art, such as Sambrook, supra, Chapters 1-4 and 16-18; Ausubel, supra, Chapters 1, 9, 13, 15, 16.

At least one antibody disclosed herein can be expressed in a modified form, such as a fusion protein, and can include not only secretion signals, but also additional heterologous functional regions. For instance, a region of additional amino acids, particularly charged amino acids, can be added to the N-terminus of an antibody to improve stability and persistence in the host cell, during purification, or during subsequent handling and storage. Also, peptide moieties can be added to an antibody disclosed herein to facilitate purification. Such regions can be removed prior to final preparation of an antibody or at least one fragment thereof. Such methods are described in many standard laboratory manuals, such as Sambrook, supra, Chapters 17.29-17.42 and 18.1-18.74; Ausubel, supra, Chapters 16, 17 and 18.

Those of ordinary skill in the art are knowledgeable in the numerous expression systems available for expression of a nucleic acid encoding a protein used in the method of the present invention. Alternatively, nucleic acids can be expressed in a host cell by turning on (by manipulation) in a host cell that contains endogenous DNA encoding an antibody. Such methods are well known in the art, e.g., as described in US patent Nos. 5,580,734, 5,641,670, 5,733,746, and 5,733,761.

Illustrative of cell cultures useful for the production of the antibodies, specified portions or variants thereof, are mammalian cells. Mammalian cell systems often will be in the form of monolayers of cells although mammalian cell suspensions or bioreactors can also be used. A number of suitable host cell lines capable of expressing intact glycosylated proteins have been developed in the art, and include the COS-1 (e.g., ATCC CRL 1650), COS-7 (e.g., ATCC CRL-1651), HEK293, BHK21 (e.g., ATCC CRL-10), CHO (e.g., ATCC CRL 1610) and BSC-1 (e.g., ATCC CRL-26) cell lines, Cos-7 cells, CHO cells, hep G2 cells, P3X63Ag8.653, SP2/0-Ag14, 293 cells, HeLa cells and the like, which are readily available from, for example, American Type Culture Collection, Manassas, Va (www.atcc.org). Preferred host cells include cells of lymphoid origin, such as myeloma and lymphoma cells. Particularly preferred host cells are P3X63Ag8.653 cells (ATCC Accession Number CRL-1580) and SP2/0-Ag14 cells (ATCC Accession Number CRL-1851). In a particularly preferred embodiment, the recombinant cell is a P3X63Ab8.653 or a SP2/0-Ag14 cell.

Expression vectors for these cells can include one or more of the following expression control sequences, such as, but not limited to, an origin of replication; a promoter (e.g., late or early SV40 promoters, the CMV promoter (US Pat.Nos. 5,168,062; 5,385,839), an HSV tk promoter, a pgk (phosphoglycerate kinase) promoter, an EF-1 alpha promoter (US Pat.No. 5,266,491), at least one human immunoglobulin promoter; an enhancer, and/or processing information sites, such as ribosome binding sites, RNA splice sites, polyadenylation sites (e.g., an SV40 large T Ag poly A addition site), and transcriptional terminator sequences. See, e.g., Ausubel et al., supra; Sambrook, et al., supra. Other cells useful for production of nucleic acids or proteins of the present invention are known and/or available, for instance, from the American Type Culture Collection Catalogue of Cell Lines and Hybridomas (www.atcc.org) or other known or commercial sources.

When eukaryotic host cells are employed, polyadenlyation or transcription terminator sequences are typically incorporated into the vector. An example of a terminator sequence is the polyadenlyation sequence from the bovine growth hormone gene. Sequences for accurate splicing of the transcript can also be included. An example of a splicing sequence is the VP1 intron from SV40 (Sprague, et al., J. Virol. 45:773-781 (1983)). Additionally, gene sequences to control replication in the host cell can be incorporated into the vector, as known in the art.

### Purification of an Antibody

An anti-IL-23 antibody can be recovered and purified from recombinant cell cultures by well-known methods including, but not limited to, protein A purification, ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. High performance liquid chromatography ("HPLC") can also be employed for purification. See, e.g., Colligan, Current Protocols in Immunology, or Current Protocols in Protein Science, John Wiley & Sons, NY, NY, (1997-2001), e.g., Chapters 1, 4, 6, 8, 9, 10.

Antibodies used in the method of the present invention include naturally purified products, products of chemical synthetic procedures, and products produced by recombinant techniques from a eukaryotic host, including, for example, yeast, higher plant, insect and mammalian cells. Depending upon the host employed in a recombinant production procedure, the antibody can be glycosylated or can be non-glycosylated, with glycosylated preferred. Such methods are described in many standard laboratory manuals, such as Sambrook, supra, Sections 17.37-17.42; Ausubel, supra, Chapters 10, 12, 13, 16, 18 and 20, Colligan, Protein Science, supra, Chapters 12-14.

### Anti-IL-23 Antibodies.

An anti-IL-23 antibody according to the present disclosure includes any protein or peptide containing molecule that comprises at least a portion of an immunoglobulin molecule, such as but not limited to, at least one ligand binding portion (LBP), such as but not limited to, a complementarity determining region (CDR) of a heavy or light chain or a ligand binding portion thereof, a heavy chain or light chain variable region, a framework region (e.g., FR1, FR2, FR3, FR4 or fragment thereof, further optionally comprising at least one substitution, insertion or deletion), a heavy chain or light chain constant region, (e.g., comprising at least one C_{H}1, hinge1, hinge2, hinge3, hinge4, C_{H}2, or C_{H}3 or fragment thereof, further optionally comprising at least one substitution, insertion or deletion), or any portion thereof, that can be incorporated into an antibody. An antibody can include or be derived from any mammal, such as but not limited to, a human, a mouse, a rabbit, a rat, a rodent, a primate, or any combination thereof, and the like.

The isolated antibodies used in the method of the present disclosure comprise the antibody amino acid sequences disclosed herein encoded by any suitable polynucleotide, or any isolated or prepared antibody. Preferably, the human antibody or antigen-binding fragment binds human IL-23 and, thereby, partially or substantially neutralizes at least one biological activity of the protein. An antibody, or specified portion or variant thereof, that partially or preferably substantially neutralizes at least one biological activity of at least one IL-23 protein or fragment can bind the protein or fragment and thereby inhibit activities mediated through the binding of IL-23 to the IL-23 receptor or through other IL-23-dependent or mediated mechanisms. As used herein, the term "neutralizing antibody" refers to an antibody that can inhibit an IL-23-dependent activity by about 20-120%, preferably by at least about 10, 20, 30, 40, 50, 55, 60, 65, 70, 75, 80, 85, 90, 91, 92, 93, 94, 95, 96, 97, 98, 99, 100% or more depending on the assay. The capacity of an anti-IL-23 antibody to inhibit an IL-23-dependent activity is preferably assessed by at least one suitable IL-23 protein or receptor assay, as described herein and/or as known in the art. A human antibody can be of any class (IgG, IgA, IgM, IgE, IgD, etc.) or isotype and can comprise a kappa or lambda light chain. Sometimes, the human antibody comprises an IgG heavy chain or defined fragment, for example, at least one of isotypes, IgG1, IgG2, IgG3 or IgG4 (e.g., γ1, y2, y3, y4). Antibodies of this type can be prepared by employing a transgenic mouse or other trangenic non-human mammal comprising at least one human light chain (e.g., IgG, IgA, and IgM) transgenes as described herein and/or as known in the art. In one embodiment, the anti-IL-23 human antibody comprises an IgG1 heavy chain.

An antibody binds at least one specified epitope specific to at least one IL-23 protein, subunit, fragment, portion or any combination thereof. The at least one epitope can comprise at least one antibody binding region that comprises at least one portion of the protein, which epitope is preferably comprised of at least one extracellular, soluble, hydrophillic, external or cytoplasmic portion of the protein.

Generally, the human antibody or antigen-binding fragment will comprise an antigen-binding region that comprises at least one human complementarity determining region (CDR1, CDR2 and CDR3) or variant of at least one heavy chain variable region and at least one human complementarity determining region (CDR1, CDR2 and CDR3) or variant of at least one light chain variable region. The CDR sequences may be derived from human germline sequences or closely match the germline sequences. For example, the CDRs from a synthetic library derived from the original non-human CDRs can be used. These CDRs may be formed by incorporation of conservative substitutions from the original non-human sequence. Sometimes, the antibody or antigen-binding portion or variant can have an antigen-binding region that comprises at least a portion of at least one light chain CDR (i.e., CDR1, CDR2 and/or CDR3) having the amino acid sequence of the corresponding CDRs 1, 2 and/or 3.

Such antibodies can be prepared by chemically joining together the various portions (e.g., CDRs, framework) of the antibody using conventional techniques, by preparing and expressing a (i.e., one or more) nucleic acid molecule that encodes the antibody using conventional techniques of recombinant DNA technology or by using any other suitable method.

The anti-IL-23 specific antibody can comprise at least one of a heavy or light chain variable region having a defined amino acid sequence. For example, sometimes the anti-IL-23 antibody comprises at least one of at least one heavy chain variable region, optionally having the amino acid sequence of SEQ ID NO:106 and/or at least one light chain variable region, optionally having the amino acid sequence of SEQ ID NO: 116. Antibodies that bind to human IL-23 and that comprise a defined heavy or light chain variable region can be prepared using suitable methods, such as phage display (Katsube, Y., et al., Int J Mol. Med, 1(5):863-868 (1998)) or methods that employ transgenic animals, as known in the art and/or as described herein. For example, a transgenic mouse, comprising a functionally rearranged human immunoglobulin heavy chain transgene and a transgene comprising DNA from a human immunoglobulin light chain locus that can undergo functional rearrangement, can be immunized with human IL-23 or a fragment thereof to elicit the production of antibodies. If desired, the antibody producing cells can be isolated and hybridomas or other immortalized antibody-producing cells can be prepared as described herein and/or as known in the art. Alternatively, the antibody, specified portion or variant can be expressed using the encoding nucleic acid or portion thereof in a suitable host cell.

Also disclosed herein are antibodies, antigen-binding fragments, immunoglobulin chains and CDRs comprising amino acids in a sequence that is substantially the same as an amino acid sequence described herein. Preferably, such antibodies or antigen-binding fragments and antibodies comprising such chains or CDRs can bind human IL-23 with high affinity (e.g., K_{D} less than or equal to about 10⁻⁹ M). Amino acid sequences that are substantially the same as the sequences described herein include sequences comprising conservative amino acid substitutions, as well as amino acid deletions and/or insertions. A conservative amino acid substitution refers to the replacement of a first amino acid by a second amino acid that has chemical and/or physical properties (e.g., charge, structure, polarity, hydrophobicity/hydrophilicity) that are similar to those of the first amino acid. Conservative substitutions include, without limitation, replacement of one amino acid by another within the following groups: lysine (K), arginine (R) and histidine (H); aspartate (D) and glutamate (E); asparagine (N), glutamine (Q), serine (S), threonine (T), tyrosine (Y), K, R, H, D and E; alanine (A), valine (V), leucine (L), isoleucine (I), proline (P), phenylalanine (F), tryptophan (W), methionine (M), cysteine (C) and glycine (G); F, W and Y; C, S and T.

### Amino Acid Codes

The amino acids that make up anti-IL-23 antibodies are often abbreviated. The amino acid designations can be indicated by designating the amino acid by its single letter code, its three letter code, name, or three nucleotide codon(s) as is well understood in the art (see Alberts, B., et al., Molecular Biology of The Cell, Third Ed., Garland Publishing, Inc., New York, 1994):

| SINGLE LETTER CODE | THREE LETTER CODE | NAME | THREE NUCLEOTIDE CODON(S) |
|---|---|---|---|
| A | Ala | Alanine | GCA, GCC, GCG, GCU |
| C | Cys | Cysteine | UGC, UGU |
| D | Asp | Aspartic acid | GAC, GAU |
| E | Glu | Glutamic acid | GAA, GAG |
| F | Phe | Phenylanine | UUC, UUU |
| G | Gly | Glycine | GGA, GGC, GGG, GGU |
| H | His | Histidine | CAC, CAU |
| I | Ile | Isoleucine | AUA, AUC, AUU |
| K | Lys | Lysine | AAA, AAG |
| L | Leu | Leucine | UUA, UUG, CUA, CUC, CUG, CUU |
| M | Met | Methionine | AUG |
| N | Asn | Asparagine | AAC, AAU |
| P | Pro | Proline | CCA, CCC, CCG, CCU |
| Q | Gln | Glutamine | CAA, CAG |
| R | Arg | Arginine | AGA, AGG, CGA, CGC, CGG, CGU |
| S | Ser | Serine | AGC, AGU, UCA, UCC, UCG, UCU |
| T | Thr | Threonine | ACA, ACC, ACG, ACU |
| V | Val | Valine | GUA, GUC, GUG, GUU |
| W | Trp | Tryptophan | UGG |
| Y | Tyr | Tyrosine | UAC, UAU |

An anti-IL-23 antibody disclosed herein can include one or more amino acid substitutions, deletions or additions, either from natural mutations or human manipulation, as specified herein.

The number of amino acid substitutions a skilled artisan would make depends on many factors, including those described above. Generally speaking, the number of amino acid substitutions, insertions or deletions for any given anti-IL-23 antibody, fragment or variant will not be more than 40, 30, 20, 19, 18, 17, 16, 15, 14, 13, 12, 11, 10, 9, 8, 7, 6, 5, 4, 3, 2, 1, such as 1-30 or any range or value therein, as specified herein.

Amino acids in an anti-IL-23 specific antibody that are essential for function can be identified by methods known in the art, such as site-directed mutagenesis or alanine-scanning mutagenesis (e.g., Ausubel, supra, Chapters 8, 15; Cunningham and Wells, Science 244:1081-1085 (1989)). The latter procedure introduces single alanine mutations at every residue in the molecule. The resulting mutant molecules are then tested for biological activity, such as, but not limited to, at least one IL-23 neutralizing activity. Sites that are critical for antibody binding can also be identified by structural analysis, such as crystallization, nuclear magnetic resonance or photoaffinity labeling (Smith, et al., J. Mol. Biol. 224:899-904 (1992) and de Vos, et al., Science 255:306-312 (1992)).

Anti-IL-23 antibodies can include, but are not limited to, at least one portion, sequence or combination selected from 5 to all of the contiguous amino acids of at least one of SEQ ID NOS: 5, 20, 44, 50, 56, and 73.

IL-23 antibodies or specified portions or variants can include, but are not limited to, at least one portion, sequence or combination selected from at least 3-5 contiguous amino acids of the SEQ ID NOs above; 5-17 contiguous amino acids of the SEQ ID NOs above, 5-10 contiguous amino acids of the SEQ ID NOs above, 5-11 contiguous amino acids of the SEQ ID NOs above, 5-7 contiguous amino acids of the SEQ ID NOs above; 5-9 contiguous amino acids of the SEQ ID NOs above.

An anti-IL-23 antibody can further optionally comprise a polypeptide of at least one of 70-100% of 5, 17, 10, 11, 7, 9, 119, or 108 contiguous amino acids of the SEQ ID NOs above. Sometimes, the amino acid sequence of an immunoglobulin chain, or portion thereof (e.g., variable region, CDR) has about 70-100% identity (e.g., 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100 or any range or value therein) to the amino acid sequence of the corresponding chain of at least one of the SEQ ID NOs above. For example, the amino acid sequence of a light chain variable region can be compared with the sequence of the SEQ ID NOs above, or the amino acid sequence of a heavy chain CDR3 can be compared with the SEQ ID NOs above. Preferably, 70-100% amino acid identity (i.e., 90, 91, 92, 93, 94, 95, 96, 97, 98, 99, 100 or any range or value therein) is determined using a suitable computer algorithm, as known in the art.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as determined by the match between strings of such sequences. "Identity" and "similarity" can be readily calculated by known methods, including, but not limited to, those described in Computational Molecular Biology, Lesk, A. M., ed., Oxford University Press, New York, 1988; Biocomputing:Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., Siam J. Applied Math., 48:1073 (1988). In addition, values for percentage identity can be obtained from amino acid and nucleotide sequence alignments generated using the default settings for the AlignX component of Vector NTI Suite 8.0 (Informax, Frederick, MD).

Preferred methods to determine identity are designed to give the largest match between the sequences tested. Methods to determine identity and similarity are codified in publicly available computer programs. Preferred computer program methods to determine identity and similarity between two sequences include, but are not limited to, the GCG program package (Devereux, J., et al., Nucleic Acids Research 12(1): 387 (1984)), BLASTP, BLASTN, and FASTA (Atschul, S. F. et al., J. Molec. Biol. 215:403-410 (1990)). The BLAST X program is publicly available from NCBI and other sources (BLAST Manual, Altschul, S., et al., NCBINLM NIH Bethesda, Md. 20894: Altschul, S., et al., J. Mol. Biol. 215:403-410 (1990). The well-known Smith Waterman algorithm may also be used to determine identity.

Preferred parameters for polypeptide sequence comparison include the following: (1) Algorithm: Needleman and Wunsch, J. Mol Biol. 48:443-453 (1970) Comparison matrix: BLOSSUM62 from Hentikoff and Hentikoff, Proc. Natl. Acad. Sci, USA. 89:10915-10919 (1992)
Gap Penalty: 12
Gap Length Penalty: 4
A program useful with these parameters is publicly available as the "gap" program from Genetics Computer Group, Madison Wis. The aforementioned parameters are the default parameters for peptide sequence comparisons (along with no penalty for end gaps).

Preferred parameters for polynucleotide comparison include the following:
(1) Algorithm: Needleman and Wunsch, J. Mol Biol. 48:443-453 (1970)
Comparison matrix: matches=+10, mismatch=0
Gap Penalty: 50
Gap Length Penalty: 3
Available as: The "gap" program from Genetics Computer Group, Madison Wis. These are the default parameters for nucleic acid sequence comparisons.

By way of example, a polynucleotide sequence may be identical to another sequence, that is 100% identical, or it may include up to a certain integer number of nucleotide alterations as compared to the reference sequence. Such alterations are selected from the group consisting of at least one nucleotide deletion, substitution, including transition and transversion, or insertion, and wherein the alterations may occur at the 5' or 3' terminal positions of the reference nucleotide sequence or anywhere between those terminal positions, interspersed either individually among the nucleotides in the reference sequence or in one or more contiguous groups within the reference sequence. The number of nucleotide alterations is determined by multiplying the total number of nucleotides in the sequence by the numerical percent of the respective percent identity (divided by 100) and subtracting that product from the total number of nucleotides in the sequence, or:
n.sub.n.ltorsim.x.sub.n -(x.sub.n.y),
wherein n.sub.n is the number of nucleotide alterations, x.sub.n is the total number of nucleotides in sequence, and y is, for instance, 0.70 for 70%, 0.80 for 80%, 0.85 for 85%, 0.90 for 90%, 0.95 for 95%, etc., and wherein any non-integer product of x.sub.n and y is rounded down to the nearest integer prior to subtracting from x.sub.n.

Alterations of a polynucleotide sequence encoding the the SEQ ID NOs above may create nonsense, missense or frameshift mutations in this coding sequence and thereby alter the polypeptide encoded by the polynucleotide following such alterations. Similarly, a polypeptide sequence may be identical to the reference sequence of the SEQ ID NOs above, that is be 100% identical, or it may include up to a certain integer number of amino acid alterations as compared to the reference sequence such that the percentage identity is less than 100%. Such alterations are selected from the group consisting of at least one amino acid deletion, substitution, including conservative and non-conservative substitution, or insertion, and wherein the alterations may occur at the amino- or carboxy-terminal positions of the reference polypeptide sequence or anywhere between those terminal positions, interspersed either individually among the amino acids in the reference sequence or in one or more contiguous groups within the reference sequence. The number of amino acid alterations for a given % identity is determined by multiplying the total number of amino acids in the SEQ ID NOs above by the numerical percent of the respective percent identity (divided by 100) and then subtracting that product from the total number of amino acids in the SEQ ID NOs above, or:
n.sub.a.ltorsim.x.sub.a -(x.sub.a.y),
wherein n.sub.a is the number of amino acid alterations, x.sub.a is the total number of amino acids in the SEQ ID NOs above, and y is, for instance 0.70 for 70%, 0.80 for 80%, 0.85 for 85% etc., and wherein any non-integer produce of x.sub.a and y is rounded down to the nearest integer prior to subtracting it from x.sub.a.

Exemplary heavy chain and light chain variable regions sequences and portions thereof are provided in the SEQ ID NOs above. The antibodies disclosed herein, or specified variants thereof, can comprise any number of contiguous amino acid residues from an antibody dislcosed herein, wherein that number is selected from the group of integers consisting of from 10-100% of the number of contiguous residues in an anti-IL-23 antibody. Optionally, this subsequence of contiguous amino acids is at least about 10, 20, 30, 40, 50, 60, 70, 80, 90, 100, 110, 120, 130, 140, 150, 160, 170, 180, 190, 200, 210, 220, 230, 240, 250 or more amino acids in length, or any range or value therein. Further, the number of such subsequences can be any integer selected from the group consisting of from 1 to 20, such as at least 2, 3, 4, or 5.

As those of skill will appreciate, biologically active antibodies have a specific activity at least 20%, 30%, or 40%, and, preferably, at least 50%, 60%, or 70%, and, most preferably, at least 80%, 90%, or 95%-100% or more (including, without limitation, up to 10 times the specific activity) of that of the native (non-synthetic), endogenous or related and known antibody. Methods of assaying and quantifying measures of enzymatic activity and substrate specificity are well known to those of skill in the art.

Also disclosed herein are human antibodies and antigen-binding fragments, as described herein, which are modified by the covalent attachment of an organic moiety. Such modification can produce an antibody or antigen-binding fragment with improved pharmacokinetic properties (e.g., increased *in vivo* serum half-life). The organic moiety can be a linear or branched hydrophilic polymeric group, fatty acid group, or fatty acid ester group. Sometimes, the hydrophilic polymeric group can have a molecular weight of about 800 to about 120,000 Daltons and can be a polyalkane glycol (e.g., polyethylene glycol (PEG), polypropylene glycol (PPG)), carbohydrate polymer, amino acid polymer or polyvinyl pyrolidone, and the fatty acid or fatty acid ester group can comprise from about eight to about forty carbon atoms.

The modified antibodies and antigen-binding fragments can comprise one or more organic moieties that are covalently bonded, directly or indirectly, to the antibody. Each organic moiety that is bonded to an antibody or antigen-binding fragment dislcosed herein can independently be a hydrophilic polymeric group, a fatty acid group or a fatty acid ester group. As used herein, the term "fatty acid" encompasses mono-carboxylic acids and di-carboxylic acids. A "hydrophilic polymeric group," as the term is used herein, refers to an organic polymer that is more soluble in water than in octane. For example, polylysine is more soluble in water than in octane. Thus, an antibody modified by the covalent attachment of polylysine isdisclosed. Hydrophilic polymers suitable for modifying antibodies can be linear or branched and include, for example, polyalkane glycols (e.g., PEG, monomethoxy-polyethylene glycol (mPEG), PPG and the like), carbohydrates (e.g., dextran, cellulose, oligosaccharides, polysaccharides and the like), polymers of hydrophilic amino acids (e.g., polylysine, polyarginine, polyaspartate and the like), polyalkane oxides (e.g., polyethylene oxide, polypropylene oxide and the like) and polyvinyl pyrolidone. Preferably, the hydrophilic polymer that modifies an antibody disclosed herein has a molecular weight of about 800 to about 150,000 Daltons as a separate molecular entity. For example, PEG₅₀₀₀ and PEG_{20,000,} wherein the subscript is the average molecular weight of the polymer in Daltons, can be used. The hydrophilic polymeric group can be substituted with one to about six alkyl, fatty acid or fatty acid ester groups. Hydrophilic polymers that are substituted with a fatty acid or fatty acid ester group can be prepared by employing suitable methods. For example, a polymer comprising an amine group can be coupled to a carboxylate of the fatty acid or fatty acid ester, and an activated carboxylate (e.g., activated with N, N-carbonyl diimidazole) on a fatty acid or fatty acid ester can be coupled to a hydroxyl group on a polymer.

Fatty acids and fatty acid esters suitable for modifying antibodies can be saturated or can contain one or more units of unsaturation. Fatty acids that are suitable for modifying antibodies include, for example, n-dodecanoate (C₁₂, laurate), n-tetradecanoate (C₁₄, myristate), n-octadecanoate (C₁₈, stearate), n-eicosanoate (C₂₀, arachidate), n-docosanoate (C₂₂, behenate), n-triacontanoate (C₃₀), n-tetracontanoate (C₄₀), *cis*-Δ9-octadecanoate (C₁₈, oleate), all *cis-*Δ5,8,11,14-eicosatetraenoate (C₂₀, arachidonate), octanedioic acid, tetradecanedioic acid, octadecanedioic acid, docosanedioic acid, and the like. Suitable fatty acid esters include monoesters of dicarboxylic acids that comprise a linear or branched lower alkyl group. The lower alkyl group can comprise from one to about twelve, preferably, one to about six, carbon atoms.

The modified human antibodies and antigen-binding fragments can be prepared using suitable methods, such as by reaction with one or more modifying agents. A "modifying agent" as the term is used herein, refers to a suitable organic group (e.g., hydrophilic polymer, a fatty acid, a fatty acid ester) that comprises an activating group. An "activating group" is a chemical moiety or functional group that can, under appropriate conditions, react with a second chemical group thereby forming a covalent bond between the modifying agent and the second chemical group. For example, amine-reactive activating groups include electrophilic groups, such as tosylate, mesylate, halo (chloro, bromo, fluoro, iodo), N-hydroxysuccinimidyl esters (NHS), and the like. Activating groups that can react with thiols include, for example, maleimide, iodoacetyl, acrylolyl, pyridyl disulfides, 5-thiol-2-nitrobenzoic acid thiol (TNB-thiol), and the like. An aldehyde functional group can be coupled to amine- or hydrazide-containing molecules, and an azide group can react with a trivalent phosphorous group to form phosphoramidate or phosphorimide linkages. Suitable methods to introduce activating groups into molecules are known in the art (see for example, Hermanson, G. T., Bioconjugate Techniques, Academic Press: San Diego, CA (1996)). An activating group can be bonded directly to the organic group (e.g., hydrophilic polymer, fatty acid, fatty acid ester), or through a linker moiety, for example, a divalent C₁-C₁₂ group wherein one or more carbon atoms can be replaced by a heteroatom, such as oxygen, nitrogen or sulfur. Suitable linker moieties include, for example, tetraethylene glycol, -(CH₂)₃-, -NH-(CH₂)₆-NH-, -(CH₂)₂-NH- and -CH₂-O-CH₂-CH₂-O-CH₂-CH₂-O-CH-NH-. Modifying agents that comprise a linker moiety can be produced, for example, by reacting a mono-Boc-alkyldiamine (e.g., mono-Boc-ethylenediamine, mono-Boc-diaminohexane) with a fatty acid in the presence of 1-ethyl-3-(3-dimethylaminopropyl) carbodiimide (EDC) to form an amide bond between the free amine and the fatty acid carboxylate. The Boc protecting group can be removed from the product by treatment with trifluoroacetic acid (TFA) to expose a primary amine that can be coupled to another carboxylate, as described, or can be reacted with maleic anhydride and the resulting product cyclized to produce an activated maleimido derivative of the fatty acid. (See, for example, Thompson, et al., WO 92/16221)

The modified antibodies can be produced by reacting a human antibody or antigen-binding fragment with a modifying agent. For example, the organic moieties can be bonded to the antibody in a non-site specific manner by employing an amine-reactive modifying agent, for example, an NHS ester of PEG. Modified human antibodies or antigen-binding fragments can also be prepared by reducing disulfide bonds (e.g., intra-chain disulfide bonds) of an antibody or antigen-binding fragment. The reduced antibody or antigen-binding fragment can then be reacted with a thiol-reactive modifying agent to produce the modified antibody. Modified human antibodies and antigen-binding fragments comprising an organic moiety that is bonded to specific sites of an antibody can be prepared using suitable methods, such as reverse proteolysis (Fisch et al., Bioconjugate Chem., 3:147-153 (1992); Werlen et al., Bioconjugate Chem., 5:411-417 (1994); Kumaran et al., Protein Sci. 6(10):2233-2241 (1997); Itoh et al., Bioorg. Chem., 24(1): 59-68 (1996); Capellas et al., Biotechnol. Bioeng., 56(4):456-463 (1997)), and the methods described in Hermanson, G. T., Bioconjugate Techniques, Academic Press: San Diego, CA (1996).

The method of the present invention also uses an anti-IL-23 antibody composition comprising guselkumab and at least one, at least two, at least three, at least four, at least five, or more anti-IL-23 antibodies thereof, as described herein and/or as known in the art that are provided in a non-naturally occurring composition, mixture or form. Such compositions comprise non-naturally occurring compositions comprising guselkumab and at least one full length, C- and/or N-terminally deleted variant, domain, fragment, or specified variant, of the anti-IL-23 antibody amino acid sequence selected from the group consisting of 70-100% of the contiguous amino acids of the SEQ ID NOs above, or specified fragments, domains or variants thereof. Preferred anti-IL-23 antibody compositions include guselkumab and at least one full length, fragment, domain or variant as at least one CDR or LBP containing portion of the anti-IL-23 antibody sequence described herein, for example, 70-100% of the SEQ ID NOs above, or specified fragments, domains or variants thereof. Further preferred compositions comprise, for example, guselkumab and 40-99% of at least one of 70-100% of the SEQ ID NOs above, etc., or specified fragments, domains or variants thereof. Such composition percentages are by weight, volume, concentration, molarity, or molality as liquid or dry solutions, mixtures, suspension, emulsions, particles, powder, or colloids, as known in the art or as described herein.

### Antibody Compositions Comprising Further Therapeutically Active Ingredients

The antibody compositions used in the method of the invention can optionally further comprise an effective amount of at least one compound or protein selected from at least one of an anti-infective drug, a cardiovascular (CV) system drug, a central nervous system (CNS) drug, an autonomic nervous system (ANS) drug, a respiratory tract drug, a gastrointestinal (GI) tract drug, a hormonal drug, a drug for fluid or electrolyte balance, a hematologic drug, an antineoplastic, an immunomodulation drug, an ophthalmic, otic or nasal drug, a topical drug, a nutritional drug or the like. Such drugs are well known in the art, including formulations, indications, dosing and administration for each presented herein (see, e.g., Nursing 2001 Handbook of Drugs, 21st edition, Springhouse Corp., Springhouse, PA, 2001; Health Professional's Drug Guide 2001, ed., Shannon, Wilson, Stang, Prentice-Hall, Inc, Upper Saddle River, NJ; Pharmcotherapy Handbook, Wells et al., ed., Appleton & Lange, Stamford, CT).

By way of example of the drugs that can be combined with the antibodies for the method of the present invention, the anti-infective drug can be at least one selected from amebicides or at least one antiprotozoals, anthelmintics, antifungals, antimalarials, antituberculotics or at least one antileprotics, aminoglycosides, penicillins, cephalosporins, tetracyclines, sulfonamides, fluoroquinolones, antivirals, macrolide anti-infectives, and miscellaneous anti-infectives. The hormonal drug can be at least one selected from corticosteroids, androgens or at least one anabolic steroid, estrogen or at least one progestin, gonadotropin, antidiabetic drug or at least one glucagon, thyroid hormone, thyroid hormone antagonist, pituitary hormone, and parathyroid-like drug. The at least one cephalosporin can be at least one selected from cefaclor, cefadroxil, cefazolin sodium, cefdinir, cefepime hydrochloride, cefixime, cefmetazole sodium, cefonicid sodium, cefoperazone sodium, cefotaxime sodium, cefotetan disodium, cefoxitin sodium, cefpodoxime proxetil, cefprozil, ceftazidime, ceftibuten, ceftizoxime sodium, ceftriaxone sodium, cefuroxime axetil, cefuroxime sodium, cephalexin hydrochloride, cephalexin monohydrate, cephradine, and loracarbef.

The at least one coricosteroid can be at least one selected from betamethasone, betamethasone acetate or betamethasone sodium phosphate, betamethasone sodium phosphate, cortisone acetate, dexamethasone, dexamethasone acetate, dexamethasone sodium phosphate, fludrocortisone acetate, hydrocortisone, hydrocortisone acetate, hydrocortisone cypionate, hydrocortisone sodium phosphate, hydrocortisone sodium succinate, methylprednisolone, methylprednisolone acetate, methylprednisolone sodium succinate, prednisolone, prednisolone acetate, prednisolone sodium phosphate, prednisolone tebutate, prednisone, triamcinolone, triamcinolone acetonide, and triamcinolone diacetate. The at least one androgen or anabolic steroid can be at least one selected from danazol, fluoxymesterone, methyltestosterone, nandrolone decanoate, nandrolone phenpropionate, testosterone, testosterone cypionate, testosterone enanthate, testosterone propionate, and testosterone transdermal system.

The at least one immunosuppressant can be at least one selected from azathioprine, basiliximab, cyclosporine, daclizumab, lymphocyte immune globulin, muromonab-CD3, mycophenolate mofetil, mycophenolate mofetil hydrochloride, sirolimus, and tacrolimus.

The at least one local anti-infective can be at least one selected from acyclovir, amphotericin B, azelaic acid cream, bacitracin, butoconazole nitrate, clindamycin phosphate, clotrimazole, econazole nitrate, erythromycin, gentamicin sulfate, ketoconazole, mafenide acetate, metronidazole (topical), miconazole nitrate, mupirocin, naftifine hydrochloride, neomycin sulfate, nitrofurazone, nystatin, silver sulfadiazine, terbinafine hydrochloride, terconazole, tetracycline hydrochloride, tioconazole, and tolnaftate. The at least one scabicide or pediculicide can be at least one selected from crotamiton, lindane, permethrin, and pyrethrins. The at least one topical corticosteroid can be at least one selected from betamethasone dipropionate, betamethasone valerate, clobetasol propionate, desonide, desoximetasone, dexamethasone, dexamethasone sodium phosphate, diflorasone diacetate, fluocinolone acetonide, fluocinonide, flurandrenolide, fluticasone propionate, halcionide, hydrocortisone, hydrocortisone acetate, hydrocortisone butyrate, hydrocorisone valerate, mometasone furoate, and triamcinolone acetonide. (See, e.g., pp. 1098-1136 of Nursing 2001 Drug Handbook*.*)

Anti-IL-23 antibody compositions can further comprise at least one of any suitable and effective amount of a composition or pharmaceutical composition comprising at least one anti-IL-23 antibody contacted or administered to a cell, tissue, organ, animal or patient in need of such modulation, treatment or therapy, optionally further comprising at least one selected from at least one TNF antagonist (e.g., but not limited to a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, infliximab, eternacept, CDP-571, CDP-870, afelimomab, lenercept, and the like), an antirheumatic (e.g., methotrexate, auranofin, aurothioglucose, azathioprine, etanercept, gold sodium thiomalate, hydroxychloroquine sulfate, leflunomide, sulfasalzine), an immunization, an immunoglobulin, an immunosuppressive (e.g., basiliximab, cyclosporine, daclizumab), a cytokine or a cytokine antagonist. Non-limiting examples of such cytokines include, but are not limited to, any of IL-1 to IL-23 et al. (e.g., IL-1, IL-2, etc.). Suitable dosages are well known in the art. See, e.g., Wells et al., eds., Pharmacotherapy Handbook, 2nd Edition, Appleton and Lange, Stamford, CT (2000); PDR Pharmacopoeia, Tarascon Pocket Pharmacopoeia 2000, Deluxe Edition, Tarascon Publishing, Loma Linda, CA (2000).

Anti-IL-23 antibody compounds, compositions or combinations used in the method of the present invention can further comprise at least one of any suitable auxiliary, such as, but not limited to, diluent, binder, stabilizer, buffers, salts, lipophilic solvents, preservative, adjuvant or the like. Pharmaceutically acceptable auxiliaries are preferred. Non-limiting examples of, and methods of preparing such sterile solutions are well known in the art, such as, but limited to, Gennaro, Ed., Remington's Pharmaceutical Sciences, 18th Edition, Mack Publishing Co. (Easton, PA) 1990. Pharmaceutically acceptable carriers can be routinely selected that are suitable for the mode of administration, solubility and/or stability of the anti-IL-23 antibody, fragment or variant composition as well known in the art or as described herein.

Pharmaceutical excipients and additives useful in the present composition include, but are not limited to, proteins, peptides, amino acids, lipids, and carbohydrates (e.g., sugars, including monosaccharides, di-, tri-, tetra-, and oligosaccharides; derivatized sugars, such as alditols, aldonic acids, esterified sugars and the like; and polysaccharides or sugar polymers), which can be present singly or in combination, comprising alone or in combination 1-99.99% by weight or volume. Exemplary protein excipients include serum albumin, such as human serum albumin (HSA), recombinant human albumin (rHA), gelatin, casein, and the like. Representative amino acid/antibody components, which can also function in a buffering capacity, include alanine, glycine, arginine, betaine, histidine, glutamic acid, aspartic acid, cysteine, lysine, leucine, isoleucine, valine, methionine, phenylalanine, aspartame, and the like. One preferred amino acid is glycine.

Carbohydrate excipients suitable for use in the invention include, for example, monosaccharides, such as fructose, maltose, galactose, glucose, D-mannose, sorbose, and the like; disaccharides, such as lactose, sucrose, trehalose, cellobiose, and the like; polysaccharides, such as raffinose, melezitose, maltodextrins, dextrans, starches, and the like; and alditols, such as mannitol, xylitol, maltitol, lactitol, xylitol sorbitol (glucitol), myoinositol and the like. Preferred carbohydrate excipients for use in the present invention are mannitol, trehalose, and raffinose.

Anti-IL-23 antibody compositions can also include a buffer or a pH adjusting agent; typically, the buffer is a salt prepared from an organic acid or base. Representative buffers include organic acid salts, such as salts of citric acid, ascorbic acid, gluconic acid, carbonic acid, tartaric acid, succinic acid, acetic acid, or phthalic acid; Tris, tromethamine hydrochloride, or phosphate buffers. Preferred buffers for use in the present compositions are organic acid salts, such as citrate.

Additionally, anti-IL-23 antibody compositions can include polymeric excipients/additives, such as polyvinylpyrrolidones, ficolls (a polymeric sugar), dextrates (e.g., cyclodextrins, such as 2-hydroxypropyl-β-cyclodextrin), polyethylene glycols, flavoring agents, antimicrobial agents, sweeteners, antioxidants, antistatic agents, surfactants (e.g., polysorbates, such as "TWEEN 20" and "TWEEN 80"), lipids (*e.g.*, phospholipids, fatty acids), steroids (e.g., cholesterol), and chelating agents (e.g., EDTA).

These and additional known pharmaceutical excipients and/or additives suitable for use in the anti-IL-23 antibody, portion or variant compositions according to the invention are known in the art, e.g., as listed in "Remington: The Science & Practice of Pharmacy," 19th ed., Williams & Williams, (1995), and in the "Physician's Desk Reference," 52nd ed., Medical Economics, Montvale, NJ (1998). Preferred carrier or excipient materials are carbohydrates (e.g., saccharides and alditols) and buffers (e.g., citrate) or polymeric agents. An exemplary carrier molecule is the mucopolysaccharide, hyaluronic acid, which may be useful for intraarticular delivery.

### Formulations

As noted above, the invention provides for stable formulations, which preferably comprise a phosphate buffer with saline or a chosen salt, as well as preserved solutions and formulations containing a preservative as well as multi-use preserved formulations suitable for pharmaceutical or veterinary use, comprising at least one anti-IL-23 antibody in a pharmaceutically acceptable formulation. Preserved formulations contain at least one known preservative or optionally selected from the group consisting of at least one phenol, m-cresol, p-cresol, o-cresol, chlorocresol, benzyl alcohol, phenylmercuric nitrite, phenoxyethanol, formaldehyde, chlorobutanol, magnesium chloride (e.g., hexahydrate), alkylparaben (methyl, ethyl, propyl, butyl and the like), benzalkonium chloride, benzethonium chloride, sodium dehydroacetate and thimerosal, or mixtures thereof in an aqueous diluent. Any suitable concentration or mixture can be used as known in the art, such as 0.001-5%, or any range or value therein, such as, but not limited to 0.001, 0.003, 0.005, 0.009, 0.01, 0.02, 0.03, 0.05, 0.09, 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.3, 4.5, 4.6, 4.7, 4.8, 4.9, or any range or value therein. Non-limiting examples include, no preservative, 0.1-2% m-cresol (e.g., 0.2, 0.3. 0.4, 0.5, 0.9, 1.0%), 0.1-3% benzyl alcohol (e.g., 0.5, 0.9, 1.1, 1.5, 1.9, 2.0, 2.5%), 0.001-0.5% thimerosal (e.g., 0.005, 0.01), 0.001-2.0% phenol (e.g., 0.05, 0.25, 0.28, 0.5, 0.9, 1.0%), 0.0005-1.0% alkylparaben(s) (e.g., 0.00075, 0.0009, 0.001, 0.002, 0.005, 0.0075, 0.009, 0.01, 0.02, 0.05, 0.075, 0.09, 0.1, 0.2, 0.3, 0.5, 0.75, 0.9, 1.0%), and the like.

As noted above, the method of the invention uses an article of manufacture, comprising packaging material and at least one vial comprising a solution of at least one anti-IL-23 specific antibody with the prescribed buffers and/or preservatives, optionally in an aqueous diluent, wherein said packaging material comprises a label that indicates that such solution can be held over a period of 1, 2, 3, 4, 5, 6, 9, 12, 18, 20, 24, 30, 36, 40, 48, 54, 60, 66, 72 hours or greater. The invention further uses an article of manufacture, comprising packaging material, a first vial comprising lyophilized anti-IL-23 specific antibody, and a second vial comprising an aqueous diluent of prescribed buffer or preservative, wherein said packaging material comprises a label that instructs a patient to reconstitute the anti-IL-23 specific antibody in the aqueous diluent to form a solution that can be held over a period of twenty-four hours or greater.

The anti-IL-23 specific antibody used in accordance with the present invention can be produced by recombinant means, including from mammalian cell or transgenic preparations, or can be purified from other biological sources, as described herein or as known in the art.

The range of the anti-IL-23 specific antibody includes amounts yielding upon reconstitution, if in a wet/dry system, concentrations from about 1.0 µg/ml to about 1000 mg/ml, although lower and higher concentrations are operable and are dependent on the intended delivery vehicle, e.g., solution formulations will differ from transdermal patch, pulmonary, transmucosal, or osmotic or micro pump methods.

Preferably, the aqueous diluent optionally further comprises a pharmaceutically acceptable preservative. Preferred preservatives include those selected from the group consisting of phenol, m-cresol, p-cresol, o-cresol, chlorocresol, benzyl alcohol, alkylparaben (methyl, ethyl, propyl, butyl and the like), benzalkonium chloride, benzethonium chloride, sodium dehydroacetate and thimerosal, or mixtures thereof. The concentration of preservative used in the formulation is a concentration sufficient to yield an anti-microbial effect. Such concentrations are dependent on the preservative selected and are readily determined by the skilled artisan.

Other excipients, e.g., isotonicity agents, buffers, antioxidants, and preservative enhancers, can be optionally and preferably added to the diluent. An isotonicity agent, such as glycerin, is commonly used at known concentrations. A physiologically tolerated buffer is preferably added to provide improved pH control. The formulations can cover a wide range of pHs, such as from about pH 4 to about pH 10, and preferred ranges from about pH 5 to about pH 9, and a most preferred range of about 6.0 to about 8.0. Preferably, the formulations of the present invention have a pH between about 6.8 and about 7.8. Preferred buffers include phosphate buffers, most preferably, sodium phosphate, particularly, phosphate buffered saline (PBS).

Other additives, such as a pharmaceutically acceptable solubilizers like Tween 20 (polyoxyethylene (20) sorbitan monolaurate), Tween 40 (polyoxyethylene (20) sorbitan monopalmitate), Tween 80 (polyoxyethylene (20) sorbitan monooleate), Pluronic F68 (polyoxyethylene polyoxypropylene block copolymers), and PEG (polyethylene glycol) or nonionic surfactants, such as polysorbate 20 or 80 or poloxamer 184 or 188, Pluronic^{®} polyls, other block co-polymers, and chelators, such as EDTA and EGTA, can optionally be added to the formulations or compositions to reduce aggregation. These additives are particularly useful if a pump or plastic container is used to administer the formulation. The presence of pharmaceutically acceptable surfactant mitigates the propensity for the protein to aggregate.

The formulations can be prepared by a process which comprises mixing at least one anti-IL-23 specific antibody and a preservative selected from the group consisting of phenol, m-cresol, p-cresol, o-cresol, chlorocresol, benzyl alcohol, alkylparaben, (methyl, ethyl, propyl, butyl and the like), benzalkonium chloride, benzethonium chloride, sodium dehydroacetate and thimerosal or mixtures thereof in an aqueous diluent. Mixing the at least one anti-IL-23 specific antibody and preservative in an aqueous diluent is carried out using conventional dissolution and mixing procedures. To prepare a suitable formulation, for example, a measured amount of at least one anti-IL-23 specific antibody in buffered solution is combined with the desired preservative in a buffered solution in quantities sufficient to provide the protein and preservative at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

The formulations can be provided to patients as clear solutions or as dual vials comprising a vial of lyophilized anti-IL-23 specific antibody that is reconstituted with a second vial containing water, a preservative and/or excipients, preferably, a phosphate buffer and/or saline and a chosen salt, in an aqueous diluent. Either a single solution vial or dual vial requiring reconstitution can be reused multiple times and can suffice for a single or multiple cycles of patient treatment and thus can provide a more convenient treatment regimen than currently available.

The present articles of manufacture are useful for administration over a period ranging from immediate to twenty-four hours or greater. Accordingly, the presently claimed articles of manufacture offer significant advantages to the patient. Formulations of the invention can optionally be safely stored at temperatures of from about 2°C to about 40°C and retain the biologically activity of the protein for extended periods of time, thus allowing a package label indicating that the solution can be held and/or used over a period of 6, 12, 18, 24, 36, 48, 72, or 96 hours or greater. If preserved diluent is used, such label can include use up to 1-12 months, one-half, one and a half, and/or two years.

The solutions of anti-IL-23 specific antibody can be prepared by a process that comprises mixing at least one antibody in an aqueous diluent. Mixing is carried out using conventional dissolution and mixing procedures. To prepare a suitable diluent, for example, a measured amount of at least one antibody in water or buffer is combined in quantities sufficient to provide the protein and, optionally, a preservative or buffer at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

The claimed products can be provided to patients as clear solutions or as dual vials comprising a vial of lyophilized at least one anti-IL-23 specific antibody that is reconstituted with a second vial containing the aqueous diluent. Either a single solution vial or dual vial requiring reconstitution can be reused multiple times and can suffice for a single or multiple cycles of patient treatment and thus provides a more convenient treatment regimen than currently available.

The claimed products can be provided indirectly to patients by providing to pharmacies, clinics, or other such institutions and facilities, clear solutions or dual vials comprising a vial of lyophilized at least one anti-IL-23 specific antibody that is reconstituted with a second vial containing the aqueous diluent. The clear solution in this case can be up to one liter or even larger in size, providing a large reservoir from which smaller portions of the at least one antibody solution can be retrieved one or multiple times for transfer into smaller vials and provided by the pharmacy or clinic to their customers and/or patients.

Recognized devices comprising single vial systems include pen-injector devices for delivery of a solution, such as BD Pens, BD Autojector^{®}, Humaject^{®,} NovoPen^{®}, B-D^{®}Pen, AutoPen^{®}, and OptiPen^{®}, GenotropinPen^{®}, Genotronorm Pen^{®}, Humatro Pen^{®}, Reco-Pen^{®}, Roferon Pen^{®}, Biojector^{®}, Iject^{®}, J-tip Needle-Free Injector^{®}, Intraject^{®}, Medi-Ject^{®}, Smartject^{®} e.g., as made or developed by Becton Dickensen (Franklin Lakes, NJ, www.bectondickenson.com), Disetronic (Burgdorf, Switzerland, www.disetronic.com; Bioject, Portland, Oregon (www.bioject.com); National Medical Products, Weston Medical (Peterborough, UK, www.weston-medical.com), Medi-Ject Corp (Minneapolis, MN, www.mediject.com), and similary suitable devices. Recognized devices comprising a dual vial system include those pen-injector systems for reconstituting a lyophilized drug in a cartridge for delivery of the reconstituted solution, such as the HumatroPen^{®}. Examples of other devices suitable include pre-filled syringes, auto-injectors, needle free injectors, and needle free IV infusion sets.

The products may include packaging material. The packaging material provides, in addition to the information required by the regulatory agencies, the conditions under which the product can be used. The packaging material provides instructions to the patient, as applicable, to reconstitute the at least one anti-IL-23 antibody in the aqueous diluent to form a solution and to use the solution over a period of 2-24 hours or greater for the two vial, wet/dry, product. For the single vial, solution product, pre-filled syringe or auto-injector, the label indicates that such solution can be used over a period of 2-24 hours or greater. The products are useful for human pharmaceutical product use.

The formulations used in the method of the present invention can be prepared by a process that comprises mixing an anti-IL-23 antibody and a selected buffer, preferably, a phosphate buffer containing saline or a chosen salt. Mixing the anti-IL-23 antibody and buffer in an aqueous diluent is carried out using conventional dissolution and mixing procedures. To prepare a suitable formulation, for example, a measured amount of at least one antibody in water or buffer is combined with the desired buffering agent in water in quantities sufficient to provide the protein and buffer at the desired concentrations. Variations of this process would be recognized by one of ordinary skill in the art. For example, the order the components are added, whether additional additives are used, the temperature and pH at which the formulation is prepared, are all factors that can be optimized for the concentration and means of administration used.

The method of the invention provides pharmaceutical compositions comprising various formulations useful and acceptable for administration to a human or animal patient. Such pharmaceutical compositions are prepared using water at "standard state" as the diluent and routine methods well known to those of ordinary skill in the art. For example, buffering components such as histidine and histidine monohydrochloride hydrate, may be provided first followed by the addition of an appropriate, non-final volume of water diluent, sucrose and polysorbate 80 at "standard state." Isolated antibody may then be added. Last, the volume of the pharmaceutical composition is adjusted to the desired final volume under "standard state" conditions using water as the diluent. Those skilled in the art will recognize a number of other methods suitable for the preparation of the pharmaceutical compositions.

The pharmaceutical compositions may be aqueous solutions or suspensions comprising the indicated mass of each constituent per unit of water volume or having an indicated pH at "standard state." As used herein, the term "standard state" means a temperature of 25°C +/- 2°C and a pressure of 1 atmosphere. The term "standard state" is not used in the art to refer to a single art recognized set of temperatures or pressure, but is instead a reference state that specifies temperatures and pressure to be used to describe a solution or suspension with a particular composition under the reference "standard state" conditions. This is because the volume of a solution is, in part, a function of temperature and pressure. Those skilled in the art will recognize that pharmaceutical compositions equivalent to those disclosed here can be produced at other temperatures and pressures. Whether such pharmaceutical compositions are equivalent to those disclosed here should be determined under the "standard state" conditions defined above (*e.g*. 25°C +/- 2°C and a pressure of 1 atmosphere).

Importantly, such pharmaceutical compositions may contain component masses "about" a certain value (*e.g*. "about 0.53 mg L-histidine") per unit volume of the pharmaceutical composition or have pH values about a certain value. A component mass present in a pharmaceutical composition or pH value is "about" a given numerical value if the isolated antibody present in the pharmaceutical composition is able to bind a peptide chain while the isolated antibody is present in the pharmaceutical composition or after the isolated antibody has been removed from the pharmaceutical composition (*e.g*., by dilution). Stated differently, a value, such as a component mass value or pH value, is "about" a given numerical value when the binding activity of the isolated antibody is maintained and detectable after placing the isolated antibody in the pharmaceutical composition.

Competition binding analysis is performed to determine if the IL-23 specific mAbs bind to similar or different epitopes and/or compete with each other. Abs are individually coated on ELISA plates. Competing mAbs are added, followed by the addition of biotinylated hrIL-23. For positive control, the same mAb for coating may be used as the competing mAb ("self-competition"). IL-23 binding is detected using streptavidin. These results demonstrate whether the mAbs recognize similar or partially overlapping epitopes on IL-23.

In one embodiment of the pharmaceutical compositions, the isolated antibody concentration is from about 77 to about 104 mg per ml of the pharmaceutical composition. In another embodiment of the pharmaceutical compositions the pH is from about 5.5 to about 6.5.

The stable or preserved formulations can be provided to patients as clear solutions or as dual vials comprising a vial of lyophilized at least one anti-IL-23 antibody that is reconstituted with a second vial containing a preservative or buffer and excipients in an aqueous diluent. Either a single solution vial or dual vial requiring reconstitution can be reused multiple times and can suffice for a single or multiple cycles of patient treatment and thus provides a more convenient treatment regimen than currently available.

Other formulations or methods of stabilizing the anti-IL-23 antibody may result in other than a clear solution of lyophilized powder comprising the antibody. Among non-clear solutions are formulations comprising particulate suspensions, said particulates being a composition containing the anti-IL-23 antibody in a structure of variable dimension and known variously as a microsphere, microparticle, nanoparticle, nanosphere, or liposome. Such relatively homogenous, essentially spherical, particulate formulations containing an active agent can be formed by contacting an aqueous phase containing the active agent and a polymer and a nonaqueous phase followed by evaporation of the nonaqueous phase to cause the coalescence of particles from the aqueous phase as taught in U.S. 4,589,330. Porous microparticles can be prepared using a first phase containing active agent and a polymer dispersed in a continuous solvent and removing said solvent from the suspension by freeze-drying or dilution-extraction-precipitation as taught in U.S. 4,818,542. Preferred polymers for such preparations are natural or synthetic copolymers or polymers selected from the group consisting of gleatin agar, starch, arabinogalactan, albumin, collagen, polyglycolic acid, polylactic aced, glycolide-L(-) lactide poly(episilon-caprolactone, poly(epsilon-caprolactone-CO-lactic acid), poly(epsilon-caprolactone-CO-glycolic acid), poly(β-hydroxy butyric acid), polyethylene oxide, polyethylene, poly(alkyl-2-cyanoacrylate), poly(hydroxyethyl methacrylate), polyamides, poly(amino acids), poly(2-hydroxyethyl DL-aspartamide), poly(ester urea), poly(L-phenylalanine/ethylene glycol/1,6-diisocyanatohexane) and poly(methyl methacrylate). Particularly preferred polymers are polyesters, such as polyglycolic acid, polylactic aced, glycolide-L(-) lactide poly(episilon-caprolactone, poly(epsilon-caprolactone-CO-lactic acid), and poly(epsilon-caprolactone-CO-glycolic acid. Solvents useful for dissolving the polymer and/or the active include: water, hexafluoroisopropanol, methylenechloride, tetrahydrofuran, hexane, benzene, or hexafluoroacetone sesquihydrate. The process of dispersing the active containing phase with a second phase may include pressure forcing said first phase through an orifice in a nozzle to affect droplet formation.

Dry powder formulations may result from processes other than lyophilization, such as by spray drying or solvent extraction by evaporation or by precipitation of a crystalline composition followed by one or more steps to remove aqueous or nonaqueous solvent. Preparation of a spray-dried antibody preparation is taught in U.S. 6,019,968. The antibody-based dry powder compositions may be produced by spray drying solutions or slurries of the antibody and, optionally, excipients, in a solvent under conditions to provide a respirable dry powder. Solvents may include polar compounds, such as water and ethanol, which may be readily dried. Antibody stability may be enhanced by performing the spray drying procedures in the absence of oxygen, such as under a nitrogen blanket or by using nitrogen as the drying gas. Another relatively dry formulation is a dispersion of a plurality of perforated microstructures dispersed in a suspension medium that typically comprises a hydrofluoroalkane propellant as taught in WO 9916419. The stabilized dispersions may be administered to the lung of a patient using a metered dose inhaler. Equipment useful in the commercial manufacture of spray dried medicaments are manufactured by Buchi Ltd. or Niro Corp.

An anti-IL-23 antibody in either the stable or preserved formulations or solutions described herein, can be administered to a patient in accordance with the present invention via a variety of delivery methods including SC or IM injection; transdermal, pulmonary, transmucosal, implant, osmotic pump, cartridge, micro pump, or other means appreciated by the skilled artisan, as well-known in the art.

### Therapeutic Applications

Any method disclosed herein can comprise administering an effective amount of a composition or pharmaceutical composition comprising an anti-IL-23 antibody to a cell, tissue, organ, animal or patient in need of such modulation, treatment or therapy. Such a method can optionally further comprise co-administration or combination therapy for treating such diseases or disorders, wherein the administering of said at least one anti-IL-23 antibody, specified portion or variant thereof, further comprises administering, before concurrently, and/or after, at least one selected from at least one TNF antagonist (e.g., but not limited to, a TNF chemical or protein antagonist, TNF monoclonal or polyclonal antibody or fragment, a soluble TNF receptor (e.g., p55, p70 or p85) or fragment, fusion polypeptides thereof, or a small molecule TNF antagonist, e.g., TNF binding protein I or II (TBP-1 or TBP-II), nerelimonmab, infliximab, eternacept (Enbrel^{™}), adalimulab (Humira^{™}), CDP-571, CDP-870, afelimomab, lenercept, and the like), an antirheumatic (e.g., methotrexate, auranofin, aurothioglucose, azathioprine, gold sodium thiomalate, hydroxychloroquine sulfate, leflunomide, sulfasalzine), a muscle relaxant, a narcotic, a non-steroid anti-inflammatory drug (NSAID), an analgesic, an anesthetic, a sedative, a local anesthetic, a neuromuscular blocker, an antimicrobial (e.g., aminoglycoside, an antifungal, an antiparasitic, an antiviral, a carbapenem, cephalosporin, a flurorquinolone, a macrolide, a penicillin, a sulfonamide, a tetracycline, another antimicrobial), an antipsoriatic, a corticosteriod, an anabolic steroid, a diabetes related agent, a mineral, a nutritional, a thyroid agent, a vitamin, a calcium related hormone, an antidiarrheal, an antitussive, an antiemetic, an antiulcer, a laxative, an anticoagulant, an erythropoietin (e.g., epoetin alpha), a filgrastim (e.g., G-CSF, Neupogen), a sargramostim (GM-CSF, Leukine), an immunization, an immunoglobulin, an immunosuppressive (e.g., basiliximab, cyclosporine, daclizumab), a growth hormone, a hormone replacement drug, an estrogen receptor modulator, a mydriatic, a cycloplegic, an alkylating agent, an antimetabolite, a mitotic inhibitor, a radiopharmaceutical, an antidepressant, antimanic agent, an antipsychotic, an anxiolytic, a hypnotic, a sympathomimetic, a stimulant, donepezil, tacrine, an asthma medication, a beta agonist, an inhaled steroid, a leukotriene inhibitor, a methylxanthine, a cromolyn, an epinephrine or analog, dornase alpha (Pulmozyme), a cytokine or a cytokine antagonist. Suitable dosages are well known in the art. See, e.g., Wells et al., eds., Pharmacotherapy Handbook, 2nd Edition, Appleton and Lange, Stamford, CT (2000); PDR Pharmacopoeia, Tarascon Pocket Pharmacopoeia 2000, Deluxe Edition, Tarascon Publishing, Loma Linda, CA (2000); Nursing 2001 Handbook of Drugs, 21st edition, Springhouse Corp., Springhouse, PA, 2001; Health Professional's Drug Guide 2001, ed., Shannon, Wilson, Stang, Prentice-Hall, Inc, Upper Saddle River, NJ.

### Therapeutic Treatments

Typically, treatment of psoriasis is affected by administering an effective amount or dosage of an anti-IL-23 antibody composition that total, on average, a range from at least about 0.01 to 500 milligrams of an anti-IL-23 antibody per kilogram of patient per dose, and, preferably, from at least about 0.1 to 100 milligrams antibody/kilogram of patient per single or multiple administration, depending upon the specific activity of the active agent contained in the composition. Alternatively, the effective serum concentration can comprise 0.1-5000 µg/ml serum concentration per single or multiple administrations. Suitable dosages are known to medical practitioners and will, of course, depend upon the particular disease state, specific activity of the composition being administered, and the particular patient undergoing treatment. In some instances, to achieve the desired therapeutic amount, it can be necessary to provide for repeated administration, *i.e.,* repeated individual administrations of a particular monitored or metered dose, where the individual administrations are repeated until the desired daily dose or effect is achieved.

Preferred doses can optionally include 0.1, 0.2, 0.3, 0.4, 0.5, 0.6, 0.7, 0.8, 0.9, 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99 and/or 100-500 mg/kg/administration, or any range, value or fraction thereof, or to achieve a serum concentration of 0.1, 0.5, 0.9, 1.0, 1.1, 1.2, 1.5, 1.9, 2.0, 2.5, 2.9, 3.0, 3.5, 3.9, 4.0, 4.5, 4.9, 5.0, 5.5, 5.9, 6.0, 6.5, 6.9, 7.0, 7.5, 7.9, 8.0, 8.5, 8.9, 9.0, 9.5, 9.9, 10, 10.5, 10.9, 11, 11.5, 11.9, 20, 12.5, 12.9, 13.0, 13.5, 13.9, 14.0, 14.5, 4.9, 5.0, 5.5., 5.9, 6.0, 6.5, 6.9, 7.0, 7.5, 7.9, 8.0, 8.5, 8.9, 9.0, 9.5, 9.9, 10, 10.5, 10.9, 11, 11.5, 11.9, 12, 12.5, 12.9, 13.0, 13.5, 13.9, 14, 14.5, 15, 15.5, 15.9, 16, 16.5, 16.9, 17, 17.5, 17.9, 18, 18.5, 18.9, 19, 19.5, 19.9, 20, 20.5, 20.9, 21, 22, 23, 24, 25, 26, 27, 28, 29, 30, 35, 40, 45, 50, 55, 60, 65, 70, 75, 80, 85, 90, 96, 100, 200, 300, 400, 500, 600, 700, 800, 900, 1000, 1500, 2000, 2500, 3000, 3500, 4000, 4500, and/or 5000 µg/ml serum concentration per single or multiple administration, or any range, value or fraction thereof.

Alternatively, the dosage administered can vary depending upon known factors, such as the pharmacodynamic characteristics of the particular agent, and its mode and route of administration; age, health, and weight of the recipient; nature and extent of symptoms, kind of concurrent treatment, frequency of treatment, and the effect desired. Usually a dosage of active ingredient can be about 0.1 to 100 milligrams per kilogram of body weight. Ordinarily 0.1 to 50, and, preferably, 0.1 to 10 milligrams per kilogram per administration or in sustained release form is effective to obtain desired results.

As a non-limiting example, treatment of humans or animals can be provided as a one-time or periodic dosage of at least one antibody of the present invention 0.1 to 100 mg/kg, such as 0.5, 0.9, 1.0, 1.1, 1.5, 2, 3, 4, 5, 6, 7, 8, 9, 10, 11, 12, 13, 14, 15, 16, 17, 18, 19, 20, 21, 22, 23, 24, 25, 26, 27, 28, 29, 30, 40, 45, 50, 60, 70, 80, 90 or 100 mg/kg, per day, on at least one of day 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40, or, alternatively or additionally, at least one of week 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, or 52, or, alternatively or additionally, at least one of 1, 2, 3, 4, 5, 6,, 7, 8, 9, 10, 11, 12, 13, 14, 15, 16, 17, 18, 19, or 20 years, or any combination thereof, using single, infusion or repeated doses.

Dosage forms (composition) suitable for internal administration generally contain from about 0.001 milligram to about 500 milligrams of active ingredient per unit or container. In these pharmaceutical compositions the active ingredient will ordinarily be present in an amount of about 0.5-99.999% by weight based on the total weight of the composition.

For parenteral administration, the antibody can be formulated as a solution, suspension, emulsion, particle, powder, or lyophilized powder in association, or separately provided, with a pharmaceutically acceptable parenteral vehicle. Examples of such vehicles are water, saline, Ringer's solution, dextrose solution, and 1-10% human serum albumin. Liposomes and nonaqueous vehicles, such as fixed oils, can also be used. The vehicle or lyophilized powder can contain additives that maintain isotonicity (e.g., sodium chloride, mannitol) and chemical stability (e.g., buffers and preservatives). The formulation is sterilized by known or suitable techniques.

Suitable pharmaceutical carriers are described in the most recent edition of Remington's Pharmaceutical Sciences, A. Osol, a standard reference text in this field.

### Alternative Administration

Many known and developed modes can be used according to the present invention for administering pharmaceutically effective amounts of an anti-IL-23 antibody. While pulmonary administration is used in the following description, other modes of administration can be used according to the present invention with suitable results. IL-23 specific antibodies of the present invention can be delivered in a carrier, as a solution, emulsion, colloid, or suspension, or as a dry powder, using any of a variety of devices and methods suitable for administration by inhalation or other modes described here within or known in the art.

### Parenteral Formulations and Administration

Formulations for parenteral administration can contain as common excipients sterile water or saline, polyalkylene glycols, such as polyethylene glycol, oils of vegetable origin, hydrogenated naphthalenes and the like. Aqueous or oily suspensions for injection can be prepared by using an appropriate emulsifier or humidifier and a suspending agent, according to known methods. Agents for injection can be a non-toxic, non-orally administrable diluting agent, such as aqueous solution, a sterile injectable solution or suspension in a solvent. As the usable vehicle or solvent, water, Ringer's solution, isotonic saline, etc. are allowed; as an ordinary solvent or suspending solvent, sterile involatile oil can be used. For these purposes, any kind of involatile oil and fatty acid can be used, including natural or synthetic or semisynthetic fatty oils or fatty acids; natural or synthetic or semisynthtetic mono- or di- or tri-glycerides. Parental administration is known in the art and includes, but is not limited to, conventional means of injections, a gas pressured needle-less injection device as described in U.S. Pat. No. 5,851,198, and a laser perforator device as described in U.S. Pat. No. 5,839,446.

### Alternative Delivery

The invention further relates to the administration of an anti-IL-23 antibody by parenteral, subcutaneous, intramuscular, intravenous, intrarticular, intrabronchial, intraabdominal, intracapsular, intracartilaginous, intracavitary, intracelial, intracerebellar, intracerebroventricular, intracolic, intracervical, intragastric, intrahepatic, intramyocardial, intraosteal, intrapelvic, intrapericardiac, intraperitoneal, intrapleural, intraprostatic, intrapulmonary, intrarectal, intrarenal, intraretinal, intraspinal, intrasynovial, intrathoracic, intrauterine, intravesical, intralesional, bolus, vaginal, rectal, buccal, sublingual, intranasal, or transdermal means. An anti-IL-23 antibody composition can be prepared for use for parenteral (subcutaneous, intramuscular or intravenous) or any other administration particularly in the form of liquid solutions or suspensions; for use in vaginal or rectal administration particularly in semisolid forms, such as, but not limited to, creams and suppositories; for buccal, or sublingual administration, such as, but not limited to, in the form of tablets or capsules; or intranasally, such as, but not limited to, the form of powders, nasal drops or aerosols or certain agents; or transdermally, such as not limited to a gel, ointment, lotion, suspension or patch delivery system with chemical enhancers such as dimethyl sulfoxide to either modify the skin structure or to increase the drug concentration in the transdermal patch (Junginger, et al. In "Drug Permeation Enhancement;" Hsieh, D. S., Eds., pp. 59-90 (Marcel Dekker, Inc. New York 1994), or with oxidizing agents that enable the application of formulations containing proteins and peptides onto the skin (WO 98/53847), or applications of electric fields to create transient transport pathways, such as electroporation, or to increase the mobility of charged drugs through the skin, such as iontophoresis, or application of ultrasound, such as sonophoresis (U.S. Pat. Nos. 4,309,989 and 4,767,402).

Having generally described the invention, the same will be more readily understood by reference to the following Examples, which are provided by way of illustration and are not intended as limiting. Further details of the invention are illustrated by the following non-limiting Examples.

### Example 1: Comparison of the efficacy and safety of guselkumab (GUS) with the anti-TNFα antibody adalimumab (ADA) and placebo (PBO) in patients treated through one year.

To confirm findings from earlier studies, two pivotal, phase III trials were conducted: VOYAGE 1 and VOYAGE 2. Efficacy, safety, and patient-reported outcome (PRO) findings from VOYAGE 1 are reported, which compared guselkumab with adalimumab, a widely used TNF-α inhibitor, and placebo in psoriasis patients treated continuously for one year. In addition, an additional trial, known as VOYAGE 2 (described in Example 2), included a randomized withdrawal period.
**VOYAGE 1 Materials/Methods Summary:** VOYAGE 1 is a phase 3, randomized, double-blind, placebo- and active comparator-controlled trial. Eligible patients (age≥18 years) had plaque psoriasis for ≥6 months, an Investigator's Global Assessment [IGA] score ≥3, a Psoriasis Area and Severity Index [PASI] score ≥12, and body surface area involvement ≥10%, and were candidates for systemic therapy or phototherapy. At baseline, 837 patients were randomized to either PBO at weeks 0/4/12 then GUS 100 mg at weeks 16/20, and q8wk through week 44 (n=174); GUS 100 mg at weeks 0/4/12, and q8wk through week 44 (n=329); or ADA 80 mg at week 0, 40 mg at week 1, and 40 mg q2wk through week 47 (n=334). The co-primary endpoints were the proportions of GUS vs PBO patients achieving cleared/minimal disease (IGA 0/1) and 90% improvement in PASI score (PASI 90) at week16. Other endpoints included the proportions of GUS vs ADA patients achieving IGA 0/1, IGA 0, PASI 90, and PASI 100 at weeks 16/24/48, and a Dermatology Life Quality Index score of 0/1 (DLQI 0/1), indicating no impact of psoriasis on health-related quality of life, at weeks 24/48. Safety was monitored through week 48.
**VOYAGE 1 Summary Results:** Significantly higher (p<0.001) proportions of patients in the GUS vs PBO group achieved IGA 0/1 (85.1% vs 6.9%) and PASI 90 (73.3% vs 2.9%) at week 16. GUS was also superior to ADA based on the proportions of patients achieving IGA 0/1 (85.1% vs 65.9%) and PASI 90 (73.3% vs 49.7%) at week 16 (p<0.001). Likewise, significantly higher (p<0.001) proportions of patients achieved responses to GUS vs ADA, respectively, at week 24: IGA 0 (52.6% vs 29.3%), IGA 0/1 (84.2% vs 61.7%), PASI 100 (44.4% vs 24.9%), and PASI 90 (80.2% vs 53.0%). Corresponding response rates at week 48 were: IGA 0 (50.5% vs 25.7%), IGA 0/1 (80.5% vs 55.4%), PASI 100 (47.4% vs 23.4%), and PASI 90 (76.3% vs 47.9%), all p<0.001. The proportion of patients with a DLQI score of 0/1 among GUS vs ADA patients was 60.9% vs 39.5% at week 24 and 62.5% vs 38.9% at week 48 (both p<0.001). Through week 48, adverse events occurred in 73.9% and 74.5% of GUS and ADA patients, respectively; serious adverse event rates were also similar for the GUS and ADA groups (4.9% vs 4.5%). Serious infections occurred in two GUS patients and three ADA patients. Two malignancies (prostate and breast) occurred in the GUS group. One myocardial infarction occurred in each active treatment group.
**VOYAGE 1 Summary Conclusions:** GUS was superior to ADA in treating moderate-to-severe psoriasis, and was well tolerated, through one year of treatment.
**Background:** Guselkumab, an interleukin-23 (IL-23) blocker, was superior to adalimumab in treating moderate-to-severe psoriasis in a phase II trial.
**Objectives:** To compare efficacy and safety of guselkumab with adalimumab and placebo in psoriasis patients treated for one year.
**Methods:** Patients were randomized to guselkumab 100 mg at week0/4/12, then q8wk (n=329); placebo at week0/4/16 followed by guselkumab 100 mg at week16/20, then q8wk (n=174); or adalimumab 80 mg at week0, 40 mg at week1, 40mg q2wk (n=334). Physician-reported outcomes (Investigators Global Assessment [IGA], Psoriasis Area and Severity Index [PASI]), patient-reported outcomes (PROs; Dermatology Life Quality Index [DLQI], Psoriasis Symptom and Sign Diary [PSSD]), and safety were evaluated through week48.
**Results:** Guselkumab was superior (p<0.001) to placebo at week16 (85.1% vs. 6.9% [IGA0/1] and 73.3% vs. 2.9%, [PASI90]). Guselkumab was also superior (p<0.001) vs. adalimumab for IGA0/1 and PASI90 at week16 (85.1% vs. 65.9%, 73.3% vs. 49.7%); week24 (84.2% vs. 61.7%, 80.2% vs. 53.0%); and week48 (80.5% vs. 55.4%, 76.3% vs. 47.9%). Furthermore, guselkumab significantly improved PROs through week48. Adverse event rates were comparable between treatments through week48.
**Limitations:** Analyses were limited to 48weeks.
**Conclusions:** Guselkumab demonstrated superior efficacy compared with adalimumab and is well-tolerated in psoriasis patients through one year.

### MATERIALS AND METHODS

### Patients

The trial enrolled patients aged ≥18 years with moderate-to-severe plaque psoriasis (i.e., Investigator's Global Assessment [IGA] ≥3, Psoriasis Area and Severity Index [PASI] ≥12, and body surface area [BSA] involvement ≥10%) for at least 6 months who were candidates for systemic therapy or phototherapy. Patients were ineligible if they had a history or current signs of severe, progressive, or uncontrolled medical conditions or had current or history of malignancy within 5 years, except nonmelanoma skin cancer (NMSC). Patients with history or symptoms of active tuberculosis (TB) were excluded. Patients could not participate if they had received guselkumab or adalimumab previously; other anti-TNF-α therapy within 3 months; other treatment targeting IL-12/23, IL-17, or IL-23 within 6 months; or any systemic immunosuppressants (e.g., methotrexate) or phototherapy within 4 weeks.

### Study design

VOYAGE 1 was a phase III, randomized, double-blind, placebo- and active comparator-controlled trial conducted at 104 global sites (December 2014 - April 2016). The study comprised an active-comparator period when guselkumab was compared with adalimumab (week 0-48) and a placebo-controlled period (weeks 0-16), after which placebo patients crossed over to receive guselkumab through week 48. Patients were randomized at baseline in a 2:1:2 ratio to guselkumab 100 mg at weeks 0, 4, 12, and every-8-weeks thereafter through week 44; placebo at weeks 0, 4, 12 followed by guselkumab 100 mg at weeks 16, 20, and every-8-weeks thereafter through week 44; or adalimumab 80 mg at week 0, 40 mg at week 1, and 40 mg every-2weeks thereafter through week 47. To maintain the blind, matching placebos were utilized. An institutional review board or ethics committee approved the study protocol at participating sites; patients provided written informed consent before study initiation.

### Assessments

Efficacy was evaluated using the IGA, PASI, scalp-specific IGA (ss-IGA), fingernail Physician's Global Assessment (f-PGA), Nail Psoriasis Area and Severity Index (NAPSI), and PGA of the hands/feet (hf-PGA). Patient-reported outcomes were assessed utilizing the Dermatology Life Quality Index (DLQI) and Psoriasis Symptom and Sign Diary (PSSD). Safety monitoring included collection of adverse events (AEs) and laboratory testing.

Antibodies-to-guselkumab were detected using a highly sensitive and drug-tolerent electrochemiluminescence immunoassay; the sensitivity was 3.1 ng/mL in guselkumab-free serum and 15 ng/mL with serum guselkumab concentrations up to 3.125 µg/mL, which exceeds mean trough serum guselkumab levels.

### Statistical Analyses

Co-primary endpoints were the proportions of patients achieving an IGA score of cleared or minimal disease (IGA 0/1) and 90% improvement in PASI response (PASI 90) at week 16 in the guselkumab group compared with placebo. Major secondary endpoints were also measured. All randomized patients were included in the primary and selected secondary efficacy analyses; data were analyzed by randomized treatment group. The primary and major secondary analyses were tested in a fixed sequence to control for multiplicity.

The co-primary endpoints and binary major secondary endpoints were analyzed using a Cochran-Mantel-Haenszel (CMH) chi-square statistical test stratified by pooled investigator site. With a sample size of approximately 750 patients, the power to detect a significant difference was >99% for both co-primary endpoints. Continuous response parameters were compared using an analysis of variance model with pooled investigator site as a covariate. All statistical testing was performed 2-sided (α=0.05).

Patients who discontinued study agent due to lack of efficacy or an AE of psoriasis worsening *or* who started a protocol-prohibited psoriasis treatment were considered non-responders for binary endpoints, and had baseline values carried over for continuous endpoints. Other patients with missing data were considered non-responders for binary endpoints (non-responder imputation) and had last observation carried forward for continuous endpoints (and all PSSD endpoints).

Safety analyses included all patients who received at least one administration of study agent and were summarized by actual treatment. The proportion of patients with antibodies-to-guselkumab was summarized for those receiving at least one dose of the biologic.

### RESULTS

At baseline, 837 patients were randomized to placebo (n=174), guselkumab (n=329), or adalimumab (n=334). Overall, 6.9%, 8.5%, and 15.6% of patients discontinued treatment in the placebo, guselkumab, and adalimumab groups, respectively, through week 48. Demographic and disease characteristics were comparable across treatment groups at baseline ).

### Clinical responses

Guselkumab was superior to both placebo and/or adalimumab with respect to co-primary endpoints and all major secondary endpoints (all p<0.001). Compared with placebo, significantly higher proportions of patients in the guselkumab group achieved IGA 0/1 (6.9% vs. 85.1%) and PASI 90 (2.9% vs. 73.3%) at week 16. Additionally, the proportions achieving at least 75% improvement in PASI (PASI 75) as well as IGA 0 and PASI 100 were significantly higher for guselkumab vs. placebo at week 16. Guselkumab was superior to adalimumab as measured by the proportion of patients achieving IGA 0/1 (85.1% vs. 65.9%), PASI 90 (73.3% vs. 49.7%), and PASI 75 (91.2% vs. 73.1%) at week 16. Significantly better responses to guselkumab compared with adalimumab were maintained at week 24 (IGA 0 [52.6% vs. 29.3%], IGA 0/1 [84.2% vs. 61.7%], and PASI 90 [80.2% vs. 53.0%]) and week 48 (50.5% vs. 25.7%, 80.5% vs. 55.4%, and 76.3% vs. 47.9%, respectively). Additionally, higher proportions of patients receiving guselkumab attained response in higher PASI categories compared with adalimumab at week 48. After initiating guselkumab at week 16, patients in the placebo crossover group achieved responses similar to those observed in the guselkumab group.

### Regional psoriasis measures

Regional psoriasis was evaluated based on the ss-IGA, f-PGA, NAPSI, and hf-PGA assessments. The proportion of patients achieving ss-IGA 0/1 (absent/very mild scalp psoriasis) in the guselkumab group was significantly higher compared with placebo (83.4% vs. 14.5%, p<0.001) at week 16; significantly better responses to guselkumab vs. adalimumab were observed at week 24 (p<0.001) and week 48 (p=0.045). The proportion of patients achieving f-PGA 0/1 (clear/minimal) and percent improvement in NAPSI were significantly higher for guselkumab vs. placebo at week 16 (p<0.001). The f-PGA responses were comparable at week 24, though guselkumab was superior to adalimumab by week 48 (p=0.038). Mean percent improvement in NAPSI with guselkumab was significantly higher than that for placebo at week 16 (p<0.001) and comparable between guselkumab and adalimumab at weeks 24 and 48. Finally, the proportion of patients achieving hf-PGA 0/1 (clear/almost clear) was significantly higher for guselkumab vs. placebo at week 16, and responses to guselkumab were superior to adalimumab at weeks 24 and 48 (p<0.001).

### Health-related quality of life measures

At week 16, the improvement from baseline in DLQI was significantly greater in the guselkumab group compared with placebo (mean change, -0.6 vs. -11.2), as were the proportions of patients achieving DLQI 0/1 (no impact of psoriasis on HRQoL) (both p<0.001). At weeks 24 and 48, both improvements from baseline in DLQI and proportions of patients achieving DLQI 0/1were significantly higher for guselkumab vs. adalimumab (p<0.001).

At week 16, the improvement from baseline in the PSSD symptom score was significantly greater for guselkumab vs. placebo (mean change, -3.0 vs. -41.9); mean changes in the PSSD sign score were similarly favorable for guselkumab (both p<0.001). Likewise, at weeks 24 and 48, mean changes in the PSSD symptom and sign scores in the guselkumab group were significantly greater than those in the adalimumab group (p<0.001). The proportions of patients achieving a PSSD symptom score=0 with guselkumab and adalimumab, respectively, were 36.3% and 21.6% at week 24, and the significantly better response to guselkumab was maintained at week 48 (p<0.001). Similar results were observed for the proportions of patients achieving a PSSD sign score=0 at weeks 24 and 48 (p<0.001).

### Safety outcomes

During the placebo-controlled period (weeks 0-16), the proportion of patients with at least one AE was comparable across treatment groups, and the most commonly reported events were nasopharyngitis and upper respiratory tract infection in all three groups. Serious AEs (SAEs) and AEs leading to study agent discontinuation occurred infrequently and in similar proportions of patients for each treatment. Rates of overall infections and infections requiring antibiotic treatment were comparable across treatment groups. Two patients in the adalimumab group experienced serious infections (both cellulitis). One NMSC (i.e., basal cell carcinoma [BCC]) was reported in the guselkumab group, and no other malignancies occurred in any group. One myocardial infarction (i.e., major adverse cardiovascular event [MACE]) occurred in each active treatment group through week 16.

The types and patterns of AEs reported through week 48 were similar to those reported during the placebo-controlled period. The proportions of patients with at least one AE, an AE leading to discontinuation, or an SAE were similar in the guselkumab and adalimumab groups. Between weeks 16-48, serious infections were reported in two patients in the guselkumab group (i.e., cellulitis in one and thigh abscess with post-operative wound infection in another) and two in the adalimumab group (i.e., one abdominal abscess and one staphylococcal pneumonia with a fatal outcome in the patient with abdominal wall cellulitis reported earlier). Overall infections and infections requiring antibiotic treatment occurred at comparable rates across treatment groups. No AEs of active tuberculosis or opportunistic infection were reported during the study. Two additional NMSCs (i.e., one BCC each in the guselkumab and adalimumab groups) and two malignancies (i.e., prostate and breast in the guselkumab group) were reported through week 48. No additional MACE occurred after week 16. A single suicide attempt was reported in an adalimumab patient. Incidence rates of candidiasis and neutropenia were low and comparable between the guselkumab and placebo groups (data not shown), and no events of Crohn's disease were reported through week 48.

Through week 48, the proportion of patients with an ISR (2.2% vs. 9.0%) and the proportion of injections associated with ISRs (0.5% vs 1.2%) were lower for guselkumab compared with adalimumab; most ISRs were considered mild. Laboratory abnormalities rates were low, and no between-group differences were noted (data not shown). Antibodies-to-guselkumab were detected in 26/492 patients (5.3%) through week 44; titers were generally low (81% ≤1:320). No apparent association was observed between antibody development and either reduced efficacy or ISR occurrence (data not shown).

### DISCUSSION

The findings in VOYAGE 1 study, together with the VOYAGE 2 results described in Example 2 below, confirm that two injections of guselkumab 100 mg (weeks 0 and 4) and every 8-week maintenance therapy effectively treats moderate-to-severe psoriasis. Guselkumab was superior to placebo by substantial margins at week 16 using two rigorous endpoints (IGA 0/1 and PASI 90). The onset of action of guselkumab was rapid, with significant response evident as early as week 2 compared with placebo. Guselkumab was also superior to adalimumab, which is a widely used and very effective subcutaneous TNF-α inhibitor, at the week-16 endpoints of IGA 0/1, PASI 90, and PASI 75. Response rates continued to improve with guselkumab beyond week 16. At weeks 24 and 48, approximately half of all patients in the guselkumab group achieved complete clearance (IGA 0), which is associated with optimal HRQoL for patients with psoriasis. Patient-reported outcome endpoints (PSSD and DLQI) based on total change, or indicating minimal/no impact on HRQoL or no symptoms or signs of psoriasis, demonstrated guselkumab responses that were superior to placebo at week 16 and adalimumab at weeks 24 and 48.

This study assessed multiple areas of the body where psoriasis is challenging to treat, and guselkumab was highly effective in all regions based on rigorous endpoints. Guselkumab was superior to placebo at week 16 and to adalimumab at weeks 24/48, indicating complete or nearly complete clearance of scalp and hand/foot psoriasis in at least 75% of guselkumab-treated patients. Based on both the proportion of patients with clear/minimal fingernail psoriasis (f-PGA 0/1) and the mean percent improvement in NAPSI, guselkumab was superior to placebo at week 16. Nail responses were comparable between active treatments at weeks 24 and 48, and guselkumab was superior to adalimumab (75% vs. 62%) for f-PGA 0/1 at week 48.

Rates and types of AEs, SAEs, and laboratory abnormalities were generally comparable between the guselkumab and placebo groups through week 16 and between the guselkumab and adalimumab groups through week 48. Rates of serious infections, malignancies, and MACE were low across treatment groups. No notable differences in the incidence of neutropenia or candidiasis, were observed between the guselkumab and control groups. No AEs of Crohn's disease occurred in any treatment group, and one suicide attempt was reported in the adalimumab group. The number of injections and proportions of patients with ISRs were higher for adalimumab compared with guselkumab. The size and duration of this study may not allow for assessment of uncommon events or those with a long latency; however, longer-term treatment will be evaluated in ongoing study extensions.

VOYAGE 1 confirms the role of IL-23 in the pathogenesis of psoriasis. TNF-α inhibitors are effective in many diseases, and TNF-α is involved in normal systemic inflammatory and immunologic processes. Selective targeting of the IL-23 pathway provides more psoriasis-specific cytokine inhibition with a higher degree of efficacy while maintaining a favorable safety profile, when compared with TNF-α blockade. IL-23 is a key driver of Th17 cell differentiation and survival and an upstream regulator of IL-17A, a central pro-inflammatory effector cytokine implicated in the pathogenesis of psoriasis. Moreover, IL-23 stimulates the production of other Th17 cytokines (e.g., IL-22) by other cell types, including innate lymphoid cells type 3 (ILC3) cells and γδ T-cells. Therefore, inhibition of IL-23 blocks downstream production of IL-17A, IL-22, and other cell types. Since many IL-17A-producing cells are dependent upon IL-23 for survival, inhibition of IL-23 may reduce the number of these pathogenic cells. This may explain the long duration of effect and allow for the convenient dosing interval of guselkumab compared with both anti-TNF-α and anti-IL-17 agents.

The findings, together with those from VOYAGE 2, demonstrate the superior efficacy of guselkumab compared with adalimumab in psoriasis, efficacy in regional disease of the scalp, nails, and hands/feet, and a positive safety profile. The favorable safety profile through one year is not unexpected, considering the reassuring long-term safety findings reported for a related treatment, ustekinumab, which blocks both IL-12 and IL-23. Study extensions will continue to examine the efficacy and safety of guselkumab and add to the understanding of long-term IL-23 blockade based on studies of ustekinumab.

### Example 2: VOYAGE 2 Results

To confirm the therapeutic potential of guselkumab, the Phase 3 VOYAGE 1 and VOYAGE 2 studies assessed the efficacy and safety of guselkumab versus placebo and adalimumab. VOYAGE 1 assessed continuous 1-year treatment, and VOYAGE 2 evaluated the efficacy and safety of interrupted treatment, as treatment gaps frequently occur in clinical practice. Additionally, VOYAGE 2 assessed the transition from adalimumab to guselkumab, providing clinically-relevant information about patients who switch biologics.

### MATERIALS AND METHODS

### Patients

Adults (aged ≥18 years) with moderate-to-severe plaque-type psoriasis were eligible. Major inclusion/exclusion criteria are summarized in VOYAGE 1 above. The study protocol was approved by an investigational review board at each site, and written informed consent was provided by all patients.

### Study design

VOYAGE 2 was a Phase 3, multicenter, randomized, double-blind, placebo- and adalimumab comparator-controlled study (NCT02207244) conducted in 115 global sites between November 2014 and June 2016. The study consisted of a placebo-controlled period (weeks 0 to 16), an active comparator-controlled period (weeks 0 to 28), and a randomized withdrawal and retreatment period (weeks 28 to 72). Study results through week 48 are presented here. Patients were randomized at baseline 2:1:1 to guselkumab 100 mg at weeks 0, 4, 12, and 20; placebo at weeks 0, 4, 12, then guselkumab at weeks 16 and 20; or adalimumab 80 mg at week 0, 40 mg at week 1, and every-two-weeks (q2w) thereafter through week 23.

At week 28, guselkumab-treated patients achieving a 90% improvement from baseline in Psoriasis Area and Severity Index (PASI 90; responders) were re-randomized in a 1:1 ratio to guselkumab or placebo. Upon loss of ≥50% of week-28 PASI response, patients were retreated with guselkumab, another dose 4 weeks later, then q8w thereafter. Guselkumab nonresponders continued guselkumab treatment. Placebo→guselkumab nonresponders at week 28 continued guselkumab q8w, while responders received placebo q8w beginning at week 28. Upon loss of ≥50% of week 28-PASI response, patients were retreated with guselkumab, followed by another dose 4 weeks later, then q8w thereafter. Adalimumab nonresponders initiated guselkumab at week 28, another dose 4 weeks later, then q8w thereafter. Adalimumab responders received placebo and upon loss of ≥50% of week 28-PASI response, initiated guselkumab, another dose 4 weeks later, then q8w thereafter. To maintain the blind, both guselkumab and adalimumab placebos were administered as necessary.

### Efficacy and safety assessments

Efficacy was assessed through week 24 and safety through week 28. Key efficacy, including general and regional psoriasis, patient-reported outcomes, and safety assessments are discussed in detail in the VOYAGE 1 study. In addition, the Medical Outcomes Study 36-Item Short Form (SF-36) was evaluated in this study.

### Study Endpoints

The co-primary endpoints were the proportions of patients achieving an IGA score of cleared (0) or minimal (1) at week 16 and the proportion of patients achieving a PASI 90 response at week 16, comparing the guselkumab and placebo groups. Major secondary endpoints were also measured. Regional psoriasis endpoints evaluating the scalp, fingernails, and hand/feet were described in detail elsewhere.

### Statistical analysis

All randomized patients were included in the primary analysis and some secondary efficacy analyses according to their assigned treatment group. To assess maintenance dosing versus treatment withdrawal for the major secondary endpoints, all patients who underwent the second randomization and had PASI evaluation after week 28 were included in the analyses.

The co-primary endpoints and binary major secondary endpoints were analyzed using a two-sided Cochran-Mantel-Haenszel (CMH) chi-squared statistical test (α=0.05) stratified by investigator site. Continuous response parameters were compared using an analysis of variance model with site as a covariate. For the time to loss of PASI 90 response, the log-rank test stratified by site was used.

Patients who discontinued study treatment due to lack of efficacy or an AE of worsening of psoriasis, or who started a protocol-prohibited medication/therapy that could improve psoriasis were considered treatment failures. Patients who met treatment failure criteria before week 16 and patients not returning for week-16 evaluation were considered nonresponders for the week-16 primary endpoint. For patients randomized to placebo, only those who crossed-over to receive guselkumab at/after week 16 were included in the efficacy analyses after week 16.

To control the overall Type 1 error rate, the primary analysis and major secondary analyses were tested in a fixed sequence, with the first major secondary endpoint being tested only if the co-primary endpoints were met, and the subsequent endpoint(s) tested only if the preceding endpoint in the sequence were met.

Safety analyses included all patients who received at least one study agent administration. Adverse events and serious adverse events (SAEs) were grouped according to the treatment received. Antibodies to guselkumab were analyzed.

### RESULTS

### Patient disposition and baseline demographic characteristics

A total of 1279 patients were screened and 992 were randomized 2:1:1 to receive guselkumab (n=496), placebo (n=248), or adalimumab (n=248) (Fig 2). Overall, 9.7% (96/992) of patients discontinued the study agent through week 48 (guselkumab: 7.9%; placebo: 11.7%; adalimumab: 11.3%). Baseline demographics and disease characteristics were generally comparable among groups.

### Efficacy

Guselkumab was superior to both placebo and/or adalimumab with respect to the co-primary endpoints and all major secondary endpoints (all p<0.001).

### Placebo-controlled period (weeks 0-16)

At week 16, significantly greater proportions of guselkumab patients achieved an IGA of cleared (0) or minimal (1) compared with the placebo patients (84.1% vs. 8.5%; p<0.001), and achieved a PASI 90 response (70.0% vs. 2.4%; p<0.001) (co-primary endpoints). Significantly higher PASI percent improvement was observed as early as week 2 in guselkumab vs. placebo patients (p<0.001). Additionally, at week 16, significantly higher proportions of guselkumab patients achieved PASI 75 and PASI 100 responses compared with placebo patients. Guselkumab patients achieved greater improvement in all regional psoriasis outcome assessments, compared with placebo at week 16 including: ss-IGA, f-PGA, NAPSI, and hf-PGA. Similar to VOYAGE 1, guselkumab was superior to placebo at week 16 in all patient-reported outcomes including Dermatology Life Quality Index (DLQI) and Psoriasis Symptom and Sign Diary (PSSD), and in addition, SF-36.

### Active-comparator period (weeks 0-24)

Significantly greater proportions of patients in the guselkumab group achieved IGA 0/1, PASI 90, and PASI 75 responses at week 16. At week 24, significantly higher response rates were maintained in the guselkumab vs. adalimumab patients for IGA 0 (51.5% vs. 31.5%), IGA 0/1 (83.5% vs. 64.9%), PASI 90 (75.2% vs. 54.8%), and PASI 100 (44.2% vs. 26.6%). Consistent with VOYAGE 1, greater improvements in regional psoriasis disease scores were observed at week 24 in guselkumab patients compared with adalimumab patients, with the exception of f-PGA 0/1 and percent improvement in NAPSI, which were comparable. At week 24, the proportions of patients achieving DLQI 0/1, mean changes in the PSSD symptom and sign scores, proportions of patients achieving a PSSD symptom score of 0 and a sign score of 0 were significantly greater in the guselkumab group than in the adalimumab group (p<0.001)

### Randomized withdrawal and retreatment period (weeks 28-48)

PASI 90 responses were better maintained in guselkumab week-28 responders continuing guselkumab (maintenance group) versus responders re-randomized to placebo (withdrawal group). The median time to loss of PASI 90 response was 15.2 weeks for patients randomized to the withdrawal group. Among patients withdrawn from guselkumab at week 28, PASI 90 response rates began to diverge from the maintenance group at week 32. Through week 48, 88.6% of the maintenance patients sustained a PASI 90 response versus 36.8% of withdrawal patients. In addition, at week 48, clinical responses (IGA, PASI) were significantly greater in maintenance group than withdrawal group (p<0.001). Improvements in DLQI and PSSD symptom or sign scores from baseline were also significantly greater at week 48 in the maintenance vs. withdrawal groups (both p<0.001). Through week 48, a small number of patients (n=16) were retreated with guselkumab.

### Switching adalimumab nonresponders to guselkumab

Overall, 112 adalimumab nonresponders initiated guselkumab at week 28 (5 weeks after the last adalimumab dose). In these patients, PASI 90 (relative to baseline) and PASI 100 response rates increased after switching, reaching 66.1% and 28.6%, respectively at week 48.

### Safety

### Placebo-controlled period (weeks 0-16)

The proportions of patients with at least one AE, AEs leading to discontinuation, and SAEs were comparable between the guselkumab and placebo groups. The most commonly reported events were nasopharyngitis, headache, and upper respiratory tract infection. Rates of infections, infections requiring treatment, and serious infections were similar among groups. No malignancies or nonmelanoma skin cancers (NMSC) were reported through week 16. One major adverse cardiovascular event (MACE) (myocardial infarction [MI]) occurred in the adalimumab group. A higher proportion of adalimumab patients had injection site reactions (ISR) (6.9% vs 2.6%) and injections resulting in ISRs (1.5% vs. 0.9%) compared with guselkumab patients. All injection site reactions were mild.

### Active-comparator period (weeks 0-28)

The types of AEs were similar to those reported in the placebo-controlled period. The proportions of patients with at least one AE, AEs leading to discontinuation, and SAEs were comparable between the guselkumab and adalimumab groups. Infections and infections requiring treatment were also comparable between guselkumab and adalimumab groups. Three serious infections each were reported in the guselkumab (bronchitis, erysipelas, and soft tissue infection) and adalimumab groups (two cases of tuberculosis [one disseminated], and one injection site abscess). One malignancy (prostate cancer) and two NMSCs (one squamous cell carcinoma [SCC] in the guselkumab group and one basal cell carcinoma [BCC] in the placebo→guselkumab group) were reported. Two MACE (one MI each in guselkumab and adalimumab groups) were reported.

### Randomized withdrawal and retreatment period (weeks 28-48)

From weeks 28-48, no patients discontinued due to AEs; one serious infection (appendicitis) was reported in the maintenance group. No additional malignancies, NMSC, or MACE were reported. No AEs were reported among the 16 retreated patents.

### Additional safety through week 48

Through week 48, one additional BCC and one additional SCC of the skin occurred in the placebo→guselkumab group (data not shown). Between weeks 28-48, one additional MACE (MI) was reported in a placebo→guselkumab patient. No events of serious infections, malignancies, or MACE occurred in the adalimumab→guselkumab group. No deaths, opportunistic infections, hypersensitivity, or anaphylactic reactions occurred through week 48. Rates of abnormal labs were low and comparable between the treatment groups through week 48. Antibodies to guselkumab were detected in 57/869 patients (6.6%) through week 48; titers were generally low (88% ≤1:160). No apparent associations were observed between antibody development and decreased efficacy or ISR development (data not shown).

### DISCUSSION

VOYAGE 2 confirms the results of VOYAGE 1 demonstrating that guselkumab is highly effective in treating a broad moderate-to-severe psoriasis population. Guselkumab was superior to placebo at the week-16 co-primary endpoints of IGA cleared/minimal and PASI 90 response. Guselkumab was also superior to adalimumab at the week-16 endpoints of IGA cleared/minimal, PASI 75/90, and by week 24, IGA cleared and PASI 90/100. Guselkumab also successfully treated difficult regional psoriasis, including scalp, nails, and hands/feet. Investigator-assessed improvements were mirrored by improvements in the patient-reported outcomes evaluated in VOYAGE 1, (DLQI and PSSD, a newly validated instrument that measures signs and symptoms of psoriasis). In addition, significant improvements in quality of life (QoL) (SF-36) were reported in VOYAGE 2. The combined robust and comprehensive results from VOYAGE 1 and VOYAGE 2 demonstrated superiority to both placebo and adalimumab.

Consistent with findings of other biologics for psoriasis, VOYAGE 2 demonstrated that maintenance is superior to interrupted therapy, and that blockade of IL-23 did not reverse the underlying mechanism of psoriasis. Unlike VOYAGE 1, in which patients continued treatment for 48 weeks, in VOYAGE 2, PASI 90-responders were randomized at week 28 to continue guselkumab or receive placebo. Guselkumab-treated patients maintained response, including PASI 90, PASI 100, and IGA cleared, while psoriasis slowly recurred and QoL was reduced in placebo patients. The median time to loss of PASI 90 response for withdrawal patients was 15.2 weeks, which is relevant because discontinuation rates are significant with psoriasis biologics use through 1 year (often >50%).

Guselkumab was also effective in treating adalimumab nonresponders (did not achieve PASI 90). Patients were classified as responders/ nonresponders at week 28, 5 weeks after the last adalimumab injection. After 20 weeks of guselkumab treatment, 2/3 of the 112 adalimumab nonresponders who switched to guselkumab achieved PASI 90 (relative to baseline). Determining the rate at which patients who have had sub-optimal responses achieve treatment goals could have a significant impact on treatment decisions for those who seek greater clearance, as more effective psoriasis therapies continue to enter the market. While the mechanism of action of the broader efficacy of guselkumab is unknown, in psoriasis, tumor necrosis factor-α (released from CD163+ macrophages/CD11c+ DCs) and IL-17A (released from neutrophils, mast cells, and Th17 cells) are effector cytokines primarily acting on keratinocytes. IL-23 could be seen as the overarching master cytokine for psoriasis because it drives the activation of T cells and neutrophils, and induces IL-17 production. Moreover, IL-23 induces Th17 and other innate cells to produce IL-22, another cytokine implicated in psoriasis pathogenesis. Therefore, targeting the IL-23 pathway with an antibody such as guselkumab can provide higher efficacy and durable responses.

The safety profile of guselkumab in this study was consistent with VOYAGE 1. The rates and types of AEs, SAEs, and laboratory abnormalities were generally comparable to placebo though week 16 and adalimumab through week 28. Through week 48, rates of serious infections, malignancies, and MACE were low across treatment groups. Overall, there were 2 cases of TB, both in adalimumab patients. There were 5 malignancies in the guselkumab-treated patients, 4 of which were NMSCs (2 BCC and 2 SCC). ISRs occurred more frequently in adalimumab patients. While the safety profile of guselkumab is favorable, the size and duration of the study was inadequate to detect rare events.

There are several other limitations of VOYAGE 2. While comparison of guselkumab to adalimumab in VOYAGE 2 was limited to 24 weeks, VOYAGE 1 extended the comparison to adalimumab through 48 weeks. More importantly, at week 48, few VOYAGE 2 patients withdrawn from active therapy had lost adequate response to allow assessment of the efficacy and safety of retreatment. However, these numbers will be augmented at a later timepoint.

In conclusion, VOYAGE 2 confirms the results of VOYAGE 1 that guselkumab demonstrated superior efficacy to adalimumab and comparable safety when administered in a convenient dosage regimen of a single 100-mg injection at weeks 0, 4, and every-8-weeks. These results suggest that guselkumab may be an important addition to psoriasis treatment alternatives. Additionally, VOYAGE 2 provides important data on the need for continuing therapy with guselkumab to maintain the highest level of response, as well as successful transition from adalimumab to guselkumab.

### ABBREVIATIONS AND ACRONYMS

- AE: adverse event
- BCC: basal cell carcinoma
- BMI: body mass index
- BSA: body surface area
- DLQI: Dermatology Life Quality Index
- f-PGA: Fingernail Physician's Global Assessment
- hf-PGA: Physician's Global Assessment of Hands and/or Feet
- HRQoL: health-related quality of life
- IGA: Investigator's Global Assessment
- IL: interleukin
- NAPSI: Nail Psoriasis Area and Severity Index
- NMSC: nonmelanoma skin cancer
- PASI: Psoriasis Area and Severity Index
- PRO: patient-reported outcome
- PSSD: Psoriasis Sign and Symptom Diary
- SAE: serious adverse event
- ss-IGA: Scalp-Specific Investigator's Global Assessment
- TNFα-inhibitor: tumor necrosis factor-α inhibitor

### US Regulatory Approval

The anti-IL-23 specific antibody guselkumab has been approved for marketing in the U.S. by the U.S. Food and Drug Administration for the treatment of adult patients with moderate-to-severe plaque psoriasis who are candidates for systemic therapy or phototherapy by way of a Biologics License Application. The initial approved dosage is 100 mg administered by subcutaneous injection at Week 0, Week 4 and every 8 weeks thereafter. The antibody is at a concentration of 100 mg/mL in a single-dose prefilled syringe.

## Claims

1. An IL-23 antibody for use in a method of treating psoriasis in a patient that is a non-responder to a TNF inhibitor, comprising administering the antibody to the patient in a safe and effective amount, wherein the antibody is guselkumab and the TNF inhibitor is adalimumab.

2. The antibody for use of claim 1, wherein the patient is determined to be a non-responder to the TNF inhibitor by measuring the PASI and/or IGA score.

3. The antibody for use of claim 1 or claim 2, wherein the antibody is administered at a dose of between 25 mg and 200 mg.

4. The antibody for use of claim 3, wherein the antibody is administered at a dose of 50 mg or 100 mg.

5. The antibody for use of any one of claims 1-4, wherein the antibody is administered subcutaneously.

6. The antibody for use of claim 5, wherein the anti-IL-23 antibody is safe and effective for treating psoriasis at an area of a patient selected from the group consisting of scalp, nails, hands and feet.

7. The antibody for use of claim 6, wherein the antibody is in a composition comprising:
(a) 100 mg/mL of antibody; 7.9% (w/v) sucrose, 4.0mM Histidine, 6.9 mM L-Histidine monohydrochloride monohydrate; 0.053% (w/v) Polysorbate 80 of the pharmaceutical composition; wherein the diluent is water at standard state, or
(b) 50 mg/mL of antibody; 7.9% (w/v) sucrose, 4.0mM Histidine, 6.9 mM L-Histidine monohydrochloride monohydrate; 0.053% (w/v) Polysorbate 80 of the pharmaceutical composition; wherein the diluent is water at standard state.

8. The antibody for use of any one of claims 1-7, wherein the antibody is administered in an initial dose, 4 weeks after the initial dose and every 8 weeks after the dose at 4 weeks.

9. The antibody for use of any one of claims 1-8, wherein the patient achieves an IGA score of 0 or 1 and PASI90 or PASI100 at week 16.

10. The antibody for use of claim 9, wherein the PASI90, PASI100 or IGA 0 or 1 score is measured 16, 20 or 28 weeks after initial treatment.

11. The antibody for use of any one of claims 1-10, further comprising administering to the patient one or more additional drugs used to treat psoriasis,
optionally wherein the additional drug is selected from the group consisting of: immunosuppressive agents, non-steroidal anti-inflammatory drugs (NSAIDs), methotrexate (MTX), anti-B-cell surface marker antibodies, anti-CD20 antibodies, rituximab, TNF-inhibitors, corticosteroids, and co-stimulatory modifiers.

12. The antibody for use of any one of claims 1-11, wherein the antibody is effective to reduce a symptom of psoriasis in the patient, induce clinical response, induce or maintain clinical remission, inhibit disease progression, or inhibit a disease complication in the patient.

## Patentansprüche

1. IL-23-Antikörper zur Verwendung in einem Verfahren zur Behandlung von Psoriasis bei einem Patienten, der nicht auf einen TNF-Inhibitor anspricht, umfassend die Verabreichung des Antikörpers an den Patienten in einer sicheren und wirksamen Menge, wobei der Antikörper Guselkumab und der TNF-Inhibitor Adalimumab ist.

2. Antikörper zur Verwendung nach Anspruch 1, wobei durch Messen des PASI- und/oder IGA-Score festgestellt wird, dass der Patient nicht auf den TNF-Inhibitor anspricht.

3. Antikörper zur Verwendung nach Anspruch 1 oder Anspruch 2, wobei der Antikörper in einer Dosis zwischen 25 mg und 200 mg verabreicht wird.

4. Antikörper zur Verwendung nach Anspruch 3, wobei der Antikörper in einer Dosis von 50 mg oder 100 mg verabreicht wird.

5. Antikörper zur Verwendung nach einem der Ansprüche 1 bis 4, wobei der Antikörper subkutan verabreicht wird.

6. Antikörper zur Verwendung nach Anspruch 5, wobei der Anti-IL-23-Antikörper sicher und wirksam zur Behandlung von Psoriasis an einer Stelle eines Patienten ist, die aus der Gruppe ausgewählt ist, die aus Kopfhaut, Nägeln, Händen und Füßen besteht.

7. Antikörper zur Verwendung nach Anspruch 6, wobei der Antikörper in einer Zusammensetzung vorliegt, die Folgendes umfasst:
(a) 100 mg/ml Antikörper; 7,9 % (w/v) Saccharose, 4,0 mM Histidin, 6,9 mM L-Histidinmonohydrochlorid-Monohydrat; 0,053 % (w/v) Polysorbat 80 der pharmazeutischen Zusammensetzung; wobei das Verdünnungsmittel Wasser im Standardzustand ist, oder
(b) 50 mg/ml Antikörper; 7,9 % (w/v) Saccharose, 4,0 mM Histidin, 6,9 mM L-Histidinmonohydrochlorid-Monohydrat; 0,053 % (w/v) Polysorbat 80 der pharmazeutischen Zusammensetzung; wobei das Verdünnungsmittel Wasser im Standardzustand ist.

8. Antikörper zur Verwendung nach einem der Ansprüche 1 bis 7, wobei der Antikörper in einer Anfangsdosis, 4 Wochen nach der Anfangsdosis und alle 8 Wochen nach der Dosis nach 4 Wochen verabreicht wird.

9. Antikörper zur Verwendung nach einem der Ansprüche 1 bis 8, wobei der Patient einen IGA-Score von 0 oder 1 und PASI90 oder PASI100 bei Woche 16 erreicht.

10. Antikörper zur Verwendung nach Anspruch 9, wobei der PASI90-, PASI100- oder IGA-Wert 0 oder 1 16, 20 oder 28 Wochen nach der Erstbehandlung gemessen wird.

11. Antikörper zur Verwendung nach einem der Ansprüche 1 bis 10, der ferner die Verabreichung eines oder mehrerer zusätzlicher Medikamente zur Behandlung von Psoriasis an den Patienten umfasst,
wobei das zusätzliche Arzneimittel optional aus der Gruppe ausgewählt ist, die aus Immunsuppressiva, nichtsteroidalen Antirheumatika (NSAIDs), Methotrexat (MTX), Antikörpern gegen B-Zell-Oberflächenmarker, Anti-CD20-Antikörpern, Rituximab, TNF-Inhibitoren, Kortikosteroiden und co-stimulierenden Modifikatoren besteht.

12. Antikörper zur Verwendung nach einem der Ansprüche 1 bis 11, wobei der Antikörper wirksam ist, um ein Symptom der Psoriasis beim Patienten zu reduzieren, eine klinische Reaktion hervorzurufen, eine klinische Remission herbeizuführen oder aufrechtzuerhalten, das Fortschreiten der Krankheit zu hemmen oder eine Krankheitskomplikation beim Patienten zu hemmen.

## Revendications

1. Anticorps IL-23 destiné à être utilisé dans une méthode de traitement du psoriasis chez un patient qui est non-répondeur à un inhibiteur du TNF, comprenant l'administration de l'anticorps au patient dans une quantité sûre et efficace, dans lequel l'anticorps est le guselkumab et l'inhibiteur du TNF est l'adalimumab.

2. Anticorps destiné à être utilisé selon la revendication 1, dans lequel on détermine que le patient est non-répondeur à l'inhibiteur du TNF en mesurant le score PASI et/ou IGA.

3. Anticorps destiné à être utilisé selon la revendication 1 ou la revendication 2, dans lequel l'anticorps est administré à une dose comprise entre 25 mg et 200 mg.

4. Anticorps destiné à être utilisé selon la revendication 3, dans lequel l'anticorps est administré à une dose de 50 mg ou 100 mg.

5. Anticorps destiné à être utilisé selon l'une quelconque des revendications 1 à 4, dans lequel l'anticorps est administré par voie sous-cutanée.

6. Anticorps destiné à être utilisé selon la revendication 5, dans lequel l'anticorps anti-IL-23 est sûr et efficace pour traiter le psoriasis sur une zone d'un patient choisie dans le groupe constitué du cuir chevelu, des ongles, des mains et des pieds.

7. Anticorps destiné à être utilisé selon la revendication 6, dans lequel l'anticorps est dans une composition comprenant :
(a) 100 mg/ml d'anticorps ; 7,9 % (p/v) de saccharose, 4,0 mM d'histidine, 6,9 mM de monochlorhydrate de L-histidine monohydraté ; 0,053 % (p/v) de polysorbate 80 de la composition pharmaceutique ; dans lequel le diluant est de l'eau à l'état standard, ou
(b) 50 mg/ml d'anticorps ; 7,9 % (p/v) de saccharose, 4,0 mM d'histidine, 6,9 mM de monochlorhydrate de L-histidine monohydraté ; 0,053 % (p/v) de polysorbate 80 de la composition pharmaceutique ; dans lequel le diluant est de l'eau à l'état standard.

8. Anticorps destiné à être utilisé selon l'une quelconque des revendications 1 à 7, dans lequel l'anticorps est administré en dose initiale, 4 semaines après la dose initiale et toutes les 8 semaines après la dose de 4 semaines.

9. Anticorps destiné à être utilisé selon l'une quelconque des revendications 1 à 8, dans lequel le patient obtient un score IGA de 0 ou 1 et un PASI90 ou PASI100 à la semaine 16.

10. Anticorps destiné à être utilisé selon la revendication 9, dans lequel le score PASI90, PASI100 ou IGA 0 ou 1 est mesuré 16, 20 ou 28 semaines après le traitement initial.

11. Anticorps destiné à être utilisé selon l'une des revendications 1 à 10, comprenant en outre l'administration au patient d'un ou de plusieurs médicaments supplémentaires utilisés pour traiter le psoriasis,
éventuellement, dans lequel le médicament supplémentaire est choisi dans le groupe constitué par : les agents immunosuppresseurs, les anti-inflammatoires non stéroïdiens (AINS), le méthotrexate (MTX), les anticorps anti-marqueurs de surface des cellules B, les anticorps anti-CD20, le rituximab, les inhibiteurs du TNF, les corticostéroïdes et les modificateurs de la co-stimulation.

12. Anticorps destiné à être utilisé selon l'une quelconque des revendications 1 à 11, dans lequel l'anticorps est efficace pour réduire un symptôme du psoriasis chez le patient, induire une réponse clinique, induire ou maintenir une rémission clinique, inhiber la progression de la maladie ou inhiber une complication de la maladie chez le patient.


### Sequence Listing

<210> 1
   <211> 5
   <212> PRT
   <213> Homo sapiens
<400> 1
   Asn Tyr Ala Ile Ser
    1               5
<210> 2
   <211> 5
   <212> PRT
   <213> Homo sapiens
<400> 2
   Ser Asn Tyr Ile Ser
   1 5
<210> 3
   <211> 5
   <212> PRT
   <213> Homo sapiens
<400> 3
   Asn Tyr Trp Ile Ser
   1 5
<210> 4
   <211> 5
   <212> PRT
   <213> Homo sapiens
<400> 4
   Ser Tyr Trp Ile Thr
   1 5
<210> 5
   <211> 5
   <212> PRT
   <213> Homo sapiens
<400> 5
   Asn Tyr Trp Ile Gly
   1 5
<210> 6
   <211> 5
   <212> PRT
   <213> Homo sapiens
<400> 6
   Ser Phe Gly Met Ser
   1 5
<210> 7
   <211> 17
   <212> PRT
   <213> Homo sapiens
<400> 7
   Gly Ile Ile Pro Met Phe Gly Tyr Ala Asn Tyr Ala Gln Lys Phe Gln
   1 5 10 15
   Gly
<210> 8
   <211> 17
   <212> PRT
   <213> Homo sapiens
<400> 8
   Gly Ile Ile Pro Val Phe Gly Phe Thr His Tyr Ala Gln Lys Phe Gln
    1               5                  10                  15
   Gly
<210> 9
   <211> 17
   <212> PRT
   <213> Homo sapiens
<400> 9
   Gly Ile Ile Pro Ile Phe Gly His Ala Asn Tyr Ala Gln Lys Phe Gln
    1               5                  10                  15
   Gly
<210> 10
   <211> 17
   <212> PRT
   <213> Homo sapiens
<400> 10
   Ile Ile Ile Pro Pro Ile Gly Asn Ala Trp Tyr Ala Gln Lys Phe Gln
    1               5                  10                  15
   Gly
<210> 11
   <211> 17
   <212> PRT
   <213> Homo sapiens
<400> 11
   Leu Ile Asp Pro Asn Phe Gly Gly Ala Tyr Tyr Ala Gln Lys Phe Gln
    1               5                  10                  15
   Gly
<210> 12
   <211> 17
   <212> PRT
   <213> Homo sapiens
<400> 12
   Leu Ile Asp Pro Val Phe Gly Gly Ala Tyr Tyr Ala Gln Lys Phe Gln
    1               5                  10                  15
   Gly
<210> 13
   <211> 17
   <212> PRT
   <213> Homo sapiens
<400> 13
   Leu Ile Asp Pro Met Phe Gly Gly Ala Tyr Tyr Ala Gln Lys Phe Gln
    1               5                  10                  15
   Gly
<210> 14
   <211> 16
   <212> PRT
   <213> Homo sapiens
<400> 14
   Ile Asn Ala His Leu Gly Gly Thr Trp Tyr Ala Gln Lys Phe Gln Gly
    1               5                  10                  15
<210> 15
   <211> 17
   <212> PRT
   <213> Homo sapiens
<400> 15
   Ile Ser Pro Gly Thr Gly Ile Asn Ala Tyr Tyr Ala Gln Lys Phe Gln
    1               5                  10                  15
   Gly
<210> 16
   <211> 17
   <212> PRT
   <213> Artificial Sequence
<220>
   <223> Synthesized human sequence
<220>
   <221> unsure
   <222> (1)
   <223> Where Xaa can be G, I, or L
<220>
   <221> unsure
   <222> (2)
   <223> Where Xaa can be I or S
<220>
   <221> unsure
   <222> (3)
   <223> Where Xaa can be I, P, N, or D
<220>
   <221> unsure
   <222> (4)
   <223> Where Xaa can be P, G, or A
<220>
   <221> unsure
   <222> (5)
   <223> Where Xaa can be I, M, P,
<223> T, H, N, or V
<220>
   <221> unsure
   <222> (6)
   <223> Where Xaa can be F, I, G, or L
<220>
   <221> unsure
   <222> (7)
   <223> Where Xaa can G or I
<220>
   <221> unsure
   <222> (8)
   <223> Where Xaa can be H, Y, N, or G
<220>
   <221> unsure
   <222> (9)
   <223> Where Xaa can be A or T
<220>
   <221> unsure
   <222> (10)
   <223> Where Xaa can be N, W, or Y
<400> 16
   Xaa Xaa Xaa Xaa Xaa Xaa Xaa Xaa Xaa Xaa Tyr Ala Gln Lys Phe Gln
      1               5                  10                  15
   Gly
<210> 17
   <211> 17
   <212> PRT
   <213> Homo sapiens
<400> 17
   Trp Ile Arg Pro Gly Asp Ser Asp Thr Arg Tyr Ser Pro Ser Phe Glu
    1               5                  10                  15
   Gly
<210> 18
   <211> 19
   <212> PRT
   <213> Homo sapiens
<400> 18
   Val Ser Tyr Ile Ser Ser Ser Gly Ser Ser Thr Tyr Tyr Ala Asp Ser
    1               5                   10                  15
   Val Lys Gly
<210> 19
   <211> 17
   <212> PRT
   <213> Homo sapiens
<400> 19
   Ile Ile Asp Pro Ser Asn Ser Tyr Thr Asn Tyr Ser Pro Ser Phe Gln
    1               5                   10                  15
   Gly
<210> 20
   <211> 17
   <212> PRT
   <213> Homo sapiens
<400> 20
   Ile Ile Asp Pro Ser Asn Ser Tyr Thr Arg Tyr Ser Pro Ser Phe Gln
    1               5                   10                  15
   Gly
<210> 21
   <211> 17
   <212> PRT
   <213> Homo sapiens
<400> 21
   Ile Ile Asp Pro Ser Asn Ser Tyr Thr Asp Tyr Ser Pro Ser Phe Gln
    1               5                   10                  15
   Gly
<210> 22
   <211> 17
   <212> PRT
   <213> Homo sapiens
<400> 22
   Ile Ile Ser Pro Thr Gly Ser Val Thr Trp Tyr Ser Pro Ser Phe Gln
    1               5                   10                  15
   Gly
<210> 23
   <211> 17
   <212> PRT
   <213> Homo sapiens
<400> 23
   Ile Ile Ser Pro Thr Gly Ser Ser Thr Trp Tyr Ser Pro Ser Phe Gln
    1              5                   10                  15
   Gly
<210> 24
   <211> 17
   <212> PRT
   <213> Homo sapiens
<400> 24
   Phe Ile Ser Pro Asp Gly Ser His Thr Trp Tyr Ser Pro Ser Phe Gln
    1              5                   10                  15
   Gly
<210> 25
   <211> 17
   <212> PRT
   <213> Homo sapiens
<400> 25
   Ile Ile Ser Pro Ser Gly Ser Thr Thr Trp Tyr Ser Pro Ser Phe Gln
    1              5                   10                  15
   Gly
<210> 26
   <211> 17
   <212> PRT
   <213> Homo sapiens
<400> 26
   Ile Ile Ser Pro Thr Gly Ser Ala Thr Trp Tyr Ser Pro Ser Phe Gln
    1              5                   10                  15
   Gly
<210> 27
   <211> 17
   <212> PRT
   <213> Homo sapiens
<400> 27
   Ile Ile Asp Pro Val Ser Ser Trp Thr Lys Tyr Ser Pro Ser Phe Gln
    1              5                   10                  15
   Gly
<210> 28
   <211> 17
   <212> PRT
   <213> Artificial Sequence
<220>
   <223> Synthesized human sequence
<220>
   <221> unsure
   <222> (3)
   <223> Where Xaa can be D or S
<220>
   <221> unsure
   <222> (5)
   <223> Where Xaa can be S, V, D, or T
<220>
   <221> unsure
   <222> (6)
   <223> Where Xaa can be N, S, or G
<220>
   <221> unsure
   <222> (8)
   <223> Where Xaa can be Y, W, T, H, V, S, or A
<220>
   <221> unsure
   <222> (10)
   <223> Where Xaa can be N, D, R, K, or W
<400> 28
   Ile Ile Xaa Pro Xaa Xaa Ser Xaa Thr Xaa Tyr Ser Pro Ser Phe Gln
    1               5                   10                  15
   Gly
<210> 29
   <211> 17
   <212> PRT
   <213> Homo sapiens
<400> 29
   Asn Ile Ser Ser Ser Gly Ser Ser Thr Tyr Tyr Ala Asp Ser Val Lys
    1               5                   10                  15
   Gly
<210> 30
   <211> 19
   <212> PRT
   <213> Homo sapiens
<400> 30
   Asn Ile Glu His Lys Tyr Leu Asn Tyr Ala Thr Tyr Tyr Ala Ala Ser
    1               5                   10                  15
   Val Lys Gly
<210> 31
   <211> 19
   <212> PRT
   <213> Homo sapiens
<400> 31
   Asn Ile Glu His Lys Phe Met Gly Tyr Thr Thr Tyr Tyr Ala Ala Gly
    1               5                  10                  15
   Val Lys Gly
<210> 32
   <211> 19
   <212> PRT
   <213> Homo sapiens
<400> 32
   Gly Ile Glu His Lys Tyr Leu Ser Tyr Thr Thr His Tyr Ala Ala Ser
    1               5                  10                  15
   Val Lys Gly
<210> 33
   <211> 19
   <212> PRT
   <213> Homo sapiens
<400> 33
   Ser Ile Glu His Lys Tyr Thr Gly Tyr Thr Thr Tyr Tyr Ala Ala Pro
    1               5                  10                  15
   Val Lys Gly
<210> 34
   <211> 19
   <212> PRT
   <213> Homo sapiens
<400> 34
   Gln Ile Glu His Lys Tyr Leu Ser Tyr Thr Thr Leu Tyr Ala Ala Ser
    1               5                  10                  15
   Val Lys Gly
<210> 35
   <211> 19
   <212> PRT
   <213> Homo sapiens
<400> 35
   Ser Ile Glu His Lys Tyr Leu Ser Tyr Thr Thr Phe Tyr Ala Ala Ser
    1               5                  10                  15
   Val Lys Gly
<210> 36
   <211> 19
   <212> PRT
   <213> Homo sapiens
<400> 36
   Asn Ile Glu Gly Lys Tyr Thr Ser Tyr Thr Thr Tyr Tyr Ala Ala Ser
    1              5                   10                  15
   Val Lys Gly
<210> 37
   <211> 19
   <212> PRT
   <213> Homo sapiens
<400> 37
   Gly Ile Glu His Lys Tyr Leu Ser Tyr Ala Thr Leu Tyr Ala Ala Ser
    1              5                   10                  15
   Val Lys Gly
<210> 38
   <211> 19
   <212> PRT
   <213> Homo sapiens
<400> 38
   Asn Ile Glu His Lys Tyr Leu Gly Tyr Ala Thr Val Tyr Ala Ala Ser
    1              5                   10                  15
   Val Lys Gly
<210> 39
   <211> 19
   <212> PRT
   <213> Homo sapiens
<400> 39
   Ser Ile Glu His Lys Tyr Leu Ser Tyr Ala Thr Tyr Tyr Ala Ala Gly
    1              5                   10                  15
   Val Lys Gly
<210> 40
   <211> 8
   <212> PRT
   <213> Homo sapiens
<400> 40
   Asp Ile Tyr Ala Gly Met Asp Val
    1              5
<210> 41
   <211> 18
   <212> PRT
   <213> Homo sapiens
<400> 41
   Ser Lys Lys Gly Met Tyr Gly Gly Trp Thr Tyr Pro Leu Met Met Phe
    1              5                   10                  15
   Asp Leu
<210> 42
   <211> 7
   <212> PRT
   <213> Homo sapiens
<400> 42
   His Tyr Tyr Gly Met Asp Tyr
    1               5
<210> 43
   <211> 12
   <212> PRT
   <213> Homo sapiens
<400> 43
   Gly Thr Phe Trp Ser Phe Gly Asn Tyr Phe Ala Asn
    1               5                   10
<210> 44
   <211> 8
   <212> PRT
   <213> Homo sapiens
<400> 44
   Trp Tyr Tyr Lys Pro Phe Asp Val
    1               5
<210> 45
   <211> 12
   <212> PRT
   <213> Homo sapiens
<400> 45
   Tyr Trp Gly Thr Pro Tyr Leu Met Gln Phe Asp Asn
    1               5                   10
<210> 46
   <211> 12
   <212> PRT
   <213> Homo sapiens
<400> 46
   Arg Ala Ser Gln Ser Val Leu Gly Asn Tyr Leu Ala
    1               5                   10
<210> 47
   <211> 12
   <212> PRT
   <213> Homo sapiens
<400> 47
   Arg Ala Ser Gln Ser Val Ser Ser Asn Tyr Leu Ala
   1               5                   10
<210> 48
   <211> 13
   <212> PRT
   <213> Homo sapiens
<400> 48
   Ser Gly Ser Ser Ser Asn Ile Gly Ser Tyr Tyr Val Asn
   1               5                   10
<210> 49
   <211> 11
   <212> PRT
   <213> Homo sapiens
<400> 49
   Arg Ala Ser Gln Ser Ile Phe Tyr Asn Leu Ala
   1               5                   10
<210> 50
   <211> 14
   <212> PRT
   <213> Homo sapiens
<400> 50
   Thr Gly Ser Ser Ser Asn Ile Gly Ser Gly Tyr Asp Val His
   1               5                   10
<210> 51
   <211> 14
   <212> PRT
   <213> Homo sapiens
<400> 51
   Thr Gly Thr Ser Ser Asp Val Gly Gly Tyr Asn Ser Val Ser
   1               5                   10
<210> 52
   <211> 7
   <212> PRT
   <213> Homo sapiens
<400> 52
   Gly Ala Ser Ser Arg Ala Thr
   1               5
<210> 53
   <211> 7
   <212> PRT
   <213> Homo sapiens
<400> 53
   Tyr Ala Ser Arg Arg Ala Thr
   1 5
<210> 54
   <211> 7
   <212> PRT
   <213> Homo sapiens
<400> 54
   Gly Asn Thr His Arg Pro Ser
   1 5
<210> 55
   <211> 7
   <212> PRT
   <213> Homo sapiens
<400> 55
   Gly Ala Ser Asn Arg Ala Thr
   1 5
<210> 56
   <211> 7
   <212> PRT
   <213> Homo sapiens
<400> 56
   Gly Asn Ser Lys Arg Pro Ser
   1 5
<210> 57
   <211> 7
   <212> PRT
   <213> Homo sapiens
<400> 57
   Ser Val Ser Ser Arg Pro Ser
   1 5
<210> 58
   <211> 9
   <212> PRT
   <213> Homo sapiens
<400> 58
   His Gln Tyr Gly Ser Ile Ser Thr Thr
   1 5
<210> 59
   <211> 9
   <212> PRT
   <213> Homo sapiens
<400> 59
   Gln Gln Tyr Ser His Leu Leu Ile Thr
   1 5
<210> 60
   <211> 9
   <212> PRT
   <213> Homo sapiens
<400> 60
   Gln Gln Tyr Ser His Ile Ser Leu Thr
   1 5
<210> 61
   <211> 9
   <212> PRT
   <213> Homo sapiens
<400> 61
   Gln Gln Phe Ala His Ile Leu Leu Thr
   1 5
<210> 62
   <211> 9
   <212> PRT
   <213> Homo sapiens
<400> 62
   Gln Gln Thr Ser Asn Thr Pro Phe Thr
   1 5
<210> 63
   <211> 9
   <212> PRT
   <213> Homo sapiens
<400> 63
   Gln Gln Phe Ile Thr Tyr Leu Pro Thr
   1 5
<210> 64
   <211> 9
   <212> PRT
   <213> Homo sapiens
<400> 64
   Gln Gln Asp Ala Leu Ser Pro Phe Thr
   1 5
<210> 65
   <211> 9
   <212> PRT
   <213> Homo sapiens
<400> 65
   Gln Gln Asp Arg Gly Thr Pro Phe Thr
   1 5
<210> 66
   <211> 9
   <212> PRT
   <213> Homo sapiens
<400> 66
   Gln Gln Ser Leu Asn Ile Pro Phe Thr
   1 5
<210> 67
   <211> 9
   <212> PRT
   <213> Homo sapiens
<400> 67
   Gln Gln Asp Thr Ser Ser Pro Phe Thr
   1 5
<210> 68
   <211> 10
   <212> PRT
   <213> Artificial Sequence
<220>
   <223> Synthesized human sequence
<220>
   <221> unsure
   <222> (3)
   <223> Where Xaa can be T, F, D, or S
<220>
   <221> unsure
   <222> (4)
   <223> Where Xaa can be S, I, A, T, R, or L
<220>
   <221> unsure
   <222> (5)
   <223> Where Xaa can be N, T, L, S, or G
<220>
   <221> unsure
   <222> (6)
   <223> Where Xaa can be T, Y, S, or I
<220>
   <221> unsure
   <222> (7)
   <223> Where Xaa can be P or L
<220>
   <221> unsure
   <222> (8)
   <223> Where Xaa can be F or P
<400> 68
   Gln Gln Xaa Xaa Xaa Xaa Xaa Xaa Phe Thr
    1               5                   10
<210> 69
   <211> 11
   <212> PRT
   <213> Homo sapiens
<400> 69
   Gln Thr Tyr Ala Ser Leu Gly Pro Gly Glu Val
    1               5                   10
<210> 70
   <211> 9
   <212> PRT
   <213> Homo sapiens
<400> 70
   Gln Gln Tyr Ser Ser Glu Pro Val Thr
    1               5
<210> 71
   <211> 9
   <212> PRT
   <213> Homo sapiens
<400> 71
   Ser Ser Trp Thr Pro Ser Ser Val Val
    1               5
<210> 72
   <211> 11
   <212> PRT
   <213> Homo sapiens
<400> 72
   Ser Ser Trp Thr Asp Thr Pro Asn Met Ile Val
   1 5 10
<210> 73
   <211> 11
   <212> PRT
   <213> Homo sapiens
<400> 73
   Ala Ser Trp Thr Asp Gly Leu Ser Leu Val Val
   1 5 10
<210> 74
   <211> 11
   <212> PRT
   <213> Artificial Sequence
<220>
   <223> Synthesized human sequence
<220>
   <221> unsure
   <222> (1)
   <223> Where Xaa can be S or A
<220>
   <221> unsure
   <222> (6)
   <223> Where Xaa can be T or G
<220>
   <221> unsure
   <222> (7)
   <223> Where Xaa can be P or L
<220>
   <221> unsure
   <222> (8)
   <223> Where Xaa can be S or N
<220>
   <221> unsure
   <222> (9)
   <223> Where Xaa can be S, M, or L
<220>
   <221> unsure
   <222> (10)
   <223> Where Xaa can be I or V
<400> 74
   Xaa Ser Trp Thr Asp Xaa Xaa Xaa Xaa Xaa Val
   1 5 10
<210> 75
   <211> 11
   <212> PRT
   <213> Homo sapiens
<400> 75
   Ser Ser Tyr Asp Thr Asn Lys Pro Leu Val Val
   1 5 10
<210> 76
   <211> 11
   <212> PRT
   <213> Homo sapiens
<400> 76
   Gly Ser Tyr Asp Val Tyr Gly Arg Phe Tyr Val
   1 5 10
<210> 77
   <211> 11
   <212> PRT
   <213> Homo sapiens
<400> 77
   Ser Ser Tyr Tyr Phe Tyr Leu Gln Arg Ile Val
   1 5 10
<210> 78
   <211> 11
   <212> PRT
   <213> Homo sapiens
<400> 78
   Gln Thr Tyr Tyr Phe Ser Tyr Ser Gly Pro Val
   1 5 10
<210> 79
   <211> 11
   <212> PRT
   <213> Homo sapiens
<400> 79
   Gly Ser Trp Asp Pro Ile Phe Ser Tyr Glu Val
   1 5 10
<210> 80
   <211> 117
   <212> PRT
   <213> Homo sapiens
<400> 80
   Gln Val Gln Leu Val Gln Ser Gly Ala Glu Val Lys Lys Pro Gly Ser
    1               5                  10                  15
   Ser Val Lys Val Ser Cys Lys Ala Ser Gly Gly Thr Phe Ser Asn Tyr
               20                  25                  30
   Ala Ile Ser Trp Val Arg Gln Ala Pro Gly Gln Gly Leu Glu Trp Met
           35                  40                  45
   Gly Gly Ile Ile Pro Met Phe Gly Tyr Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Ile Tyr Ala Gly Met Asp Val Trp Gly Gln Gly Thr Leu
               100                 105                 110
   Val Thr Val Ser Ser
           115
<210> 81
   <211> 117
   <212> PRT
   <213> Homo sapiens
<400> 81
   Gln Val Gln Leu Val Gln Ser Gly Ala Glu Val Lys Lys Pro Gly Ser
    1               5                  10                  15
   Ser Val Lys Val Ser Cys Lys Ala Ser Gly Gly Thr Phe Ser Asn Tyr
               20                  25                  30
   Ala Ile Ser Trp Val Arg Gln Ala Pro Gly Gln Gly Leu Glu Trp Met
           35                  40                  45
   Gly Gly Ile Ile Pro Val Phe Gly Phe Thr His Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Asp Ile Tyr Ala Gly Met Asp Val Trp Gly Gln Gly Thr Leu
               100                 105                 110
   Val Thr Val Ser Ser
           115
<210> 82
   <211> 108
   <212> PRT
   <213> Homo sapiens
<400> 82
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Leu Gly Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys His Gln Tyr Gly Ser Ile Ser
                   85                  90                  95
   Thr Thr Phe Gly Gln Gly Thr Lys Val Glu Ile Lys
               100                 105
<210> 83
   <211> 108
   <212> PRT
   <213> Homo sapiens
<400> 83
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Leu Gly Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Tyr Ser His Ile Ser
                   85                  90                  95
   Leu Thr Phe Gly Gln Gly Thr Lys Val Glu Ile Lys
               100                 105
<210> 84
   <211> 108
   <212> PRT
   <213> Homo sapiens
<400> 84
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Leu Gly Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Tyr Ser His Leu Ile
                   85                  90                  95
   Ile Thr Phe Gly Gln Gly Thr Lys Val Glu Ile Lys
               100                 105
<210> 85
   <211> 108
   <212> PRT
   <213> Homo sapiens
<400> 85
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Leu Gly Asn
               20                  25                  30
   Tyr Leu Ala Trp Tyr Gln Gln Lys Pro Gly Gln Ala Pro Arg Leu Leu
           35                  40                  45
   Ile Tyr Gly Ala Ser Ser Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Phe Ala His Ile Leu
                   85                  90                  95
   Leu Thr Phe Gly Gln Gly Thr Lys Val Glu Ile Lys
               100                 105
<210> 86
   <211> 127
   <212> PRT
   <213> Homo sapiens
<400> 86
   Gln Val Gln Leu Val Gln Ser Gly Ala Glu Val Lys Lys Pro Gly Ser
    1               5                  10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Gly Ile Ile Pro Ile Phe Gly His Ala Asn Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Lys Lys Gly Met Tyr Gly Gly Trp Thr Tyr Pro Leu Met
               100                 105                 110
   Met Phe Asp Leu Trp Gly Gln Gly Thr Leu Val Thr Val Ser Ser
           115                 120                 125
<210> 87
   <211> 127
   <212> PRT
   <213> Homo sapiens
<400> 87
   Gln Val Gln Leu Val Gln Ser Gly Ala Glu Val Lys Lys Pro Gly Ser
    1               5                   10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Ile Ile Ile Pro Pro Ile Gly Asn Ala Trp Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Lys Lys Gly Met Tyr Gly Gly Trp Thr Tyr Pro Leu Met
               100                 105                 110
   Met Phe Asp Leu Trp Gly Gln Gly Thr Leu Val Thr Val Ser Ser
           115                 120                 125
<210> 88
   <211> 127
   <212> PRT
   <213> Homo sapiens
<400> 88
   Gln Val Gln Leu Val Gln Ser Gly Ala Glu Val Lys Lys Pro Gly Ser
    1               5                   10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Ile Ser Pro Gly Thr Gly Ile Asn Ala Tyr Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Lys Lys Gly Met Tyr Gly Gly Trp Thr Tyr Pro Leu Met
               100                 105                 110
   Met Phe Asp Leu Trp Gly Gln Gly Thr Leu Val Thr Val Ser Ser
           115                 120                 125
<210> 89
   <211> 126
   <212> PRT
   <213> Homo sapiens
<400> 89
   Gln Val Gln Leu Val Gln Ser Gly Ala Glu Val Lys Lys Pro Gly Ser
    1               5                   10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Ile Asn Ala His Leu Gly Gly Thr Trp Tyr Ala Gln Lys Phe Gln
       50                  55                  60
   Gly Arg Val Thr Ile Thr Ala Asp Glu Ser Thr Ser Thr Ala Tyr Met
   65                  70                  75                  80
   Glu Leu Ser Ser Leu Arg Ser Glu Asp Thr Ala Val Tyr Tyr Cys Ala
                   85                  90                  95
   Arg Ser Lys Lys Gly Met Tyr Gly Gly Trp Thr Tyr Pro Leu Met Met
               100                 105                 110
   Phe Asp Leu Trp Gly Gln Gly Thr Leu Val Thr Val Ser Ser
           115                 120                 125
<210> 90
   <211> 127
   <212> PRT
   <213> Homo sapiens
<400> 90
   Gln Val Gln Leu Val Gln Ser Gly Ala Glu Val Lys Lys Pro Gly Ser
    1               5                   10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Leu Ile Asp Pro Asn Phe Gly Gly Ala Tyr Tyr Ala Gln Lys Phe
       50                  55                  60
   Gln Gly Arg Val Thr Ile Thr Ala Asp Glu Ser Thr Ser Thr Ala Tyr
   65                  70                  75                  80
   Met Glu Leu Ser Ser Leu Arg Ser Glu Asp Thr Ala Val Tyr Tyr Cys
                   85                  90                  95
   Ala Arg Ser Lys Lys Gly Met Tyr Gly Gly Trp Thr Tyr Pro Leu Met
               100                 105                 110
   Met Phe Asp Leu Trp Gly Gln Gly Thr Leu Val Thr Val Ser Ser
           115                 120                 125
<210> 91
   <211> 127
   <212> PRT
   <213> Homo sapiens
<400> 91
   Gln Val Gln Leu Val Gln Ser Gly Ala Glu Val Lys Lys Pro Gly Ser
    1               5                   10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Leu Ile Asp Pro Val Phe Gly Gly Ala Tyr Tyr Ala Gln Lys Phe
       50                  55                  60
   Gln Gly Arg Val Thr Ile Thr Ala Asp Glu Ser Thr Ser Thr Ala Tyr
   65                  70                  75                      80
   Met Glu Leu Ser Ser Leu Arg Ser Glu Asp Thr Ala Val Tyr Tyr Cys
                   85                  90                  95
   Ala Arg Ser Lys Lys Gly Met Tyr Gly Gly Trp Thr Tyr Pro Leu Met
               100                 105                 110
   Met Phe Asp Leu Trp Gly Gln Gly Thr Leu Val Thr Val Ser Ser
           115                 120                 125
<210> 92
   <211> 127
   <212> PRT
   <213> Homo sapiens
<400> 92
   Gln Val Gln Leu Val Gln Ser Gly Ala Glu Val Lys Lys Pro Gly Ser
    1               5                   10                  15
   Ser Val Lys Val Ser Cys Lys Ala Ser Gly Gly Thr Phe Ser Ser Asn
               20                  25                  30
   Tyr Ile Ser Trp Val Arg Gln Ala Pro Gly Gln Gly Leu Glu Trp Met
           35                  40                  45
   Gly Leu Ile Asp Pro Met Phe Gly Gly Ala Tyr Tyr Ala Gln Lys Phe
       50                  55                  60
   Gln Gly Arg Val Thr Ile Thr Ala Asp Glu Ser Thr Ser Thr Ala Tyr
   65                  70                  75                      80
   Met Glu Leu Ser Ser Leu Arg Ser Glu Asp Thr Ala Val Tyr Tyr Cys
                   85                  90                  95
   Ala Arg Ser Lys Lys Gly Met Tyr Gly Gly Trp Thr Tyr Pro Leu Met
               100                 105                 110
   Met Phe Asp Leu Trp Gly Gln Gly Thr Leu Val Thr Val Ser Ser
           115                 120                 125
<210> 93
   <211> 108
   <212> PRT
   <213> Homo sapiens
<400> 93
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Tyr Ala Ser Arg Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Thr Tyr Tyr Cys Gln Gln Thr Ser Asn Thr Pro
                   85                  90                  95
   Phe Thr Phe Gly Gln Gly Thr Lys Val Glu Ile Lys
               100                 105
<210> 94
   <211> 108
   <212> PRT
   <213> Homo sapiens
<400> 94
   Glu Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Tyr Ala Ser Arg Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Thr Ser Asn Thr Pro
                   85                  90                  95
   Phe Thr Phe Gly Gln Gly Thr Lys Val Glu Ile Lys
               100                 105
<210> 95
   <211> 108
   <212> PRT
   <213> Homo sapiens
<400> 95
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Tyr Ala Ser Arg Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Phe Ile Thr Tyr Leu
                   85                  90                  95
   Pro Thr Phe Gly Gln Gly Thr Lys Val Glu Ile Lys
               100                 105
<210> 96
   <211> 108
   <212> PRT
   <213> Homo sapiens
<400> 96
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Tyr Ala Ser Arg Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Asp Ala Leu Ser Pro
                   85                  90                  95
   Phe Thr Phe Gly Gln Gly Thr Lys Val Glu Ile Lys
               100                 105
<210> 97
   <211> 108
   <212> PRT
   <213> Homo sapiens
<400> 97
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Tyr Ala Ser Arg Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Asp Arg Gly Thr Pro
                   85                  90                  95
   Phe Thr Phe Gly Gln Gly Thr Lys Val Glu Ile Lys
               100                 105
<210> 98
   <211> 108
   <212> PRT
   <213> Homo sapiens
<400> 98
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Val Ser Ser Asn
               20                  25                  30
   Tyr Leu Ala Trp Tyr Gln Gln Lys Pro Gly Gln Ala Pro Arg Leu Leu
           35                  40                  45
   Ile Tyr Tyr Ala Ser Arg Arg Ala Thr Gly Val Pro Ala Arg Phe Ser
       50                  55                  60
   Gly Ser Gly Ser Gly Thr Asp Phe Thr Leu Thr Ile Ser Ser Leu Glu
   65                  70                  75                  80
   Pro Glu Asp Phe Ala Val Tyr Tyr Cys Gln Gln Ser Leu Asn Ile Pro
                   85                  90                  95
   Phe Thr Phe Gly Gln Gly Thr Lys Val Glu Ile Lys
               100                 105
<210> 99
   <211> 116
   <212> PRT
   <213> Homo sapiens
<400> 99
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Thr Asn Tyr
               20                  25                  30
   Trp Ile Ser Trp Val Arg Gln Ala Pro Gly Lys Gly Leu Glu Trp Met
           35                  40                  45
   Gly Trp Ile Arg Pro Gly Asp Ser Asp Thr Arg Tyr Ser Pro Ser Phe
       50                  55                  60
   Glu Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg His Tyr Tyr Gly Met Asp Tyr Trp Gly Gln Gly Thr Leu Val
               100                 105                 110
   Thr Val Ser Ser
           115
<210> 100
   <211> 110
   <212> PRT
   <213> Homo sapiens
<400> 100
   Asp Ile Val Leu Thr Gln Pro Pro Ser Val Ser Gly Ala Pro Gly Gln
    1               5                  10                  15
   Arg Val Thr Ile Ser Cys Ser Gly Ser Ser Ser Asn Ile Gly Ser Tyr
               20                  25                  30
   Tyr Val Asn Trp Tyr Gln Gln Leu Pro Gly Thr Ala Pro Lys Leu Leu
           35                  40                  45
   Ile Tyr Gly Asn Thr His Arg Pro Ser Gly Val Pro Asp Arg Phe Ser
       50                  55                  60
   Gly Ser Lys Ser Gly Thr Ser Ala Ser Leu Ala Ile Thr Gly Leu Gln
   65                  70                  75                  80
   Ser Glu Asp Glu Ala Asp Tyr Tyr Cys Gln Thr Tyr Ala Ser Leu Gly
                   85                  90                  95
   Pro Gly Glu Val Phe Gly Gly Gly Thr Lys Leu Thr Val Leu
               100                 105                 110
<210> 101
   <211> 121
   <212> PRT
   <213> Homo sapiens
<400> 101
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Tyr
               20                  25                  30
   Trp Ile Thr Trp Val Arg Gln Ala Pro Gly Lys Gly Leu Glu Trp Val
           35                  40                  45
   Ser Tyr Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Gly Thr Phe Trp Ser Phe Gly Asn Tyr Phe Ala Asn Trp Gly
               100                 105                 110
   Gln Gly Thr Leu Val Thr Val Ser Ser
           115                 120
<210> 102
   <211> 107
   <212> PRT
   <213> Homo sapiens
<400> 102
   Asp Ile Val Leu Thr Gln Ser Pro Ala Thr Leu Ser Leu Ser Pro Gly
    1               5                  10                  15
   Glu Arg Ala Thr Leu Ser Cys Arg Ala Ser Gln Ser Ile Phe Tyr Asn
               20                  25                  30
   Leu Ala Trp Tyr Gln Gln Lys Pro Gly Gln Ala Pro Arg Leu Leu Ile
           35                  40                  45
   Tyr Gly Ala Ser Asn Arg Ala Thr Gly Val Pro Ala Arg Phe Ser Gly
       50                  55                  60
   Ser Gly Ser Gly Thr Asp Phe Thr Leu Thr Ile Ser Ser Leu Glu Pro
   65                  70                  75                  80
   Glu Asp Phe Ala Thr Tyr Tyr Cys Gln Gln Tyr Ser Ser Glu Pro Val
                   85                  90                  95
   Thr Phe Gly Gln Gly Thr Lys Val Glu Ile Lys
               100                 105
<210> 103
   <211> 117
   <212> PRT
   <213> Homo sapiens
<400> 103
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Asp Pro Ser Asn Ser Tyr Thr Asn Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 104
   <211> 117
   <212> PRT
   <213> Homo sapiens
<400> 104
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Asp Pro Ser Asn Ser Tyr Thr Asp Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 105
   <211> 117
   <212> PRT
   <213> Homo sapiens
<400> 105
   Gln Val Gln Leu Val Gln Ser Gly Ala Glu Val Lys Lys Pro Gly Glu
    1               5                   10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Asp Pro Ser Asn Ser Tyr Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 106
   <211> 117
   <212> PRT
   <213> Homo sapiens
<400> 106
   Glu Val Gln Leu Val Gln Ser Gly Ala Glu Val Lys Lys Pro Gly Glu
    1               5                   10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Asp Pro Ser Asn Ser Tyr Thr Arg Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 107
   <211> 117
   <212> PRT
   <213> Homo sapiens
<400> 107
   Gln Val Gln Leu Val Gln Ser Gly Ala Glu Val Lys Lys Pro Gly Glu
    1               5                   10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Asp Pro Val Ser Ser Trp Thr Lys Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 108
   <211> 117
   <212> PRT
   <213> Homo sapiens
<400> 108
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Ser Pro Ser Gly Ser Thr Thr Trp Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 109
   <211> 117
   <212> PRT
   <213> Homo sapiens
<400> 109
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Phe Ile Ser Pro Asp Gly Ser His Thr Trp Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 110
   <211> 117
   <212> PRT
   <213> Homo sapiens
<400> 110
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Ser Pro Thr Gly Ser Val Thr Trp Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 111
   <211> 117
   <212> PRT
   <213> Homo sapiens
<400> 111
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Ser Pro Thr Gly Ser Ser Thr Trp Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 112
   <211> 117
   <212> PRT
   <213> Homo sapiens
<400> 112
   Gln Val Gln Leu Val Gln Ser Gly Ala Glu Val Lys Lys Pro Gly Glu
    1               5                  10                  15
   Ser Leu Lys Ile Ser Cys Lys Gly Ser Gly Tyr Ser Phe Ser Asn Tyr
               20                  25                  30
   Trp Ile Gly Trp Val Arg Gln Met Pro Gly Lys Gly Leu Glu Trp Met
           35                  40                  45
   Gly Ile Ile Ser Pro Thr Gly Ser Ala Thr Trp Tyr Ser Pro Ser Phe
       50                  55                  60
   Gln Gly Gln Val Thr Ile Ser Ala Asp Lys Ser Ile Ser Thr Ala Tyr
   65                  70                  75                  80
   Leu Gln Trp Ser Ser Leu Lys Ala Ser Asp Thr Ala Met Tyr Tyr Cys
                   85                  90                  95
   Ala Arg Trp Tyr Tyr Lys Pro Phe Asp Val Trp Gly Gln Gly Thr Leu
               100                 105                 110
   Val Thr Val Ser Ser
           115
<210> 113
   <211> 109
   <212> PRT
   <213> Homo sapiens
<400> 113
   Asp Ile Val Leu Thr Gln Pro Pro Ser Val Ser Gly Ala Pro Gly Gln
    1               5                  10                  15
   Arg Val Thr Ile Ser Cys Thr Gly Ser Ser Ser Asn Ile Gly Ser Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Lys Arg Pro Ser Gly Val Pro Asp Arg Phe
       50                  55                  60
   Ser Gly Ser Lys Ser Gly Thr Ser Ala Ser Leu Ala Ile Thr Gly Leu
   65                  70                  75                  80
   Gln Ser Glu Asp Glu Ala Asp Tyr Tyr Cys Ser Ser Trp Thr Pro Ser
                   85                  90                  95
   Ser Val Val Phe Gly Gly Gly Thr Lys Leu Thr Val Leu
               100                 105
<210> 114
   <211> 111
   <212> PRT
   <213> Homo sapiens
<400> 114
   Asp Ile Val Leu Thr Gln Pro Pro Ser Val Ser Gly Ala Pro Gly Gln
    1               5                  10                  15
   Arg Val Thr Ile Ser Cys Thr Gly Ser Ser Ser Asn Ile Gly Ser Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Lys Arg Pro Ser Gly Val Pro Asp Arg Phe
       50                  55                  60
   Ser Gly Ser Lys Ser Gly Thr Ser Ala Ser Leu Ala Ile Thr Gly Leu
   65                  70                  75                  80
   Gln Ser Glu Asp Glu Ala Asp Tyr Tyr Cys Ser Ser Trp Thr Asp Thr
                   85                  90                  95
   Pro Asn Met Ile Val Phe Gly Gly Gly Thr Lys Leu Thr Val Leu
               100                 105                 110
<210> 115
   <211> 111
   <212> PRT
   <213> Homo sapiens
<400> 115
   Asp Ile Val Leu Thr Gln Pro Pro Ser Val Ser Gly Ala Pro Gly Gln
    1               5                  10                  15
   Arg Val Thr Ile Ser Cys Thr Gly Ser Ser Ser Asn Ile Gly Ser Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Lys Arg Pro Ser Gly Val Pro Asp Arg Phe
       50                  55                  60
   Ser Gly Ser Lys Ser Gly Thr Ser Ala Ser Leu Ala Ile Thr Gly Leu
   65                  70                  75                  80
   Gln Ser Glu Asp Glu Ala Asp Tyr Tyr Cys Ala Ser Trp Thr Asp Gly
                   85                  90                  95
   Leu Ser Leu Val Val Phe Gly Gly Gly Thr Lys Leu Thr Val Leu
               100                 105                 110
<210> 116
   <211> 111
   <212> PRT
   <213> Homo sapiens
<400> 116
   Gln Ser Val Leu Thr Gln Pro Pro Ser Val Ser Gly Ala Pro Gly Gln
    1               5                  10                  15
   Arg Val Thr Ile Ser Cys Thr Gly Ser Ser Ser Asn Ile Gly Ser Gly
               20                  25                  30
   Tyr Asp Val His Trp Tyr Gln Gln Leu Pro Gly Thr Ala Pro Lys Leu
           35                  40                  45
   Leu Ile Tyr Gly Asn Ser Lys Arg Pro Ser Gly Val Pro Asp Arg Phe
       50                  55                  60
   Ser Gly Ser Lys Ser Gly Thr Ser Ala Ser Leu Ala Ile Thr Gly Leu
   65                  70                  75                  80
   Gln Ser Glu Asp Glu Ala Asp Tyr Tyr Cys Ala Ser Trp Thr Asp Gly
                   85                  90                  95
   Leu Ser Leu Val Val Phe Gly Gly Gly Thr Lys Leu Thr Val Leu
               100                 105                 110
<210> 117
   <211> 121
   <212> PRT
   <213> Homo sapiens
<400> 117
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Asn Ile Ser Ser Ser Gly Ser Ser Thr Tyr Tyr Ala Asp Ser Val
       50                  55                  60
   Lys Gly Arg Phe Thr Ile Ser Arg Asp Asn Ser Lys Asn Thr Leu Tyr
   65                  70                  75                  80
   Leu Gln Met Asn Ser Leu Arg Ala Glu Asp Thr Ala Val Tyr Tyr Cys
                   85                  90                  95
   Ala Arg Tyr Trp Gly Thr Pro Tyr Leu Met Gln Phe Asp Asn Trp Gly
               100                 105                 110
   Gln Gly Thr Leu Val Thr Val Ser Ser
           115                 120
<210> 118
   <211> 123
   <212> PRT
   <213> Homo sapiens
<400> 118
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Asn Ile Glu His Lys Phe Met Gly Tyr Thr Thr Tyr Tyr Ala Ala
       50                  55                  60
   Gly Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 119
   <211> 123
   <212> PRT
   <213> Homo sapiens
<400> 119
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Ser Ile Glu His Lys Tyr Thr Gly Tyr Thr Thr Tyr Tyr Ala Ala
       50                  55                  60
   Pro Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 120
   <211> 123
   <212> PRT
   <213> Homo sapiens
<400> 120
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Asn Ile Glu His Lys Tyr Thr Ser Tyr Thr Thr Tyr Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 121
   <211> 123
   <212> PRT
   <213> Homo sapiens
<400> 121
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Asn Ile Glu His Lys Tyr Leu Asn Tyr Ala Thr Tyr Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 122
   <211> 123
   <212> PRT
   <213> Homo sapiens
<400> 122
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Asn Ile Glu His Lys Tyr Leu Gly Tyr Ala Thr Val Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 123
   <211> 123
   <212> PRT
   <213> Homo sapiens
<400> 123
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Ser Ile Glu His Lys Tyr Leu Ser Tyr Ala Thr Tyr Tyr Ala Ala
       50                  55                  60
   Gly Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 124
   <211> 123
   <212> PRT
   <213> Homo sapiens
<400> 124
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Ser Ile Glu His Lys Tyr Leu Ser Tyr Thr Thr Phe Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 125
   <211> 123
   <212> PRT
   <213> Homo sapiens
<400> 125
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Gly Ile Glu His Lys Tyr Leu Ser Tyr Thr Thr His Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 126
   <211> 123
   <212> PRT
   <213> Homo sapiens
<400> 126
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Gln Ile Glu His Lys Tyr Leu Ser Tyr Thr Thr Leu Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 127
   <211> 123
   <212> PRT
   <213> Homo sapiens
<400> 127
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Gly Ile Glu His Lys Tyr Leu Ser Tyr Ala Thr Leu Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 128
   <211> 111
   <212> PRT
   <213> Homo sapiens
<400> 128
   Asp Ile Ala Leu Thr Gln Pro Ala Ser Val Ser Gly Ser Pro Gly Gln
    1               5                  10                  15
   Ser Ile Thr Ile Ser Cys Thr Gly Thr Ser Ser Asp Val Gly Gly Tyr
               20                  25                  30
   Asn Ser Val Ser Trp Tyr Gln Gln His Pro Gly Lys Ala Pro Lys Leu
           35                  40                  45
   Met Ile Tyr Ser Val Ser Ser Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Asp Thr Asn
                   85                  90                  95
   Lys Pro Leu Val Val Phe Gly Gly Gly Thr Lys Leu Thr Val Leu
               100                 105                 110
<210> 129
   <211> 111
   <212> PRT
   <213> Homo sapiens
<400> 129
   Asp Ile Ala Leu Thr Gln Pro Ala Ser Val Ser Gly Ser Pro Gly Gln
    1               5                   10                  15
   Ser Ile Thr Ile Ser Cys Thr Gly Thr Ser Ser Asp Val Gly Gly Tyr
               20                  25                  30
   Asn Ser Val Ser Trp Tyr Gln Gln His Pro Gly Lys Ala Pro Lys Leu
           35                  40                  45
   Met Ile Tyr Ser Val Ser Ser Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Ser Ser Tyr Tyr Phe Tyr
                   85                  90                  95
   Leu Gln Arg Ile Val Phe Gly Gly Gly Thr Lys Leu Thr Val Leu
               100                 105                 110
<210> 130
   <211> 111
   <212> PRT
   <213> Homo sapiens
<400> 130
   Asp Ile Ala Leu Thr Gln Pro Ala Ser Val Ser Gly Ser Pro Gly Gln
    1               5                   10                  15
   Ser Ile Thr Ile Ser Cys Thr Gly Thr Ser Ser Asp Val Gly Gly Tyr
               20                  25                  30
   Asn Ser Val Ser Trp Tyr Gln Gln His Pro Gly Lys Ala Pro Lys Leu
           35                  40                  45
   Met Ile Tyr Ser Val Ser Ser Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gln Thr Tyr Tyr Phe Ser
                   85                  90                  95
   Tyr Ser Gly Pro Val Phe Gly Gly Gly Thr Lys Leu Thr Val Leu
               100                 105                 110
<210> 131
   <211> 111
   <212> PRT
   <213> Homo sapiens
<400> 131
   Asp Ile Ala Leu Thr Gln Pro Ala Ser Val Ser Gly Ser Pro Gly Gln
    1               5                   10                  15
   Ser Ile Thr Ile Ser Cys Thr Gly Thr Ser Ser Asp Val Gly Gly Tyr
               20                  25                  30
   Asn Ser Val Ser Trp Tyr Gln Gln His Pro Gly Lys Ala Pro Lys Leu
           35                  40                  45
   Met Ile Tyr Ser Val Ser Ser Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gly Ser Tyr Asp Val Tyr
                   85                  90                  95
   Gly Arg Phe Tyr Val Phe Gly Gly Gly Thr Lys Leu Thr Val Leu
               100                 105                 110
<210> 132
   <211> 111
   <212> PRT
   <213> Homo sapiens
<400> 132
   Asp Ile Ala Leu Thr Gln Pro Ala Ser Val Ser Gly Ser Pro Gly Gln
    1               5                  10                  15
   Ser Ile Thr Ile Ser Cys Thr Gly Thr Ser Ser Asp Val Gly Gly Tyr
               20                  25                  30
   Asn Ser Val Ser Trp Tyr Gln Gln His Pro Gly Lys Ala Pro Lys Leu
           35                  40                  45
   Met Ile Tyr Ser Val Ser Ser Arg Pro Ser Gly Val Ser Asn Arg Phe
       50                  55                  60
   Ser Gly Ser Lys Ser Gly Asn Thr Ala Ser Leu Thr Ile Ser Gly Leu
   65                  70                  75                  80
   Gln Ala Glu Asp Glu Ala Asp Tyr Tyr Cys Gly Ser Trp Asp Pro Ile
                   85                  90                  95
   Phe Ser Tyr Glu Val Phe Gly Gly Gly Thr Lys Leu Thr Val Leu
               100                 105                 110
<210> 133
   <211> 381
   <212> DNA
   <213> Homo sapiens
<400> 133
   caggtgcagc tggtgcagtc tggggctgag gtgaagaagc ctgggtcctc ggtgaaggtc 60
   tcctgcaagg cttctggagg caccttcagc agcaactaca tcagctgggt gcgacaggcc 120
   cctggacaag ggcttgagtg gatggggatc agccctggca ccggtatcaa cgcatactac 180
   gcacagaagt tccagggcag agtcacgatt accgcggacg aatccacgag cacagcctac 240
   atggagctga gcagcctgag atctgaggac acggccgtgt attactgtgc gagaagcaag 300
   aagggcatgt acggcggctg gacctacccc ctgatgatgt tcgacctgtg gggccagggc 360
   accctggtga ccgtgagcag c                                           381
<210> 134
   <211> 381
   <212> DNA
   <213> Homo sapiens
<400> 134
   caggtgcagc tggtgcagag cggcgccgag gtgaagaagc ccggcagcag cgtgaaggtg 60
   agctgcaagg ccagcggcgg caccttcagc agcaactaca tcagctgggt gcgccaggcc 120
   cccggccagg gcctggagtg gatgggcatc agccccggca ccggcatcaa cgcctactac 180
   gcccagaagt tccagggccg cgtgaccatc accgccgacg agagcaccag caccgcctac 240
   atggagctga gcagcctgcg cagcgaggac accgccgtgt actactgcgc ccgcagcaag 300
   aagggcatgt acggcggctg gacctacccc ctgatgatgt tcgacctgtg gggccagggc 360
   accctggtga ccgtgagcag c                                           381
<210> 135
   <211> 381
   <212> DNA
   <213> Homo sapiens
<400> 135
   caggtgcaat tggttcagtc tggcgcggaa gtgaaaaaac cgggcagcag cgtgaaagtg 60
   agctgcaaag cctccggagg cactttttct tctaattata tttcttgggt gcgccaagcc 120
   cctgggcagg gtctcgagtg gatgggcatt tctcctggta ctggtattaa tgcttattat 180
   gctcagaagt ttcagggtcg ggtgaccatt accgcggatg aaagcaccag caccgcgtat 240
   atggaactga gcagcctgcg tagcgaagat acggccgtgt attattgcgc gcgttctaag 300
   aagggtatgt atggtggttg gacttatcct cttatgatgt ttgatctttg gggccaaggc 360
   accctggtga cggttagctc a                                          381
<210> 136
   <211> 324
   <212> DNA
   <213> Homo sapiens
<400> 136
   gagatcgtgc tgacccagag ccccgccacc ctgagcctga gccccggcga gcgcgccacc 60
   ctgagctgcc gcgccagcca gagcgtgagc agcaactacc tggcctggta ccagcagaag 120
   cccggccagg ccccccgcct gctgatctac tacgccagcc gccgcgccac cggcgtgccc 180
   gcccgcttca gcggcagcgg cagcggcacc gacttcaccc tgaccatcag cagcctggag 240
   cccgaggact tcgccgtgta ctactgccag cagaccagca acaccccctt caccttcggc 300
   cagggcacca aggtggagat caag                                       324
<210> 137
   <211> 324
   <212> DNA
   <213> Homo sapiens
<400> 137
   gaaattgtgt tgacacagtc tccagccacc ctgtctttgt ctccagggga aagagccacc 60
   ctctcctgca gggccagtca gagtgttagc agcaactact tagcctggta ccaacagaaa 120
   cctggccagg ctcccaggct cctcatctat tacgcatccc gcagggccac tggcgtgcca 180
   gccaggttca gtggcagtgg gtctgggaca gacttcactc tcaccatcag cagcctagag 240
   cctgaagatt ttgcagttta ttactgtcag cagacttcta atactccttt tacctttggc 300
   cagggtacga aagttgaaat taaa                                       324
<210> 138
   <211> 324
   <212> DNA
   <213> Homo sapiens
<400> 138
   gagatcgtgc tgacccagag cccggcgacc ctgagcctgt ctccgggcga acgtgcgacc 60
   ctgagctgca gagcgagcca gtctgtttct tctaattatc tggcttggta ccagcagaaa 120
   ccaggtcaag caccgcgtct attaatttat tatgcttctc gtcgtgcaac tggggtcccg 180
   gcgcgtttta gcggctctgg atccggcacg gattttaccc tgaccattag cagcctggaa 240
   cctgaagact ttgcggtgta ttattgccag cagacttcta atactccttt tacctttggc 300
   cagggtacga aagttgaaat taaa                                       324
<210> 139
   <211> 351
   <212> DNA
   <213> Homo sapiens
<400> 139
   gaggtgcagc tggtgcagtc tggagcagag gtgaaaaagc ccggggagtc tctgaagatc 60
   tcctgtaagg gttctggata cagctttagc aactactgga tcggctgggt gcgccagatg 120
   cccgggaaag gcctggagtg gatggggatc atcgacccta gcaactctta caccagatac 180
   agcccgtcct tccaaggcca ggtcaccatc tcagccgaca agtccatcag caccgcctac 240
   ctgcagtgga gcagcctgaa ggcctcggac accgccatgt attactgtgc gagatggtac 300
   tacaagccct tcgacgtgtg gggccagggc accctggtga ccgtgagcag c         351
<210> 140
   <211> 351
   <212> DNA
   <213> Homo sapiens
<400> 140
   gaggtgcagc tggtgcagag cggcgccgag gtgaagaagc ccggcgagag cctgaagatc 60
   agctgcaagg gcagcggcta cagcttcagc aactactgga tcggctgggt gcgccagatg 120
   cccggcaagg gcctggagtg gatgggcatc atcgacccca gcaacagcta cacccgctac 180
   agccccagct tccagggcca ggtgaccatc agcgccgaca agagcatcag caccgcctac 240
   ctgcagtgga gcagcctgaa ggccagcgac accgccatgt actactgcgc ccgctggtac 300
   tacaagccct tcgacgtgtg gggccagggc accctggtga ccgtgagcag c         351
<210> 141
   <211> 351
   <212> DNA
   <213> Homo sapiens
<400> 141
   gaggtgcaat tggttcagag cggcgcggaa gtgaaaaaac cgggcgaaag cctgaaaatt 60
   agctgcaaag gttccggata ttccttttct aattattgga ttggttgggt gcgccagatg 120
   cctgggaagg gtctcgagtg gatgggcatt atcgatccgt ctaatagcta tacccgctat 180
   tctccgagct ttcagggcca ggtgaccatt agcgcggata aaagcattag caccgcgtat 240
   cttcaatgga gcagcctgaa agcgagcgat acggccatgt attattgcgc gcgttggtat 300
   tataagcctt ttgatgtttg gggccaaggc accctggtga cggttagctc a         351
<210> 142
   <211> 336
   <212> DNA
   <213> Homo sapiens
<400> 142
   cagtctgtgc tgacgcagcc gccctcagtg tctggggccc cagggcagag ggtcaccatc 60
   tcctgcactg ggagcagctc caacatcggg agcggttatg atgtacactg gtaccagcag 120
   cttccaggaa cagcccccaa actcctcatc tatggtaaca gcaagcggcc ctcaggggtc 180
   cctgaccgat tctctggctc caagtctggc acctcagcct ccctggccat cactgggctc 240
   cagagcgagg atgaggctga ttattactgc gccagctgga ccgacggcct gagcctggtg 300
   gtgttcggcg gcggcaccaa gctgaccgtg ctgggc                           336
<210> 143
   <211> 336
   <212> DNA
   <213> Homo sapiens
<400> 143
   cagagcgtgc tgacccagcc ccccagcgtg agcggcgccc ccggccagcg cgtgaccatc 60
   agctgcaccg gcagcagcag caacatcggc agcggctacg acgtgcactg gtaccagcag 120
   ctgcccggca ccgcccccaa gctgctgatc tacggcaaca gcaagcgccc cagcggcgtg 180
   cccgaccgct tcagcggcag caagagcggc accagcgcca gcctggccat caccggcctc 240
   cagagcgagg acgaggccga ctactactgt gccagctgga ccgacggcct gagcctggtg 300
   gtgttcggcg gcggcaccaa gctgaccgtg ctgggc                           336
<210> 144
   <211> 336
   <212> DNA
   <213> Homo sapiens
<400> 144
   cagagcgtgc tgacccagcc gccttcagtg agtggcgcac caggtcagcg tgtgaccatc 60
   tcgtgtacgg gcagcagcag caacattggt tctggttatg atgtgcattg gtaccagcag 120
   ttgcccggga cggcgccgaa acttctgatt tatggtaatt ctaagcgtcc ctcaggcgtg 180
   ccggatcgtt ttagcggatc caaaagcggc accagcgcga gccttgcgat tacgggcctg 240
   caaagcgaag acgaagcgga ttattattgc gcttcttgga ctgatggtct ttctcttgtt 300
   gtgtttggcg gcggcacgaa gttaaccgtt cttggc                           336
<210> 145
   <211> 189
   <212> PRT
   <213> Homo sapiens
<400> 145
<210> 146
   <211> 19
   <212> PRT
   <213> Homo sapiens
<400> 146
   Asn Ile Glu His Lys Tyr Leu Gly Tyr Ala Thr Ser Tyr Ala Ala Ser
    1               5                  10                  15
   Val Lys Gly
<210> 147
   <211> 123
   <212> PRT
   <213> Homo sapiens
<400> 147
   Gln Val Gln Leu Val Glu Ser Gly Gly Gly Leu Val Gln Pro Gly Gly
    1               5                  10                  15
   Ser Leu Arg Leu Ser Cys Ala Ala Ser Gly Phe Thr Phe Ser Ser Phe
               20                  25                  30
   Gly Met Ser Trp Val Arg Gln Ala Pro Gly Lys Gly Leu Glu Trp Val
           35                  40                  45
   Ser Asn Ile Glu His Lys Tyr Leu Gly Tyr Ala Thr Ser Tyr Ala Ala
       50                  55                  60
   Ser Val Lys Gly Arg Phe Thr Ile Ser Arg Asp Asn Ser Lys Asn Thr
   65                  70                  75                  80
   Leu Tyr Leu Gln Met Asn Ser Leu Arg Ala Glu Asp Thr Ala Val Tyr
                   85                  90                  95
   Tyr Cys Ala Arg Tyr Trp Gly Thr Pro Tyr Leu Met Gln Phe Asp Asn
               100                 105                 110
   Trp Gly Gln Gly Thr Leu Val Thr Val Ser Ser
           115                 120
<210> 148
   <211> 12
   <212> PRT
   <213> Homo sapiens
<400> 148
   His Gln Gly Leu Ile Phe Tyr Glu Lys Leu Leu Gly
           1               5                   10